# EUROPEAN PATENT SPECIFICATION

(11) **EP 2 411 395 B1**
(45) Date of publication and mention of the grant of the patent: **29.05.2013**
(21) Application number: 10716050.9
(22) Date of filing: 22.03.2010
(51) Int. Cl.: C07D 491/04

(54) **FUROPYRIMIDINEDIONE DERIVATIVES AS TRPA1 MODULATORS**
FUROPYRIMIDINDIONDERIVATE ALS TRPA1-MODULATOREN
DÉRIVÉS DE FUROPYRIMIDINEDIONE À TITRE DE MODULATEURS DE TRPA1

(30) Priority: 23.03.2009 IN MU06652009; 15.10.2009 US 251994 P; 23.09.2009 IN MU22132009; 21.04.2009 US 171355 P; 16.12.2009 IN MU29062009; 12.01.2010 US 294470 P
(43) Date of publication of application: 01.02.2012
(73) Proprietor: Glenmark Pharmaceuticals S.A., 2300 La Chaux-de-Fonds (CH)
(72) Inventor: CHAUDHARI, Sachin, Sundarlal, Maharashtra 400709 (IN); THOMAS, Abraham, Maharashtra 400 705 (IN); PATIL, Nisha, Parag, Maharashtra 400066 (IN); KHAIRATKAR-JOSHI, Neelima, Maharashtra (W)-400 602 (IN); MUKHOPADHYAY, Indranil, Maharashtra 400 706 (IN)
(74) Representative: Müller-Boré & Partner Patentanwälte
(86) International application number: PCT/IB2010/000840
(87) International publication number: WO 2010/109329

(56) References cited:
- WO-A1-2004/014916
- WO-A1-2009/002933
- WO-A2-2007/073505
- PRESS J B ET AL: "Furo[3,4-d]pyrimidine-2,4-dione derivatives with antihypertensive activity. Analogues of thienopyrimidine-2,4-diones" 1 November 1989 (1989-11-01), EUROPEAN JOURNAL OF MEDICINAL CHEMISTRY, EDITIONS SCIENTIFIQUE ELSEVIER, PARIS, FR LNKD- DOI:10.1016/0223-5234(89)90033-0, PAGE(S) 627 - 630 , XP023870333 ISSN: 0223-5234 [retrieved on 1989-11-01] the whole document

## Description

### Technical Field

The present patent application relates to furopyrimidinedione derivatives as TRPA1 modulators with transient receptor potential ankyrin I (TRPA1) activity.

### Background of the Invention

The transient receptor potential (TRP) channels or receptors are pain receptors. They have been classified into seven subfamilies: TRPC (canonical), TRPV (vanilloid), TRPM (melastatin), TRPP (polycystin), TRPML (mucolipin), TRPA (ankyrin, ANKTM1) and TRPN (NOMPC) families. The TRPC family can be divided into 4 subfamilies (i) TRPC1 (ii) TRPC2 (iii) TRPC3, TRPC6, TRPC7 and (iv) TRPC4, TRPC5 based on sequence functional similarities. Currently the TRPV family has 6 members. TRPV5 and TRPV6 are more closely related to each other than to TRPV1, TRPV2, TRPV3 or TRPV4. TRPA 1 is most closely related to TRPV3 and is more closely related to TRPV 1 and TRPV2 than to TRPV5 and TRPV6. The TRPM family has 8 members. Constituents include the following: the founding member TRPM1 (melastatin or LTRPC1), TRPM3 (KIAA1616 or LTRPC3), TRPM7 (TRP-PLIK, ChaK(1), LTRPC7), TRPM6 (ChaK2), TRPM2 (TRPC7 or LTRPC2), TRPM8 (TRP-p8 or CMR1), TRPM5 (MTR1 or LTRPC5) and TRPM4 (FLJ20041 or LTRPC4). The TRPML family consists of the mucolipins, which include TRPML1 (mucolipin 1), TRPML2 (mucolipin 2) and TRPML3 (mucolipin 3). The TRPP family consists of two groups of channels: those predicted to have six transmembrane domains and those that have eleven. TRPP2 (PKD2), TRPP3 (PKD2L1), TRPP5 (PKD2L2) are all predicted to have six transmembrane domains. TRPP1 (PKD1, PC1), PKD-REJ and PKD-1L1 are all thought to have eleven transmembrane domains. The sole mammalian member of the TRPA family is ANKTM1.

It is believed TRPA1 is expressed in nociceptive neurons. Nociceptive neurons of the nervous system sense the peripheral damage and transmit pain signals. TRPA1 is membrane bound and most likely acts as a heterodimeric voltage gated channel. It is believed to have a particular secondary structure, its N-terminus is lined with a large number of ankyrin repeats which are believed to form a spring-like edifice. TRPA1 is activated by a variety of noxious stimuli, including cold temperatures (activated at 17°C), pungent natural compounds (e.g., mustard, cinnamon and garlic) and environmental irritants (MacPherson, L. J. et al., Nature, 2007, 445; 541-545). Noxious compounds activate TRPA1 ion channels through covalent modification of cysteines to form covalently linked adducts. Variety of endogenous molecules produced during tissue inflammation / injury have been identified as pathological activators of TRPA1 receptor. These include hydrogen peroxide which is produced due to oxidative stress generated during inflammation, alkenyl aldehyde 4-HNE - an intracellular lipid pcroxidation product and cyclopentenone prostaglandin 15dPGJ2 which is produced from PGD2 during inflammation / allergic response. TRPA1 is also activated in receptor dependant fashion by Bradykinin (BK) which is released during tissue injury at peripheral terminals

The difference between TRPA1 and other TRP receptors is that TRPA1 ligand binding persists for hours due to which the physiological response (e.g., pain) is greatly prolonged. Hence to dissociate the electrophile, an effective antagonist is required.

WO 2009/158719, WO 2009/002933, WO 2008/0949099, WO 2007/073505, WO 2004/055054 and WO 2005/089206 describe the TRP channels as the targets for the treatment of pain and related conditions.

In efforts to discover better analgesics for the treatment of both acute and chronic pain and to develop treatments for various neuropathic and nociceptive pain states, there exists a need for a more effective and safe therapeutic treatment of diseases, conditions and/or disorders modulated by TRPA1.

### Summary of the Invention

The present invention describes compounds of the formula (I): 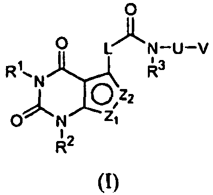 or a pharmaceutically acceptable salt thereof,
wherein,
R¹ and R², which may be the same or different, are independently selected from hydrogen, substituted or unsubstituted alkyl, alkenyl, alkynyl, cycloalkyl, cycloalkylalkyl, arylalkyl, (CR^{x}R^{y})ₙOR^{x}, COR^{x}, COOR^{x}, CONR^{x}R^{y}, (CH₂)ₙNR^{x}R^{y}, (CH₂)ₙCHR^{x}R^{y}, (CH₂)ₙNR^{x}R^{y} and (CH₂)ₙNHCOR^{x};
R³ is selected from hydrogen, substituted or unsubstituted alkyl, alkenyl, alkynyl, cycloalkyl, cycloalkylalkyl and cycloalkenyl;
Z₁ and Z₂ are independently oxygen or CR^{a}; with a proviso that one of Z₁ or Z₂ is always oxygen and other is CR^{a};
L is a linker selected from -(CR^{x}R^{y})ₙ-, -O-(CR^{x}R^{y})ₙ-, -C(O)-, -NR^{x}-, -S(O)ₘNR^{x}-, -NR^{x}(CR^{x}R^{y})ₙ- and -S(O)ₘNR^{x}(CR^{x}R^{y})ₙ;
R^{a} is selected from hydrogen, cyano, substituted or unsubstituted alkyl, alkenyl, alkynyl, cycloalkyl, cycloalkylalkyl, aryl, arylalkyl, biaryl, heteroaryl, heteroarylalkyl, heterocyclic ring, heterocyclylalkyl, OR^{x}, (CR^{x}R^{y})ₙOR^{x}, COR^{x}, COOR^{x}, CONR^{x}R^{y}, S(O)ₘNR^{x}R^{y}, NR^{x}R^{y}, NR^{x}(CR^{x}R^{y})ₙOR^{x}, (CH₂)ₙNR^{x}R^{y}, (CH₂)ₙCHR^{x}R^{y}, NR^{x}(CR^{x}R^{y})ₙCONR^{x}R^{y}, (CH₂)ₙNHCOR^{x}, (CH₂)ₙNH(CH₂)ₙSO₂R^{x}, (CH₂)ₙNHSO₂R^{x}, SR^{x} and OR^{x};
U is selected from -(CR^{x}R^{y})ₙ-, substituted or unsubstituted aryl, substituted or unsubstituted five membered heterocycles selected from the group consisting of thiazole, isothiazole, oxazole, isoxazole, thiadiazole, oxadiazole, pyrazole, imidazole, furan, thiophene, pyrroles, 1,2,3-triazoles and 1, 2, 4-triazole, or substituted or unsubstituted six membered heterocycle selected from the group consisting of pyrimidine, pyridine and pyridazine;
V is selected from hydrogen, cyano, nitro, -NR^{x}R^{y}, halogen, hydroxyl, substituted or unsubstituted alkyl, alkenyl, alkynyl, cycloalkyl, cycloalkylalkyl, cycloalkenyl, haloalkyl, haloalkoxy, cycloalkylalkoxy, aryl, arylalkyl, biaryl, heteroaryl, heteroarylalkyl, heterocyclic ring and heterocyclylalkyl, -C(O)OR^{x}, -OR^{x}, -C(O)NR^{x}R^{y}, -C(O)R^{x} and -SO₂NR^{x}R^{Y}; or
alternatively, U and V together may form an optionally substituted 3 to 7 membered saturated or unsaturated cyclic ring that may optionally include one or more heteroatoms selected from O, S and N;
at each occurrence, R^{x} and R^{y} are independently selected from hydrogen, hydroxyl, halogen, substituted or unsubstituted alkyl, alkenyl, alkynyl, cycloalkyl, cycloalkylalkyl, cycloalkenyl, aryl, arylalkyl, heteroaryl, heteroarylalkyl, heterocyclic ring and heterocyclylalkyl; and
at each occurrence, 'm' and 'n' arc independently selected from 0 to 2, both inclusive.

According to the invention, there is provided a compound of the formula (Ia): 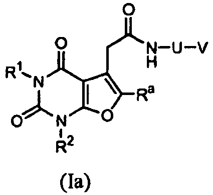 or a pharmaceutically acceptable salt thereof,
wherein,
R¹ and R², which may be the same or different, are independently selected from hydrogen, substituted or unsubstituted alkyl, haloalkyl, alkenyl, alkynyl, cycloalkyl, cycloalkylalkyl, arylalkyl, (CR^{x}R^{y})ₙOR^{x}, COR^{x}, COOR^{x}, CONR^{x}R^{y}, (CH₂)ₙNR^{x}R^{y}, (CH₂)ₙCHR^{x}R^{y}, (CH₂)ₙNR^{x}R^{y} and (CH₂)ₙNHCOR^{x};
R^{a} is selected from hydrogen, cyano, substituted or unsubstituted alkyl, alkenyl, alkynyl, cycloalkyl, cycloalkylalkyl, aryl, arylalkyl, biaryl, heteroaryl, heteroarylalkyl, heterocyclic ring, heterocyclylalkyl, OR^{x}, (CR^{x}R^{y})ₙOR^{x}, COR^{x}, COOR^{x}, CONR^{x}R^{y}, S(O)ₘNR^{x}R^{y}, NR^{x}R^{y}, NR^{x}(CR^{x}R^{y})ₙOR^{x}, (CH₂)ₙNR^{x}R^{y}, (CH₂)ₙCHR^{x}R^{y}, NR^{x}(CR^{x}R^{y})ₙCONR^{x}R^{y}, (CH₂)ₙNHCOR^{x}, (CH₂)ₙNH(CH₂)ₙSO₂R^{x}, (CH₂)ₙNHSO₂R^{x}, SR^{x} and OR^{x};
U is selected from -(CR^{x}R^{y})ₙ-, substituted or unsubstituted aryl, substituted or unsubstituted five membered heterocycles selected from the group consisting of thiazole, isothiazole, oxazole, isoxazole, thiadiazole, oxadiazole, pyrazole, imidazole, furan, thiophene, pyrroles, 1,2,3-triazoles and 1, 2, 4-triazole, or substituted or unsubstituted six membered heterocycle selected from the group consisting of pyrimidine, pyridine and pyridazine;
V is selected from hydrogen, cyano, nitro, -NR^{x}R^{y}, halogen, hydroxyl, substituted or unsubstituted alkyl, alkenyl, alkynyl, cycloalkyl, cycloalkylalkyl, cycloalkenyl, haloalkyl, haloalkoxy, cycloalkylalkoxy, aryl, arylalkyl, biaryl, heteroaryl, heteroarylalkyl, heterocyclic ring and heterocyclylalkyl, -C(O)OR^{x}, -OR^{x}, -C(O)NR^{x}R^{y}, -C(O)R^{x} and -SO₂NR^{x}R^{y}; or
alternatively, U and V together may form an optionally substituted 3 to 7 membered saturated or unsaturated cyclic ring that may optionally include one or more heteroatoms selected from O, S and N;
at each occurrence, R^{x} and R^{y} are independently selected from hydrogen, hydroxyl, halogen, substituted or unsubstituted alkyl, alkenyl, alkynyl, cycloalkyl, cycloalkylalkyl, cycloalkenyl, aryl, arylalkyl, heteroaryl, heteroarylalkyl, heterocyclic ring and heterocyclylalkyl; and
at each occurrence, 'm' and 'n' are independently selected from 0 to 2, both inclusive.

The embodiments below are illustrative of the present invention and are not intended to limit the claims to the specific embodiments exemplified.

According to one embodiment, specifically provided are compounds of the formula (Ia) in which R^{a} is hydrogen or (C₁-C₄) alkyl.

According to yet another embodiment, specifically provided are compounds of the formula (Ia) in which R¹ and R² are methyl.

According to yet another embodiment, specifically provided are compounds of the formula (Ia) in which 'U' is substituted or unsubstituted five membered heterocycle, preferably thiazole, imidazole, isoxazole, pyrazole or thiadiazole and the substituent is alkyl, halogen, haloalkyl, hydroxyalkyl or substituted or unsubstituted phenyl.

According to yet another embodiment, specifically provided are compounds of the formula (Ia) in which 'U' is substituted or unsubstituted six membered heterocycle, preferably pyridine.

According to yet another embodiment, specifically provided are compounds of the formula (Ia) in which 'U' is substituted or unsubstituted aryl, preferably phenyl.

According to yet another embodiment, specifically provided are compounds of the formula (Ia) in which 'U' is -CH₂-CH₂-linker.

According to yet another embodiment, specifically provided are compounds of the formula (Ia) in which 'U' and 'V' are fused to form benzothiazole ring which is optionally substituted with one or more halogens.

According to yet another embodiment, specifically provided are compounds of the formula (Ia) in which 'V' is substituted or unsubstituted aryl, preferably phenyl. In this embodiment the substituents on phenyl may be one or more and are independently selected from halogen (for example F, Cl or Br), cyano, alkyl (for example *iso*-butyl or *tert*-butyl), haloalkyl (for example CF₃), alkoxy (for example OCH₃, OCH₂CH(CH₃)₂, OCH₂C(CH₃)₃ or OCH₂)CH₂CH(CH₃)₂), haloalkoxy (for example OCHF₂, OCF₃, OCH₂CF₃, or OCH₂CH₂CF₃) and cycloalkylalkoxy (for example cyclopropylmethoxy).

Described is a compound of the formula (Ib): 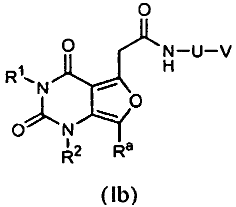 or a pharmaceutically acceptable salt thereof,
wherein,
U, V, R¹, R² and R^{a} are as defined above.

Described are compounds of the formula (Ib) in which R^{a} is hydrogen or (C₁-C₄) alkyl.

Described are compounds of the formula (Ib) in which R¹ and R² are methyl.

Described are compounds of the formula (Ib) in which 'U' is substituted or unsubstituted five membered heterocycle, preferably thiazole.

Described are compounds of the formula (Ib) in which 'V' is substituted or unsubstituted aryl, preferably phenyl. In this embodiment the substituents on phenyl may be one or more and are independently selected from halogen (for example F, Cl or Br), alkyl (CH₂CH(CH₃)₂), haloalkyl (for example CF₃) and haloalkoxy (for example OCHF₂, OCF₃ or OCH₂CF₃).

According to one embodiment, there is provided a compound of the formula (Ic): 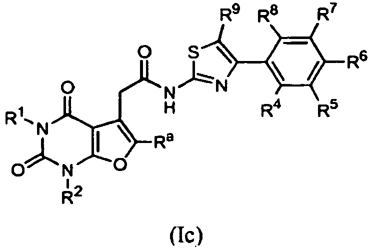 or a pharmaceutically acceptable salt thereof,
wherein,
R¹, R² and R^{a} which may be the same or different, arc each independently hydrogen or (C₁-C₄)alkyl; and
R⁴, R⁵, R⁶, R⁷, R⁸ and R⁹, which may be same or different, are each independently selected from the group comprising of hydrogen, halogen, cyano, hydroxyl, nitro, amino, substituted or unsubstituted alkyl, alkoxy, haloalkyl, haloalkoxy, cycloalkyl, cycloalkylalkyl, cycloalkenyl, cycloalkylalkoxy, aryl, arylalkyl, biaryl, heteroaryl, heteroarylalkyl, heterocyclic ring and heterocyclylalkyl.

The embodiments below are illustrative of the present invention and are not intended to limit the claims to the specific embodiments exemplified.

According to one embodiment, specifically provided are compounds of the formula (Ic) in which R^{a} is hydrogen or (C₁-C₄) alkyl, for example methyl.

According to another embodiment, specifically provided are compounds of the formula (Ic) in which R¹ and R² are methyl.

According to another embodiment, specifically provided are compounds of the formula (Ic) in which R⁴, R⁵, R⁶ and R⁷ are independently selected from hydrogen, fluoro, chloro, bromo, *iso*-butyl, 22-dimethylpropoxy, *iso*-butoxy, 3-methylbutoxy, difluoromethoxy, trifluoromethyl trifluoromethoxy, trifluoroethoxy, trifluoropropoxy, and cyclopropylmethoxy.

According to yet another embodiment, specifically provided are compounds of the formula (Ic) in which R⁸ is hydrogen.

According to one embodiment, specifically provided are compounds of the formula (Ic) in which R⁹ is hydrogen or (C₁-C₄) alkyl.

Particularly contemplated are compounds of the formulas, (Ia), and (Ic), which possess IC₅₀ of less than 250 nM, preferably, less than 100 nM, more preferably, less than 50 nM with respect to TRPAI activity as measured by method as described in the present patent application.

It should be understood that the formulas (I), (Ia), (Ib) and (Ic) structurally encompasses all stereoisomers, enantiomers and diastereomers, and pharmaceutically acceptable salts that may be contemplated from the chemical structure of the genera described herein.

The compound of the present invention as TRPA1 modulator is used herein because it is more selective for one TRP isoform than other, e.g., 2-fold, 5-fold, 10-fold, and more preferably at least 20, 40, 50, 60, 70, 80, or at least 100- or even 1000-fond more selective for TRPA1 over one or more of TRPC6, TRPV5, TRPV6, TRPM8, TRPV1, TRPV2, TRPV4 and/or TRPV3.

In accordance with another aspect, the present patent application provides a pharmaceutical composition that includes at least one compound described herein and at least one pharmaceutically acceptable excipient (such as a pharmaceutically acceptable carrier or diluent). Preferably, the pharmaceutical composition comprises a therapeutically effective amount of at least one compound described herein. The compounds described in the present patent application may be associated with a pharmaceutically acceptable excipient (such as a carrier or a diluent) or be diluted by a carrier, or enclosed within a carrier which can be in the form of a capsule, sachet, paper or other container.

The compounds of the present invention can be administered as pharmaceutical composition containing, for example, 0.1 to 99.5% (more preferably, 0.5 to 90%) of active ingredient in combination with a pharmaceutically acceptable carrier. The ultimate dose will depend on the condition being treated, the route of administration and the age, weight and condition of the patient and will be the doctor's discretion.

Compounds of the present invention may be used in the manufacture of medicaments for the treatment of any diseases disclosed herein. The compounds and pharmaceutical compositions described herein are useful for modulating TRPA1 receptors, wherein modulation is believed to be related to a variety of disease states.

The compound of the present invention can be administered alone or in combination with other therapeutic agents. For instance, the TRPA1 modulator is administered conjointly with one or more of an anti-inflammatory agent, anti-acne agent, anti-wrinkle agent, anti-scarring agent, anti-psoriatic agent, anti-proliferative agent, antifungal agent, anti-viral agent, anti-septic agent, anti-migraine agent, keratolytic agent, or a hair growth inhibitor

In accordance with another aspect, the present patent application further provides a compound of formula (Ia) for use in a method of inhibiting TRPA I receptors in a subject in need thereof by administering to the subject one or more compounds described herein in the amount effective to cause inhibition of such receptor.

### Detailed Description of the Invention

### Definitions

The terms "halogen" or "halo" includes fluorine, chlorine, bromine or iodine.

The term "alkyl" refers to a straight or branched hydrocarbon chain radical consisting solely of carbon and hydrogen atoms, containing no unsaturation, having from one to eight carbon atoms, and which is attached to the rest of the molecule by a single bond, e.g., methyl, ethyl, n-propyl, 1-methylethyl (isopropyl), n-butyl, n-pentyl and 1,1-dimethylethyl (*tert*-butyl). The term "C₁₋₆ alkyl" refers to an alkyl chain having 1 to 6 carbon atoms. Unless set forth or recited to the contrary, all alkyl groups described herein may be straight chain or branched, substituted or unsubstituted

The term "alkenyl" refers to an aliphatic hydrocarbon group containing a carbon-carbon double bond and which may be a straight or branched chain having 2 to about 10 carbon atoms, e.g., ethenyl, 1-propenyl, 2-propenyl (allyl), iso-propenyl, 2-methyl-1-propenyl, 1-butenyl and 2-butenyl. Unless set forth or recited to the contrary, all alkenyl groups described herein may be straight chain or branched, substituted or unsubstituted.

The term "alkynyl" refers to a straight or branched chain hydrocarbyl radical having at least one carbon-carbon triple bond and having 2 to about 12 carbon atoms (with radicals having 2 to about 10 carbon atoms being preferred) e.g., ethynyl, propynyl and butynyl. Unless set forth or recited to the contrary, all alkynyl groups described herein may be straight chain or branched, substituted or unsubstituted.

The term "alkoxy" refers to a straight or branched, saturated aliphatic hydrocarbon radical bonded to an oxygen atom that is attached to a core structure. Examples of alkoxy groups include but are not limited to methoxy, ethoxy, propoxy, isopropoxy, butoxy, isobutoxy, tert-butoxy, pentoxy, 3-methyl butoxy and the like. Unless set forth or recited to the contrary, all alkoxy groups described herein may be straight chain or branched, substituted or unsubstituted.

The term "haloalkyl" and "haloalkoxy" means alkyl or alkoxy, as the case may be, substituted with one or more halogen atoms, where alkyl and alkoxy groups are as defined above. The term "halo" is used herein interchangeably with the term "halogen" means F, Cl, Br or I. Examples of "haloalkyl" include but are not limited to trifluoromethyl, difluoromethyl, 2,2,2-trifluoroethyl, pentafluoroethyl, pentachloroethyl 4,4,4-trifluorobutyl, 4,4-difluorocyclohexyl, chloromethyl, dichloromethyl, trichloromethyl, 1-bromoethyl and the like. Examples of "haloalkoxy" include but are not limited to fluoromethoxy, difluoromethoxy, trifluoromethoxy, 2,2,2-trifluoroethoxy, pentafluoroethoxy, pentachloroethoxy, chloromethoxy, dichlorormethoxy, trichloromethoxy, 1-bromoethoxy and the like. Unless set forth or recited to the contrary, all "haloalkyl" and "haloalkoxy" groups described herein may be straight chain or branched, substituted or unsubstituted.

The term "cycloalkyl" denotes a non-aromatic mono or multicyclic ring system of 3 to about 12 carbon atoms, such as cyclopropyl, cyclobutyl, cyclopentyl and cyclohexyl. Examples of multicyclic cycloalkyl groups include, but are not limited to, perhydronapththyl, adamantyl and nurburnyl groups, bridged cyclic groups or sprirobicyclic groups, e.g., spiro(4,4) non-2-yl. Unless set forth or recited to the contrary, all cycloalkyl groups described herein may be substituted or unsubstituted.

The term "cycloalkylalkyl" refers to a cyclic ring-containing radical having 3 to about 8 carbon atoms directly attached to an alkyl group. The cycloalkylalkyl group may be attached to the main structure at any carbon atom in the alkyl group that result in the creation of a stable structure. Non-limiting examples of such groups include cyclopropylmethyl, cyclobutylethyl and cyclopentylethyl. Unless set forth or recited to the contrary, all cycloalkylalkyl groups described herein may be substituted or unsubstituted.

The term "cycloalkylalkoxy" is used to denote alkoxy substituted with cycloalkyl, wherein 'alkoxy' and 'cycloalkyl' are as defined above (either in the broadest aspect or a preferred aspect). Examples of cycloalkylalkoxy groups include cyclopropylmethoxy, 1-or 2-cyclopropylethoxy, 1-, 2- or 3- cyclopropylpropoxy, 1-, 2-, 3- or 4-cyclopropylbutoxy, cyclobutylmethoxy, 1- or 2- cyclobutylethoxy, 1-, 2- or 3- cyclobutylpropoxy, 1-, 2-, 3- or 4-cyclobutylbutoxy, cyclopentylmethoxy, 1- or 2-cyclopentylethoxy, 1-, 2- or 3-cyclopentylpropoxy, 1-, 2-, 3- or 4- cyclopentylbutoxy, cyclohexylmethoxy, 1- or 2-cyclohexylethoxy and 1-, 2- or 3- cyclohexylpropoxy. Preferably, 'cycloalkylalkoxy' is (C₃₋₆)cycloalkyl-(C₁₋₆)alkoxy. Unless set forth or recited to the contrary, all cycloalkylalkoxy groups described herein may be substituted or unsubstituted.

The term "cycloalkenyl" refers to a cyclic ring-containing radical having 3 to about 8 carbon atoms with at least one carbon-carbon double bond, such as cyclopropenyl, cyclobutenyl and cyclopentenyl. Unless set forth or recited to the contrary, all cycloalkenyl groups described herein may be substituted or unsubstituted.

The term "aryl" means a carbocyclic aromatic system containing one, two or three rings wherein such rings may be fused. If the rings are fused, one of the rings must be fully unsaturated and the fused ring(s) may be fully saturated, partially unsaturated or fully unsaturated. The term "fused" means that a second ring is present (ie, attached or formed) by having two adjacent atoms in common (i.e., shared) with the first ring. The term "fused" is equivalent to the term "condensed". The term "aryl" embraces aromatic radicals such as phenyl, naphthyl, tetrahydronaphthyl, indane and biphenyl. Unless set forth or recited to the contrary, all aryl groups described herein may be substituted or unsubstituted.

The term "arylalkyl" refers to an aryl group as defined above directly bonded to an alkyl group as defined above, e.g., -CH₂C₆H₅ or -C₂H₄C₆H₅. Unless set forth or recited to the contrary, all arylalkyl groups described herein may be substituted or unsubstituted.

The term "heterocyclic ring" refers to a stable 3- to 15-membered ring radical which consists of carbon atoms and from one to five heteroatoms selected from nitrogen, phosphorus, oxygen and sulfur. For purposes of this invention, the heterocyclic ring radical may be a monocyclic, bicyclic or tricyclic ring system, which may include fused, bridged or spiro ring systems and the nitrogen, phosphorus, carbon, oxygen or sulfur atoms in the heterocyclic ring radical may be optionally oxidized to various oxidation states. In addition, the nitrogen atom may be optionally quaternized; and the ring radical may be partially or fully saturated (i.e., heterocyclic or heteroaryl). Examples of such heterocyclic ring radicals include, but are not limited to, azetidinyl, acridinyl, benzodioxolyl, benzodioxanyl, benzofuranyl, carbazolyl, cinnolinyl, dioxolanyl, indolizinyl, naphthyridinyl, perhydroazepinyl, phenazinyl, phenothiazinyl, phenoxazinyl, phthalazinyl, pyridyl, pteridinyl, purinyl, quinazolinyl, quinoxalinyl, quinolinyl, isoquinolinyl, tetrazolyl, imidazolyl, tetrahydroisoqinolyl, piperidinyl, piperazinyl, 2-oxopiperazinyl, 2-oxopiperidinyl, 2-oxopyrrolidinyl, 2-oxoazepinyl, azepinyl, pyrrolyl, 4-piperidonyl, pyrrolidinyl, pyrazinyl, pyrimidinyl, pyridazinyl, oxazolyl, oxazolinyl, oxazolidinyl, triazolyl, indanyl, isoxazolyl, isoxazolidinyl, morpholinyl, thiazolyl, thiazolinyl, thiazolidinyl, isothiazolyl, quinuclidinyl, isothiazolidinyl, indolyl, isoindolyl, indolinyl, isoindolinyl, octahydroindolyl, octahydroisoindolyl, quinolyl, isoquinolyl, decahydroisoquinolyl, benzimidazolyl, thiadiazolyl, benzopyranyl, benzothiazolyl, benzooxazolyl, furyl, tetrahydrofuryl, tetrahydropyranyl, thienyl, benzothienyl, thiamorpholinyl, thiamorpholinyl sulfoxide, thiamorpholinyl sulfone, dioxaphospholanyl, oxadiazolyl, chromanyl and isochromanyl. The heterocyclic ring radical may be attached to the main structure at any heteroatom or carbon atom that results in the creation of a stable structure. Unless set forth or recited to the contrary, all heterocyclic ring described herein may be substituted or unsubstituted.

The term "heterocyclyl" refers to a heterocyclic ring radical as defined above. The heterocyclyl ring radical may be attached to the main structure at any heteroatom or carbon atom that results in the creation of a stable structure. Unless set forth or recited to the contrary, all heterocyclyl groups described herein may be substituted or unsubstituted.

The term "heterocyclylalkyl" refers to a heterocyclic ring radical directly bonded to an alkyl group. The heterocyclylalkyl radical may be attached to the main structure at any carbon atom in the alkyl group that results in the creation of a stable structure. Unless set forth or recited to the contrary, all heterocyclylalkyl groups described herein may be substituted or unsubstituted.

The term "heteroaryl" refers to an aromatic heterocyclic ring radical. The heteroaryl ring radical may be attached to the main structure at any heteroatom or carbon atom that results in the creation of a stable structure. Unless set forth or recited to the contrary, all heteroaryl groups described herein may be substituted or unsubstituted.

The term "heteroarylalkyl" refers to a heteroaryl ring radical directly bonded to an alkyl group. The heteroarylalkyl radical may be attached to the main structure at any carbon atom in the alkyl group that results in the creation of a stable structure. Unless set forth or recited to the contrary, all heteroarylalkyl groups described herein may be substituted or unsubstituted.

Unless otherwise specified, the term "substituted" as used herein refers to substitution with any one or more or any combination of the following substituents: hydroxy, halogen, carboxyl, cyano, nitro, oxo (=O), thio (=S), substituted or unsubstituted alkyl, substituted or unsubstituted haloalkyl, substituted or unsubstituted alkoxy, substituted or unsubstituted haloalkoxy, substituted or unsubstituted alkenyl, substituted or unsubstituted alkynyl, substituted or unsubstituted aryl, substituted or unsubstituted arylalkyl, substituted or unsubstituted cycloalkyl, substituted or unsubstituted cycloalkenylalkyl, substituted or unsubstituted cycloalkenyl, substituted or unsubstituted amino, substituted or unsubstituted aryl, substituted or unsubstituted heteroaryl, substituted or unsubstituted heterocyclylalkyl ring, substituted or unsubstituted heteroarylalkyl, substituted or unsubstituted heterocyclic ring, substituted or unsubstiuted guanidine, -COOR^{x'}, -C(O)R^{x'}, -C(S)R^{x'}, -C(O)NR^{x'}R^{y'}, -C(O)ONR^{x'}R^{y'}, -NR^{x'}CONR^{y'}R^{z'}, - N(R^{x'})SOR^{y'}, -N(R^{x'})SO₂R^{y'}, -(=N-N(R^{x'})R^{y'}), -NR^{x'}C(O)OR^{y'}, -NR^{x'}R^{y'}, -NR^{x'}C(O)R^{y'}, - NR^{x'}C(S)R^{y'}, -NR^{x'}C(S)NR^{y'}R^{z'}, -SONR^{x'}R^{y'}, -SO₂NR^{x'}R^{y'}, -OR^{x'}, -OR^{x'}C(O)NR^{y'}R^{z'}, - OR^{x'}C(O)OR^{y'}, -OC(O)R^{x'}, -OC(O)NR^{x'}R^{y'}, -R^{x'}NR^{y'}C(O)R^{z'}, -R^{x'}OR^{y'}, -R^{x'}C(O)OR^{y'}, - R^{x'}C(O)NR^{y'}R^{z'}, -R^{x'}C(O)R^{y'}, -R^{x'}OC(O)R^{y'}, -SR^{x'}, -SOR^{x'}, -SO₂R^{x'} and -ONO₂, wherein R^{x'}, R^{y'} and R^{z'} are independently selected from hydrogen, substituted or unsubstituted alkyl, substituted or unsubstituted alkoxy, substituted or unsubstituted alkenyl, substituted or unsubstituted alkynyl, substituted or unsubstituted aryl, substituted or unsubstituted arylalkyl, substituted or unsubstituted cycloalkyl, substituted or unsubstituted cycloalkenyl, substituted or unsubstituted amino, substituted or unsubstituted aryl, substituted or unsubstituted heteroaryl, substituted heterocyclylalkyl ring, substituted or unsubstituted heteroarylalkyl or substituted or unsubstituted heterocyclic ring.

The term "treating" or "treatment" of a state, disorder or condition includes; (a) preventing or delaying the appearance of clinical symptoms of the state, disorder or condition developing in a subject that may be afflicted with or predisposed to the state, disorder or condition but does not yet experience or display clinical or subclinical symptoms of the state, disorder or condition; (b) inhibiting the state, disorder or condition, i.e., arresting or reducing the development of the disease or at least one clinical or subclinical symptom thereof; or (c) relieving the disease, i.e., causing regression of the state, disorder or condition or at least one of its clinical or subclinical symptoms.

The term "subject" includes mammals (especially humans) and other animals, such as domestic animals (e.g., household pets including cats and dogs) and non-domestic animals (such as wildlife).

A "therapeutically effective amount" means the amount of a compound that, when administered to a subject for treating a state, disorder or condition, is sufficient to effect such treatment. The "therapeutically effective amount" will vary depending on the compound, the disease and its severity and the age, weight, physical condition and responsiveness of the subject to be treated.

The compounds described in the present patent application may form salts. Non-limiting examples of pharmaceutically acceptable salts forming part of this patent application include salts derived from inorganic bases salts of organic bases, salts of chiral bases, salts of natural amino acids and salts of non-natural amino acids.

Certain compounds of the present invention, including compounds of formula, (Ia), and (Ic) are capable of existing in stereoisomeric forms (e.g. diastereomers and enantiomers). The present invention includes these stereoisomeric forms (including diastereomers and enantiomers) and mixtures of them. The various stereoisomeric forms of the compounds of the present invention may be separated from one another by methods known in the art or a given isomer may be obtained by stereospecific or asymmetric synthesis. Tautomeric forms and mixtures of compounds described herein arc also contemplated.

### Pharmaceutical Compositions

The pharmaceutical composition of the present patent application includes at least one compound described herein and at least one pharmaceutically acceptable excipient (such as a pharmaceutically acceptable carrier or diluent). Preferably, the pharmaceutical composition includes the compound(s) described herein in an amount sufficient to inhibit TRPA1 in a subject (e.g., a human). The inhibitory activity of compounds falling within the formulas (I), (Ia), (Ib) and (Ic) may be measured by an assay provided below.

The compound of the present invention may be associated with a pharmaceutically acceptable excipient (such as a carrier or a diluent) or be diluted by a carrier, or enclosed within a carrier which can be in the form of a capsule, sachet, paper or other container.

The pharmaceutical compositions may be prepared by techniques known in the art. For example, the active compound can be mixed with a carrier, or diluted by a carrier, or enclosed within a carrier, which may be in the form of an ampoule, capsule, sachet, paper, or other container. When the carrier serves as a diluent, it may be a solid, semi-solid, or liquid material that acts as a vehicle, excipient, or medium for the active compound. The active compound can be adsorbed on a granular solid container, for example, in a sachet.

The pharmaceutical compositions may be in conventional forms, for example, capsules, tablets, aerosols, solutions, suspensions or products for topical application.

### Methods of Treatment

The compounds and pharmaceutical compositions of the present invention can be administered to treat any disorder, condition, or disease treatable by inhibition of TRP1. For instance, the compounds and pharmaceutical compositions of the present invention are suitable for treatment or prophylaxis of the following diseases, conditions and disorders mediated or associated with the activity of TRPA1 receptors: pain, chronic pain, complex regional pain syndrome, neuropathic pain, postoperative pain, rheumatoid arthritic pain, osteoarthritic pain, back pain, visceral pain, cancer pain, algesia, neuralgia, migraine, neuropathies, chemotherapy - induced neuropathies, eye - irritation, bronchial - irritation, skin - irritation (atopic dcrmatitis), Frost - bites (cold - bite), spasticity, catatonia, catalepsy, parkinsons, diabetic neuropathy, sciatica, HIV-related neuropathy, pust-herpetic neuralgia, fibromyalgia, nerve injury, ischaemia, neurodegeneration, stroke, post stroke pain, multiple sclerosis, respiratory diseases, asthma, cough, COPD, inflammatory disorders, oesophagitis, gastroeosophagal reflux disorder (GERD), irritable bowel syndrome, inflammatory bowel disease, pelvic hypersensitivity, urinary incontinence, cystitis, burns, psoriasis, eczema, emesis, stomach duodenal ulcer and pruritus. The connection between therapeutic effect and inhibition of TRPA1 is illustrated, for example, in Story, G. M. et al. Cell. 2003, 112, 819-829; McMahon, S.B. and Wood, J. N., Cell. 2006, 124, 1123-1125; Voorhoeve, P. M. et al. Cell. 2006, 124, 1169-1181; Wissenbach, U, Niemeyer, B. A. and Flockerzi, V. Biology of the Cell. 2004, 96, 47-54; and the references cited therein.

Pain can be acute or chronic. While acute pain is usually self-limiting, chronic pain persists for 3 months or longer and can lead to significant changes in a patient's personality; lifestyle, functional ability and overall quality of life (K. M. Foley, Pain, in Cecil Textbook of Medicine; J. C. Bennett & F. Plum (eds.), 20th ed., 1996, 100-107). The sensation of pain can be triggered by any number of physical or chemical stimuli and the sensory neurons which mediate the response to this harmful stimulus are termed as "nociceptors". Nociceptors are primary sensory afferent (C and Aδ fibers) neurons that are activated by a wide variety of noxious stimuli including chemical, mechanical, thermal and proton (pH<6) modalities. Nociceptors are the nerves which sense and respond to parts of the body which suffer from damage. They signal tissue irritation, impending injury, or actual injury. When activated, they transmit pain signals (via the peripheral nerves as well as the spinal cord) to the brain.

Chronic pain can be classified as either nociceptive or neuropathic. Nociceptive pain includes tissue injury-induced pain and inflammatory pain such as that associated with arthritis. Neuropathic pain is caused by damage to the sensory nerves of the peripheral or central nervous system and is maintained by aberrant somatosensory processing. The pain is typically well localized, constant and often with an aching or throbbing quality. Visceral pain is the subtype of nociceptive pain that involves the internal organs. It tends to be episodic and poorly localized. Nociceptive pain is usually time limited, meaning when the tissue damage heals, the pain typically resolves (arthritis is a notable exception in that it is not time limited).

### General Methods of Preparation

The compounds of the general formula (I), (Ia), (Ib) and (Ic) and specific examples can be prepared by methods Furthermore, in the following synthetic schemes, where specific acids, bases, reagents, coupling agents, solvents, etc. are mentioned, it is understood that other suitable acids, bases, reagents, coupling agents etc. may be used and are included within the scope of the present invention. The compounds obtained by using the general reaction sequences may be of insufficient purity. These compounds can be purified by using any of the methods for purification of organic compounds known to persons skilled in the art, for example, crystallization or silica gel or alumina column chromatography using different solvents in suitable ratios. All possible stereoisomers are envisioned within the scope of this invention.

A general approach for the synthesis of furopyrimidinyl acetamides of the general formula (I), wherein Z₁, Z₂, R¹, R², R³, U, V and L are as defined above in description is prepared as described in Scheme 1. Coupling reaction of the compounds of the formula (1) (wherein R is hydrogen or alkyl) with amines of the formula (2) in the presence of a suitable coupling agent such as 1-ethyl-3-(3'-dimethylaminopropyl)carbodiimide hydrochloride (EDCI) and base in suitable solvent gives compounds of the formula (3). The selective *N*-alkylation of the compounds of the formula (3) where X is halogen, with suitable alkylating agent of the formula (4) in the presence of base in a suitable solvent gives compounds of the general formula (I).

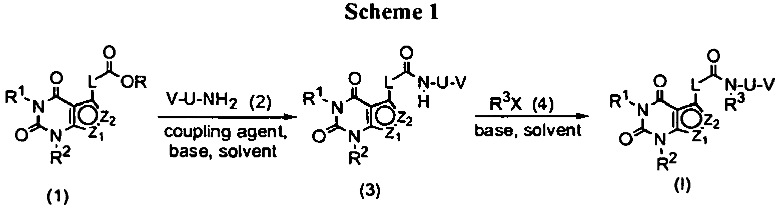

A general approach for the synthesis of furo[2,3-*d*]pyrimidinyl acetamides of formula (Ia) wherein R^{a} is hydrogen and U, V, R¹ and R² are as defined above is prepared as shown is Scheme 2. Coupling reaction of 1,3-dialkylbarbituric acid derivative of the formula (5) with chloroacetyl chloride derivative (6) in the presence of a suitable base like sodium hydride and in a suitable solvent like THF gives 5-(chloroacetyl)-6-hydroxy-1,3-dialkylpyrimidine-2,4(1*H*,3*H*)-dione as an intermediate which on cyclisation in the presence of triethylamine gives furo[2,3-*d*]pyrimidinetrione of formula (7). A similar approach is described by Strekowski, L. et al. in J. Heterocyclic Chem. 2001, 38, 359-363. Furo[2,3-*d*]pyrimidinetrione compounds of formula (7) was converted into furo[2,3-*d*]pyrimidinedione ester of compounds of general formula (8) by reaction with the lithium salt of ethyl acetate (generated from dry ethyl acetate and Lithium bis(trimethylsilyl)amide [LHMDS]) followed by dehydration and isomerization under acidic conditions. Direct coupling of compounds of the general formula (8) with appropriate amines of formula (2) by using suitable base (e.g., sodium hydride) and in suitable solvent (e.g. toluene, tetrahydrofuran) gives compounds of the general formula (Ia). Alternatively, compounds of the general formula (Ia) can be prepared in two steps by hydrolysis of (8) under basic conditions to give the carboxylic acid (9) followed by coupling with amine of the general formula (2) using a suitable coupling agents [e.g., N-ethyl-*N*'-(3-dimethylaminopropyl) carbodiimide hydrochloride (EDCI), benzotriazol-I-yloxytris (dimethylamino) phosphonium hexafluorophosphate [BOP] in suitable solvent or mixture of solvents (e.g., *N*,*N*-dimethyl formamide, tetrahydrofuran, dichloromethane etc. to give compounds of the general formula (Ia).

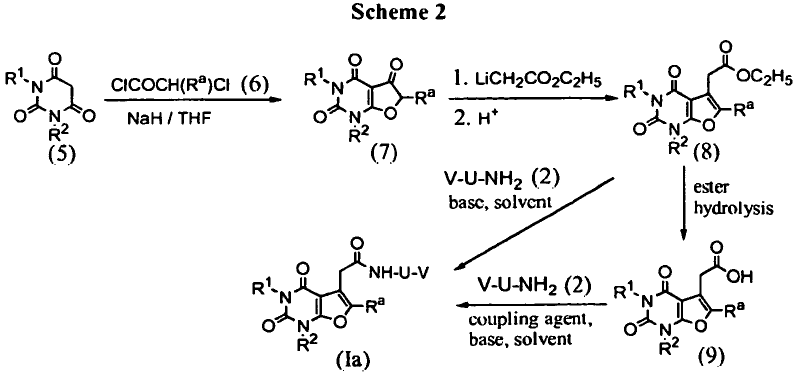

Alternatively, the carboxylic acid intermediate of the general formula (9) can be prepared as described in scheme 3. Thus, reaction of cyanomethyl lithium with furo[2,3-*d*]pyrimidinetrione of formula (7) (where R^{a} is hydrogen or alkyl) in a suitable solvent gives furo[2,3-*d*]pyrimidinyl acetonitrile of the formula (10). This intermediate on hydrolysis under acidic conditions gives intermediates of the formula (9) required for the synthesis of compounds of the general formula (Ia).

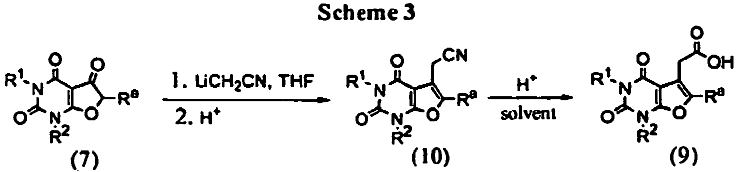

Furo[2,3-*d*]pyrimidinedione ester of the formula (8a) where R^{a} is hydrogen or alkyl can also be prepared through a Wittig reaction on intermediate (7) as shown in Scheme 4. Thus, reaction of intermediate (7) with trimethyl phosphonoacetate in the presence of a suitable base such as sodium hydride, potassium *tert*-butoxide or *n*-butyl lithium in suitable solvent (e.g., tetrahydrofuran, dimethyl sulfoxide gives ester of the formula (11). The ester (11) is then isomerized under acidic conditions to give intermediates of the formula (8a) useful for the preparation of compounds of the present invention represented by the formula (Ia).

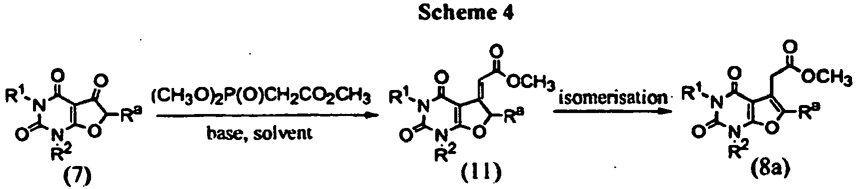

Another approach for the preparation of intermediates of the general formula (8b) is given in Scheme 5. Thus, Reformatsky reaction on intermediate (7) (where R^{a} is hydrogen or alkyl) by using BrZnCH₂CO₂R where R is alkyl (prepared from Zn and BrCH₂CO₂R) followed by in-situ dehydration of the carbinol intermediate gives intermediate (12). Double bond isomerization of intermediate (12) under acidic conditions gives the ester intermediate (8b).

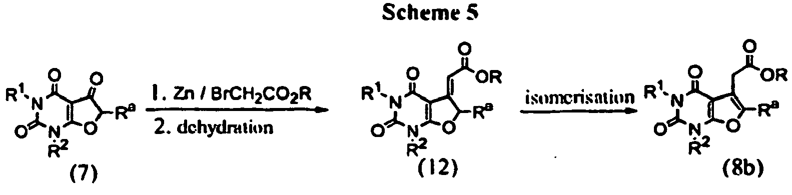

General approach for the synthesis of compound of formula (Ic) (wherein R^{a} is hydrogen or methyl and R¹, R², R⁴, R⁵, R⁶, R⁷, R⁸ and R⁹ are as defined above) is depicted in scheme 6. Direct coupling of compounds of the general formula (8) with appropriate amines of formula (23) by using suitable base (e.g., sodium hydride) and in suitable solvent (e.g. toluene, tetrahydrofuran) gives compounds of the general formula (Ic). Alternatively, compounds of the general formula (Ic) can be prepared in two steps by hydrolysis of (8) under basic conditions to give the carboxylic acid (9) followed by its coupling with amine of the general formula (23) using a suitable coupling agents [e.g., N-ethyl-*N*'-3-dimethylaminopropyl) carbodiimide hydrochloride (EDCI), benzotriazol-1-yloxytris (dimethylamino) phosphonium hexafluorophosphate [BOP]) in suitable solvent or mixture of solvents (e.g., *N,N*-dimethyl formamide, tetrahydrofuran, dichloromethane etc. to give compounds of the general formula (Ic).

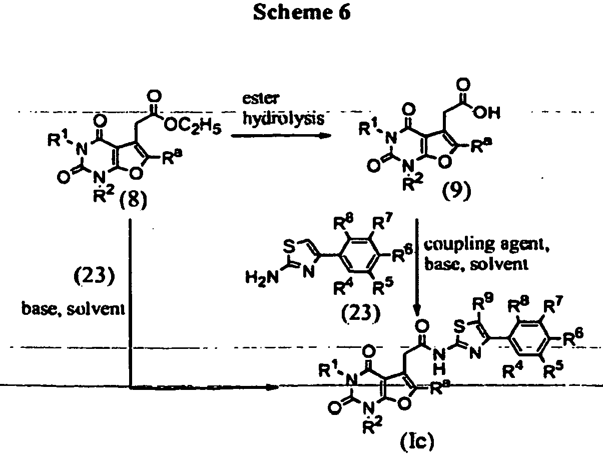

A general approach for the synthesis of furo[3,4-*d*]pyrimidinyl acetamides of formula (Ib) wherein R^{a}, U, V, R¹ and R² are as defined above is prepared as shown is Scheme 7. 5-Methyl-*4H*-furo[3,4-*d*][1,3]oxazine-2,4(1*H*)-dione of the formula (13) can be prepared according to the procedure described by Press, J. B., et al. Eur. J. Med. Chem. 1989, 24, 627-630 starting from dialkyl 2-methylfuran-3,4-dicarboxylate. The intermediate of the formula (1) is treated with alkyl amine of the formula R¹-NH₂ to give the urea derivative (14) through a ring opening reaction. Cyclisation of intermediate (14) using 1,1'-carbonyldiimidazole (CDI) in suitable solvent followed by alkylation with R²X in the presence of a suitable solvent gives 3,5-dialkylfuro[3,4-*d*]pyrimidine-2,4(1*H*,3*H*)-dione of the formula (16) via intermediate (15). Intermediate (16) is deprotonated with a strong base such as sodium hydride or n-butyl lithium and reacted with dialkyl carbonate to give the ester (17). Coupling reaction of ester of the formula (17) with appropriate amines of formula (2) in the presence of a suitable base such as sodium hydride in the presence of suitable solvent such as dry toluene or xylene gives compounds of general formula (Ib).

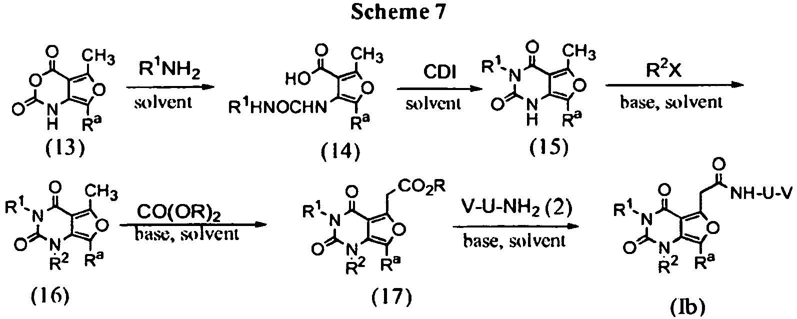

A general approach for the synthesis of substituted 2-amino-4-aryl thiazoles of the formula (23) (wherein R⁴, R⁵, R⁶, R⁷, R⁸ and R⁹ are as defined above) from benzoic acid derivatives is given in Scheme 8. Thus, benzoic acid of formula (18) was converted to the corresponding acid chloride of formula (19) using oxalyl chloride in the presence of catalytic amounts of DMF in dry dichloromethane. The acid chloride of formula (19) was converted to corresponding Weinerb amide of formula (21) by treating with *N,O-*dimethylhydroxylamine hydrochloride of formula (20) in the presence of a suitable base such as triethylamine. The addition of methyl magnesium iodide to Weinreb amide of formula (21) afforded acetophenone derivative of formula (22).

Conversion of acetophenone derivative of formula (22) to 2-amino-4-aryl thiazole of the formula (23) can be effected by two approaches (Scheme 8). In the first case acetophenone was converted to the corresponding phenacyl bromide, which in turn was reacted with thiourea in a suitable solvent such as tetrahydrofuran at refluxing condition. Alternatively, acetophenone derivative of formula (22) can be converted to 2-amino-4-aryl thiazole (23) in one step by its reaction with thiourea and iodine in refluxing ethanol (Carroll, K. et al. J. Am. Chem. Soc. 1950, 3722 and Naik, S. J.; Halkar, U. P., ARKIVOC 2005, xiii, 141-149).

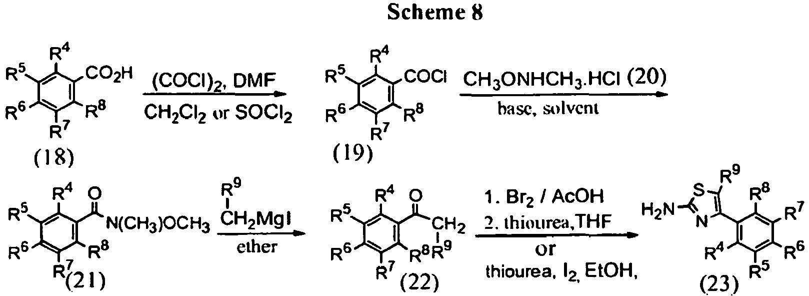

### EXPERIMENTAL

Unless otherwise stated, work-up includes distribution of the reaction mixture between the organic and aqueous phase indicated within parentheses, separation of layers and drying the organic layer over sodium sulphate, filtration and evaporation of the solvent. Purification, unless otherwise mentioned, includes purification by silica gel chromatographic techniques, generally using ethyl acetate/petroleum ether mixture of a suitable polarity as the mobile phase. Use of a different cluent system is indicated within parentheses. The following abbreviations are used in the text: DMSO-*d*₆: Hexadeuterodimethyl sulfoxide; DMF: *N*,*N*-dimethylformamide; J: Coupling constant in units of Hz; RT or rt: room temperature (22-26°C); Aq.: aqueous; AcOEt: ethyl acetate; equiv. or eq.: equivalents.

### INTERMEDIATES

### Intermediate I

### Ethyl (1,3-dimethyl-2,4-dioxo-1,2,3,4-tetrahydrofuro[2,3-d]pyrimidin-5-yl)acetate

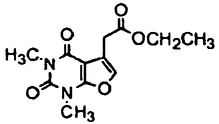

Step 1: Ethyl (2Z)-(1,3-dimethyl-2,4-dioxo-1,2,3,4-tetrahydrofuro[2,3-*d*]pyrimidin-5(6*H*)-ylidene)acetate OR Ethyl (5-hydroxy-1,3-dimethyl-2,4-dioxo-1,2,3,4,5,6-hexahydrofuro[2,3-*d*]pyrimidin-5-yl)acetate: Anhydrous THF (50.0 ml) was cooled to - 78°C. A solution of lithium bis(trimethylsilyl)amide (LiHMDS) (1.0 M in THF, 4.692 g, 28.038 mmol) was added under nitrogen atmosphere. Then, dry ethyl acetate (2.358 g, 26.763 mmol) was added and the resulting mixture was stirred at the same temperature for I h. A solution of 1,3-dimethylfuro[2,3-*d*]pyrimidine-2,4,5(1*H*,3*H*,6*H*)-trione (5.0 g, 25.489 mmol) in a mixture of THF and dichloromethane (1:3, 80.0 ml) was added dropwise to the above solution and the resulting mixture was stirred at -78°C for 2.5 h. The reaction mixture was then acidified with IN HCl (75.0 ml). The temperature was allowed to rise gradually until reaching room temperature and was extracted with ethyl acetate. The organic extracts were washed with brine (50.0 ml), dried over anhydrous Na₂SO₄ and filtered. The filtrate was evaporated under reduced pressure and the residue obtained was purified with column chromatography by using 2 % methanol in chloroform to obtain 2.1 g of ethyl (2Z)-(1,3-dimethyl-2,4-dioxo-1,2,3,4-tetrahydrofuro[2,3-*d*]pyrimidin-5(6*H*)-ylidene)acetate as a white solid: ¹H NMR (300 MHz, CDCl₃) 8 1.29 (t, *J* = 7.2 Hz, 3H), 3.35 (s, 3H), 3.45 (s, 3H), 4.17 (q, *J* = 7.5 Hz, 2H), 6.37 (s, 1H). Further elution with increasing amounts of methanol in chloroform gives 750 mg of ethyl (5-hydroxy-1,3-dimethyl-2,4-dioxo-1,2,3,4,3,6-hexahydrofuro[2,3-*d*]pyrimidin-5-yl)acetate as a light brown solid; ¹H NMR (300 MHz, CDCl₃) δ 1.33 (t, *J* = 7.2 Hz, 3H), 3.39 (s, 3H), 3.54 (s, 3H), 4.08 (s, 2H), 4.27 (q, *J*= 7.5 Hz, 2H), 4.77 (s, 1H); APCI-MS (*m*/*z*) 267.09 (M+H)⁺.
Step 2: Ethyl (1,3-dimethyl-2,4-dioxo-1,2,3,4-tetrahydrofuro[2,3-*d*]pyrimidin-5-yl)acetate: A mixture of Step I intermediate (1.1 g, 4.131 mmol) and glacial acetic acid (20.0 ml) was refluxed for 4 h. Excess of acid was evaporated under vacuum and the residue obtained after evaporation of the solvent was purified by silica gel column chromatography using 2 % methanol in chloroform to obtain 930 mg of the product as a white solid: ¹H NMR (300 MHz, CDCl₃) δ 1.19 (t, *J* = 7.5 Hz, 3H), 3.18 (s, 3H), 3.41 (s, 3H), 3.68 (s, 2H), 4.09 (q, *J* = 7.2 Hz, 2H), 7.58 (s, 1H); APCI-MS (*m*/*z*) 267.13 (M+H)⁺.

### Intermediate 2

### (1,3-Dimethyl-2,4-dioxo-1,2,3,4-tetrahydrofuro[2,3-d]pyrimidin-5-yl)acetic acid

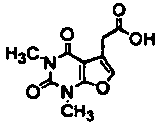

To a stirred solution of Intermediate 1 (150 mg, 0.563 mmol) in a mixture of THF (2.0 ml) and water (1.0 ml) was added LiOH.H₂O (36 mg, 0.845 mmol). The mixture was stirred for 30 min. at room temperature. Residue obtained after evaporation of the solvent was acidified with 1 N HCl (pH 4) and extracted with ethyl acetate (2 x 50 ml). The combined ethyl acetate extracts were washed with water (2 x 15 ml) and dried (Na₂SO₄). Evaporation of solvent under reduced pressure afforded 70 mg of the product as an off-white solid; ¹H NMR (300 MHz, CDCl₃) δ 3.19 (s, 3H), 3.50 (s, 3H), 3.60 (s, 2H), 7.56 (s, 1H), 12.43 (br s, 1H); APCI-MS (*m*/*z*) 239.06 (M+H)⁺.

### Intermediate 3

### Ethyl (1,3,6-trimethyl-2,4-dioxo-1,2,3,4-tetrahydrofuro[2,3-d]pyrimidin-5-yl)acetate

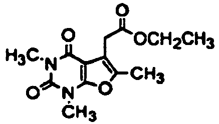

The title compound was prepared according to the procedure described in Intermediate 1 by using 1,3,6-trimethylfuro[2,3-*d*]pyrimidine-2,4,5(1*H*,3*H*,6*H*)-trione (5.0 g, 23.788 mmol) in the place of 1,3-dimethylfuro[2,3-*d*]pyrimidine-2,4,5(1*H*,3*H*,6*H*)-trione in the first step to give 1.565 g of the product as a white solid; ¹H NMR (300 MHz, CDCl₃) δ 1.28 (t, *J* = 7.5 Hz, 3H), 2.28 (s, 3H), 3.35 (s, 3H), 3.52 (s, 3H), 3.64 (s, 2H), 4.19 (q, *J*= 7.2 Hz, 2H); APCI-MS-(*m*/*z*) 281.05 (M+H)⁺.

### Intermediate 4

### (1,3,6-trimethyl-2,4-dioxo-1,2,3,4-tetrahydrofuro[2,3-d]pyrimidin-5-yl)acetic acid

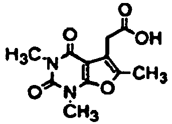

Saponification of Intermediate 3 (150 mg, 0.535 mmol) using LiOH (34 mg, 0.802 mmol) in a mixture of THF (4.0 ml) and water (1.0 ml) as described in Intermediate 2 afforded 60 mg of the title compound as a white solid; ¹H NMR (300 MHz, CDCl₃) δ 2.27 (s, 3H), 3.17 (s, 3 H), 3.39 (s, 3H), 3.55 (s, 2H), 12.32 (br s, 1H); APCI-MS (*m*/*z*) 253.09 (M+H)⁺.

### General procedure for the preparation of 2-amino-4-aryl thiazoles:

### Method 1:

A solution of acetophenone derivative (1.0 cquiv) in glacial acetic acid (5 vol) was added liquid bromine (1.0 cquiv) at 0 °C and reaction mixture was stirred at room temperature for 2h. The reaction mixture was diluted with water and extracted with ethyl acetate, washed with brine and dried over Na₂SO₄. The crude product obtained upon concentration was dissolved in dry THF (10 volumes) and thiourea (2.0 equiv) was added and refluxed overnight. The reaction mixture was diluted with ethyl acetate, washed with sodium thiosulfate solution and organic layer was treated with 1N HCl to result in salt formation of the amine. The precipitated salt was collected by filtration. The salt was then treated with saturated solution of NaHCO₃ to re-generate the amine. The mixture was extracted with dichloromethane (2 x 50 ml) and the combined organic extracts were washed with water and brine. The solvent was evaporated under reduced pressure to afford the 2-amino-4-aryl thiazole derivative.

### Method 2:

A solution of acetophenone derivative (1.0 equiv.), thiourea (2.0 equiv.) and iodine (1.0 equiv.) in dry ethanol (5 volumes) was refluxed for 24 h. The reaction mixture was diluted with ethyl acetate and the layers were separated. The organic layer was washed with sodium thiosulfate solution to remove iodine. The ethyl acetate solution was treated with IN HCl and precipitated salt collected by filtration. The free amine was regenerated as described in Method I given above.

All the 2-amino-4-aryl thiazole derivatives were prepared by either Method I or Method 2 starting from appropriate aryl alkyl ketones. Structure information and characterization data for selected intermediates are given in Table 1.

**Table 1: Structural details and ¹H NMR data of selected 2-aminothiazole intermediates**

| **Sr. No.** | **Structure** | **Mol. Formula (Mol. Wt.)** | **¹H NMR (δ ppm, 300 MHz)** |
|---|---|---|---|
| 1. | 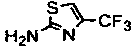 | C₄H₃F₃N₂S 168.14 | DMSO-d₆: 7.26 (s, 1H), 7.42 (br. s, 2H) |
| 2. | 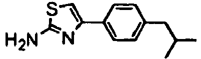 | C₁₃H₁₆N₂S 232.25 | DMSO-d₆: 0.86 (d, *J* = 6.6 Hz, 6H), 1.86-1.76 (m, 1H), 2.43 (d, *J* = 6.9 Hz, 2H), 6.92 (s, I H), 7.03 (br s, 2H), 7.13 (d, *J* = 8.1 Hz, 2H), 7.68 (d, *J* = 7.8 Hz, 2H) |
| 3. | 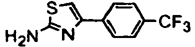 | C₁₀H₇F₃N₂S 244.24 | DMSO-d₆: 7.16 (br s, 2H), 7.24 (s, 1H), 7.69 (d, *J* = 8.1 Hz, 2H), 7.97 (d, *J*= 7.8 Hz, 2H) |
| 4. | 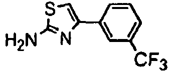 | C₁₀H₇F₃N₂S 244.24 | COCl₃: 5.02 (br. s, 2H), 6.79 (s, 1H), 7.50-7.42 (m, 2H), 7.91 (d. *J* = 6.9 Hz, 1H), 8.12-8.06 (m, 1H) |
| 5. | 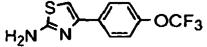 | C₁₃H₁₆(N₂S 260.24 | CDCl₃: 7.09 (s, 1H), 7.13 (br.s, 2H), 7.35 (d, *J* = 8.4, 2H); 7.90 (d, *J* = 8.1, 2H). |
| 6. | 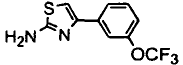 | C₁₀H₆F₄N₂S 262.24 | CDCl₃: 5.08 (br s, 2H), 6.75 (s, 1H), 7.10 (d, *J* = 7.8 Hz, 1H), 7.36 (t, *J* = 7.8 Hz, 1H), 7.68-7.61 (m, 2H) |
| 7. | 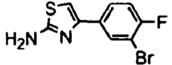 | C₉H₆BrFN₂S 273.13 | DMSO-d₆: 7.12-7.18 (m, 1H + 2H), 7.37 (t, *J* = 9.0 Hz, 1H), 7.80-7.86 (m, 1H), 8.06-8.12 (m, 1H) |
| 8. | 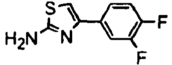 | C₉H₆F₂N₂S 212.22 | CDCl₃: 4.98 (br s, 2H), 6.66 (s, 1H), 7.18-7.07 (m, 1H), 7.48-7.43 (m, 1H), 7.60-7.53 (m, 1H) |
| 9. | 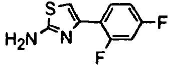 | C₉H₆F₂N₂S 212.22 | CDCl₃: 5.04 (br s, 2H), 6.93-6.80 (m, 3H), 8.04-7.95 (m, 1H) |
| 10. | 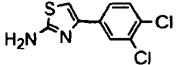 | C₉H₆Cl₂N₂S 245.13 | CDCl₃: 7.85 (s, 1H); 7.56 (dd, *J* = 8.4 Hz, 2.1, 1H); 7.39 (d, *J* = 8.4 Hz, 1H); 6.72 (s, 1H); 5.01 (br s, 2H). |
| 11. | 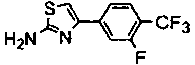 | C₁₀H₆F₄N₂S 262.23 | DMSO-d₆: 7.24 (br s, 2H), 7.40 (s, 1H), 7.88-7.73 (m, 3H) |
| 12. | 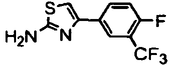 | C₁₀H₆F₄N₂S 262.23 | DMSO-d₆: 7.20 (br. s, 2H), 7.24 (s, 1H), 7.52 (t, *J* = 8.7 Hz, 1H), 8.13 (d, *J* = 6.0 Hz, 2H) |
| 13. | 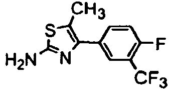 | C₁₁H₈F₄N₂S 276.25 | DMSO-d₆: 2.34 (s, 3H), 6.92 (br s, 2H), 7.53 (t, J= 9.6 Hz, 1H), 7.88-7.94 (m, 2H) |
| 14. | 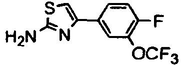 | C₁₀H₆F₄N₂OS 278.23 | DMSO-d₆: 7.18 (br. s, 3H), 7.50 (t, *J* = 8.7 Hz, 1H), 7.92-7.85 (m, 2H) |
| 15. | 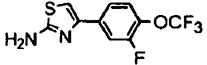 | C₁₀H₆F₄N₂OS 278.23 | DMSO-d₆: 7.18 (br.s, 2H), 7.24 (s, 1H), 7.55 (d, *J* = 8.1 Hz, 1H), 7.73 (d, *J* = 8.7 Hz, 1H), 7.87-7.80 (m, 1H) |
| 16. | 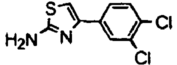 | C₉H₆Cl₂N₂S 245.13 | CDCl₃: 5.01 (br.s, 2H), 6.72 (s, I H), 7.39 (d, *J =* 8.4 Hz, 1H), 7.56 (dd, *J* = 8.4 Hz, 2.1 Hz, 1H), 7.85 (s, 1H) |
| 17. | 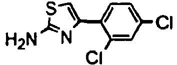 | C₉H₆Cl₂N₂S 245.13 | CDCl₃: 5.02 (br s, 2H), 7.26 (s, 1H); 7.06 (s, 1H), 7.42 (s, 1H), 7.72 (d, *J* = 8.1 Hz, 1H) |
| 18. | 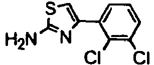 | C₉H₆Cl₂N₂S 245.13 | DMSO-*d*₆: 7.04-7.08 (m, 1H+2H), 7.38 (t, *J* = 7.8 Hz, 1H), 7.58 (d, *J*= 6.9 Hz, 1H), 7.73 (d, *J* = 7.8 Hz, 1H) |
| 19. | 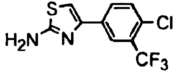 | C₁₀H₆,ClF₃N₂S 278.69 | CDCl₃: 5.05 (br. s, 2H), 6.78 (s, 1H), 7.46 (d. *J* = 8.1 Hz, 1H), 7.82 (d, *J* = 7.8 Hz, 1H), 8.08 (s, I H) |
| 20. | 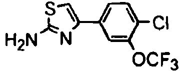 | C₁₀H₆ClF₃N₂OS 278.68 | DMSO-*d*₆: 7.20 (brs, 2H), 7.27 (s, 1H), 7.67 (d, *J* = 8.4 Hz, 1H), 7.82-7.91 (m, 2H) |
| 21. | 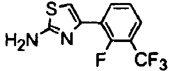 | C₁₀H₆F₄N₂S 262.23 | CDCl₃: 5.04 (br s, 2H), 7.10 (s, 1H), 7.27 (t, *J* = 7.5 Hz, 1H), 7.51 (t, *J* = 6.9 Hz, 1H), 8.28-8.21 (m, 1H) |
| 22. | 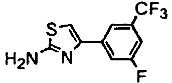 | C₁₀H₆F₄N₂OS 263.23 | DMSO-d₆: 7.23 (br. s, 2H), 7.41 (s, 1H), 7.62-7.52 (m, 1H), 7.97-7.87 (m, I H) |
| 23. | 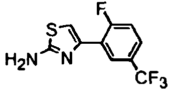 | C₁₀H₆F₄N₂S 262.23 | CDCl₃: 7.13 (s, 1H), 7.26 (br.s, 2H), 7.58-7.50 (m, 1H), 7.73-7.65 (m, 1H), 8.36-8.29 (m, 1H) |
| 24. | 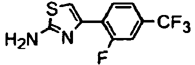 | C₁₀H₆F₄N₂S 262.23 | CDCl₃: 5.00 (br. s, 2H), 7.13 (s, 1H), 7.37-7.30 (m, 1H), 7.42 (d, *J* = 7.8 Hz, 1H), 8.15 (t, *J* = 7.2 Hz, 1H) |
| 25. | 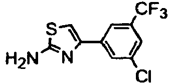 | C₁₀H₆ClF₃N₂S 278.68 | DMSO-*d*₆: 7.25 (br. s, 2H), 7.46 (s, 1H), 7.73 (s, 1H), 8.10 (s, 1H), 8.17 (s, 1H) |
| 26. | 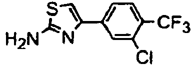 | C₁₀-H₆ClF₃N₂S 278.68 | DMSO-*d*₆: 7.25 (br s, 2H), 7.43 (s, 1H), 7.84 (d, *J* = 8.4 Hz, 1H), 7.94 (d, *J* = 8.4 Hz, 1H), 8.09 (s, 1H) |
| 27. | 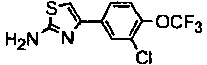 | C₁₀H₆ClF₃N₂OS 293.98 | DMSO-*d*₆: 7.19 (br s, 2H), 7.57 (d, *J* = 8.1 Hz, 1H), 7.86 (d, *J* = 8.1 Hz, 1H), 8.05 (s, 1H) |
| 28. | 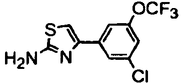 | C₁₀H₆ClF₃N₂OS 293.98 | DMSO-*d*₆: 7.21 (br s, 2H), 7.36 (s, 1H), 7.41 (s, 1H), 7.72 (s, 1H), 7.90 (s, 1H) |
| 29. | 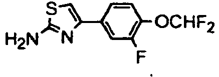 | C₁₀H₇F₃N₂OS 260.24 | DMSO-d₆: 7.12 (br s, 3H), 7.23 (t, *J* = 73.2Hz, 1H), 7.33 (t, *J* = 8.1 Hz, 1H), 7.75-7.62 (m, 2H) |
| 30. | 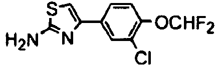 | C₁₀H₇ClF₂N₂OS 275.99 | DMSO-d₆: 6.94 (s, 1H), 7.04-7.15 (m, 3H), 7.61-7.51 (m, 2H) |
| 31. | 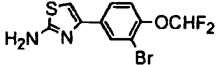 | C₁₀H₇BrF₂N₂OS 321.14 | DMSO-d₆: 7.13 (br s, 3H), 7.28 (t, *J* = 73.2 Hz, 1H), 7.33 (d, *J* = 8.1 Hz, 1H), 7.84 (d, *J* = 8.1 Hz, 1H), 8.13 (s, 1H) |
| 32. | 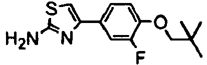 | C₁₄H₁₇FN₂OS 280.36 | DMSO-*d*₆: 1.00 (s, 9H), 3.71 (s, 2H), 6.95 (s, 1H), 7.05 (br s, 2H), 7.15 (t, *J* = 8.7 Hz, 1H), 7.52-7.62 (m, 2H) |
| 33. | 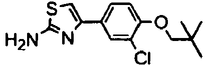 | C₁₄H₁₇ClN₂OS 296.82 | DMSO-*d*₆: 1.03 (s, 9H), 3.72 (s, 2H), 6.97 (s, 1H), 7.07-7.15 (m, 1H + 2H), 7.70 (d, *J* = 9.0 Hz, 1H), 7.84 (s, 1H) |
| 34. | 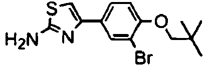 | C₁₄H₁₇BrN₂OS 341.27 | DMSO-*d*₆: 1.04 (s, 9H), 3.37 (s, 2H), 7.00 (s, 1H), 7.05 (br s, 2H), 7.70-7.76 (m, 2H), 7.99 (s, 1H) |
| 35. | 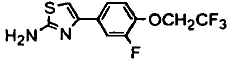 | C₁₁H₈F₄N₂OS 292.25 | CDCl₃: 7.57-7.46 (m, 2H), 7.02 (t, *J* = 8.4, 1H); 6.66 (s, 1H); 5.08 (br s, 2H); 4.43 (q, *J* = 8.4 Hz, 2H) |
| 36. | 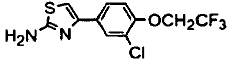 | C₁₁H₈ClF₃N₂OS 308.71 | DMSO-d₆: 4.88 (q, *J* = 8.7 Hz, 2H), 7.07-7.11 (m, 1H+2H), 7.27 (d, *J* = 8.7 Hz, 1H), 7.75 (d, *J* = 6.9 Hz, I H), 7.89 (s, 1H) |
| 37. | 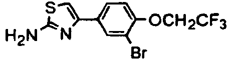 | C₁₁H₈BrF₃N₂OS 353.16 | DMSO-d₆: 4.87 (q, *J* = 8.7 Hz, 2H), 7.07-7.15 (m, 1H+2H), 7.23 (d, *J* = 8.7 Hz, 1H), 7.79 (d, *J* = 6.9 Hz, 1H), 8.05 (s, 1H) |
| 38. | 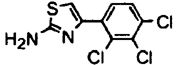 | C₉H₅Cl₃N₂S 297.57 | DMSO-*d*₆: 7.10-7.18 (m, 1H + 2H), 7.67 (d, *J* = 9.0 Hz, 1H), 7.79 (d, *J* = 8.7 Hz, 1H) |
| 39. | 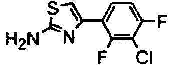 | C₉H₅ClF₂N₂S 245.98 | DMSO-*d*₆: 7.01 (s, 1H), 7.19 (br s, 2H), 7.365 (t, *J* = 8.7 Hz, 1H), 7.95 (q, *J* = 6.9 Hz, 1H) |
| 40. | 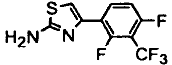 | C₁₀H₅F₅N₂S 280.22 | DMSO-d₆: 7.05 (s, 1H), 7.21 (br s, 2H), 7.43 (t, *J* = 9.0 Hz, 1H), 8.35-8.23 (m, 1H) |
| 41. | 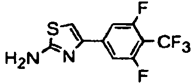 | C₁₀H₅F₅N₂S 280.22 | DMSO-d₆: 7.29 (br s, 2H), 7.52 (s, 1H), 7.72 (d, *J* = 11.7 Hz, 2H) |
| 42. | 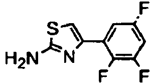 | C₉H₅F₃N₂S 230.21 | DMSO-d₆: 7.00 (s, 1H), 7.20 (br s, 2H), 7.55-7.67 (m, 1H), 7.80-7.90 (m, 1H). |
| 43. | 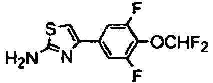 | C₁₀H₆F₄N₂OS 278.23 | DMSO-d₆: 7.20 (br s, 2H), 7.24 (t, *J* = 72.3 Hz, 1H), 7.48 (s, 1H), 7.65 (d, *J* = 9.0 Hz, 2H) |
| 44. | 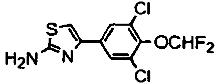 | C₁₀H₆Cl₂F₂N₂OS 311.14 | DMSO-d₆: 7.14 (t, *J* = 71.7 Hz, 1H), 7.20 (br s, 2H), 7.33 (s, 1H), 7.56 (s, 2H). |
| 45. | 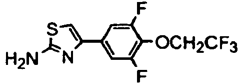 | C₁₁H₇F₅N₂OS 310.24 | DMSO-d₆: 4.82 (q, *J* = 9.0 Hz, 2H), 7.16 (br s, 2H), 7.21 (s, 1H), 7.55 (s, 1H), 7.59 (s, 1H) |
| 46. | 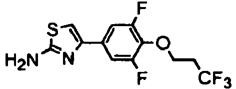 | C₁₂H₉F₅N₂OS 324.27 | DMSO-d₆: 2.50 (s, 2H), 4.80 (q, *J* = 8.7 Hz, 2H), 7.16-7.22 (m, 1H+2H), 7.58 (d, *J* = 9.0 Hz, 1H) |
| 47. | 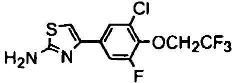 | C₁₁H₇ClF₄N₂OS 326.70 | DMSO-d₆: 4.80 (q, *J* = 8.7 Hz, 2H), 7.17 (br s, 2H), 7.23 (s, 1H) 7.67-7.77 (m, 2H) |
| 48. | 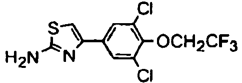 | C₁₁H₇Cl₂F₃N₂OS 343.15 | DMSO-d₆: 4.72 (q, *J* = 8.7 Hz, 2H), 7.18 (br s, 2H), 7.28 (s, 1H) 7.91 (s, 2H) |
| 49. | 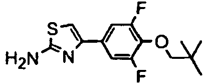 | C₁₄H₁₆F₂N₂OS 298.35 | DMSO-*d*₆: 1.00 (s, 9H), 3.76 (s, 2H), 7.12-7.18 (m, 1H+2H), 7.48-7.58 (m, 2H) |
| 50. | 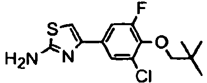 | C₁₄H₁₆ClFN₂OS 314.81 | DMSO-*d*₆: 1.02 (s, 9H), 3.74 (s, 2H), 7.12-7.18 (m, 1H+2H), 7.61-7.68 (m, 1H), 7.71 (s, 1H) |
| 51. | 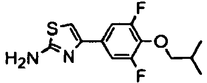 | C₁₃H₁₄F₂N₂OS 284.33 | DMSO-*d*₆: 0.97 (d, *J* = 6.6 Hz, 6H), 1.94-2.00 (m, 1H), 3.87 (d, *J* = 6.3 Hz, 2H), 7.12-7.18 (m, 1H+2H), 7.22 (s, 1H), 7.87 (s, 1H) |
| 52. | 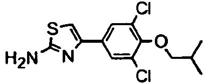 | C₁₃H₁₄Cl₂N₂OS 317.23 | DMSO-*d*₆: 1.04 (d, *J* = 6.6 Hz, 6H), 1.98-2.14 (m, 1H), 3.75 (d, *J* = 6.3 Hz, 2H), 7.12-7.18 (m, 1H+2H), 7.50 (s, 1H), 7.55 (s, 1H) |
| 53. | 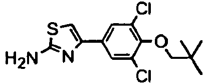 | C₁₄H₁₆ClN₂OS 331.26 | DMSO-*d*₆: 1.07 (s, 9H), 3.67 (s, 2H), 7.90 (s, 1H), 7.99 (s, 2H), 12.72 (br s, 2H) |
| 54. | 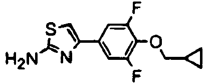 | C₁₃H₁₂F₂N₂OS 282.31 | CDCl₃: 0.31-0.25 (m, 2H), 0.61-0.56 (m, 2H), 1.28-1.22 (m, 1H), 3.97 (d, *J* = 6.9 Hz, 2H), 5.01 (br s, 2H), 6.66 (s, 1H), 7.29 (s, 1H), 7.33 (s, 1H) |
| 55. | 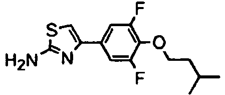 | C₁₄H₁₆F₂N₂OS 298.35 | DMSO-*d*₆: 0.92 (d, *J* = 6.6 Hz, 6H), 1.58 (q, *J* = 6.6 Hz, 2H), 1.75-1.83 (m, 1H), 4.08-4.15 (m, 2H), 7.10-7.16 (m, 3H), 7.48-7.55 (m, 2H) |
| 56. | 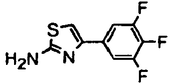 | C₉H₅F₃N₂S 230.21 | DMSO-*d*₆: 7.16-7.21 (m, 1H+2H), 7.63-7.73 (m, 2H) |
| 57. | 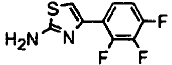 | C₉H₅F₃N₂S 230.21 | DMSO-*d*₆: 6.99 (s, 1H), 7.20 (br s, 2H), 7.35 (q, *J* = 9.3 Hz, 1H), 7.33 (q, *J* = 6.6 Hz, 1H) |
| 58. | 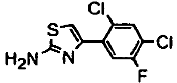 | C₉H₅Cl₂FN₂S (263.12) | CDCl₃: 4.97 (br s, 2H), 7.19 (s, I H), 7.44 (d, *J* = 6.9 Hz, 1H), 7.74 (d, *J* = 9.0 Hz, 1H) |

Further it should also be noted that several fluoro substituted 2-amino-4-aryl thiazoles can be prepared using the approach described in Scheme 8 by following Method I or Method 2 starting from appropriate fluorinated benzoic acid or fluroninated acetophenone. A few examples of such aminothiazole intermediates are given in Table 2.

**Table 2: Structure of fluoro substituted 2-amino-4-arylthiazoles**

| **S No** | **Structure** | **Name** | **Mol. Formula** | **(Mol. Wt.)** |
|---|---|---|---|---|
| 1. | 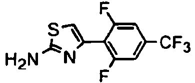 | 4-[2,6-difluoro-4-(trifluoromethyl)phenyl ]-1,3-thiazol-2-amine | C₁₀H₅F₅N₂S | 280.22 |
| 2. | 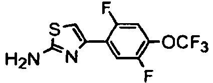 | 4-[2,5-difluro-4-(trifluoromethoxyphen yl]-1,3-thiazol-2-amine | C₁₀H₅F₅N₂OS | 296.22 |
| 3. | 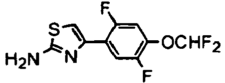 | 4-[2,5-difluoro-4-(difluoromethoxypheny 1]-1,3-thiazol-2-amine | C₁₀H₆F₄N₂OS | 278.23 |
| 4. | 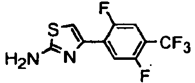 | 4-[2,5-difluoro-4-(trifluoromethyl)phenyl ]-1,3-thiazol-2-amine | C₁₀H₅F₅N₂S | 280.22 |
| 5. | 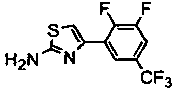 | 4-[2,3-difluoro-5-(trifluoromethyl)phenyl ]-1,3-thiazol-2-amine | C₁₀H₅F₅N₂S | 280.22 |
| 6. | 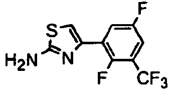 | 4-[2,5-difluoro-3-(trifluoromethyl)phenyl ]-1,3-thiazol-2-amine | C₁₀H₅F₅N₂S | 280.22 |
| 7. | 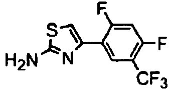 | 4-[2,4-difluoro-5-(trifluoromethyl)phenyl ]-1,3-thiazol-2-amine | C₁₀H₅F₅N₂S | 280.22 |
| 8. | 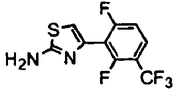 | 4-[2,6-difluoro-3-(trifluoromethyl)phenyl ]-1,3-thiazol-2-amine | C₁₀H₅F₅N₂S | 280.22 |
| 9. | 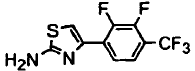 | 4-[2,3-difluoro-4-(trifluoromethyl)phenyl ]-1,3-thiazol-2-amine | C₁₀H₅F₅N₂S | 280.22 |
| 10. | 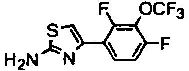 | 4-[2,4-difluoro-3-(trifluoromethoxy)phen yl]-1,3-thiazol-2-amine | C₁₀H₅F₅N₂OS | 296.22 |
| 11. | 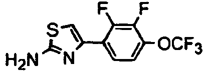 | 4-[2,3-difluoro-4-(trifluoromethoxy)phen yl]-1,3-thiazol-2-amine | C₁₀H₅F₅N₂OS | 296.22 |
| 12. | 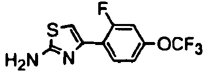 | 4-[2-fluoro-4-(trifluoromethoxy)phen yl]-1,3-thiazol-2-amine | C₁₀H₆F₄N₂OS | 278.23 |
| 13. | 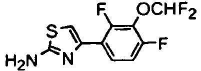 | 4-[2,4-difluoro-3-(difluoromethoxy)phen yl]-1,3-thiazol-2-amine | C₁₀H₆F₄N₂OS | 278.23 |
| 14. | 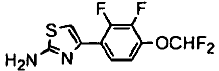 | 4-[2,3-difluoro-4-(difluoromethoxy)phen yl]-1,3-thiazol-2-amine | C₁₀H₆F₄N₂OS | 278.23 |
| 15. | 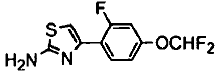 | 4-[2-fluoro-4-(difluoromethoxy)phen yl]-1,3-thiazol-2-amine | C₁₀H₇F₃N₂S | 260.24 |

For further illustration of methods of preparing the compounds of the present invention, the following examples are disclosed below.

### Examples

### General procedure

### Method A:

To a stirred solution of appropriate amine (1.2 equiv.) in dry toluene, sodium hydride (1.4 equiv.) was added and after stirring for 30 min at room temperature, furopyrimidinyl acetic acid ester derivative (1.0 equiv.) was added and heated to reflux for 48 h. The reaction mixture was concentrated and the residue obtained was acidified to pH 6.0 by addition of I N hydrochloric acid and extracted with ethyl acetate. The combined organic layers were dried (Na₂SO₄). The residue obtained after evaporation of the solvent was purified by silica gel column chromatography using suitable ethyl acetate and petroleum ether mixture to obtain the desired products.

### Method B:

To a stirred solution of furopyrimidinyl acetic acid derivative (1.0 equiv.) in 1,2-dichloroethane (5 vol.) was added EDCI (1.2 equiv.), HOBt (0.3 equiv.) and 4-dimethylaminopyridine (0.1 equiv.) and the mixture was stirred at room temperature for 10-15 min. An appropriate amine (1.0 equiv.) was then added and mixture was stirred at the same temperature for 48 h. Excess of solvent was evaporated and the residue obtained was diluted with ethyl acetate and 2 N HCl. Two layers were seperated. The organic layer was washed with water, brine, dried (Na₂SO₄) and filtered. The filtrate was concentrated under reduced pressure and the residue obtained was purified by silica gel column chromatography using 30 % ethyl acetate in petroleum ether to afford the desired compound.

### Example I

### N1-[4-(4-Trifluoromethylphenoxy)phenyl]-2-(1,3-dimethyl-2,4-dioxo-1,2,3,4-tetrahydrofuro[2.3-d]pyrimidin-5-yl)acetamide

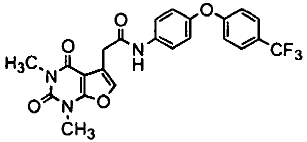

The title compound was prepared according to the general procedure (Method B) by coupling Intermediate 2 (60 mg, 0.252 mmol) with 4-[4-(trifluoromethyl)phenoxy]aniline (64 mg, 0.252 mmol) in the presence of EDCI hydrochloride (58 mg, 0.302 mmol), HOBt (10 mg, 0.075 mmol) and DMAP (3.1 mg, 0.025 mmol) in 1,2-dichloroethane (5 ml) to give 17 mg of the product as a white solid; ¹H NMR (300 MHz, DMSO-*d*₆) δ 3.20 (s, 3H), 3.43 (s, 3H), 3.74 (s, 2H), 7.06-7.13 (m, 4H), 7.60-7.73 (m, 5H), 10.27 (br s, 1H); APCI-MS *(m*/*z)* 474.40 (M+H)⁺.

### Example 2

### N1-[2-(2,4-Dichlorophenyl)ethyl]-2-(1,3-dimethyl-2,4-dioxo-1,2,3,4-tetrahydrofuro[2,3-d]pyrimidin-5-yl)acetamide

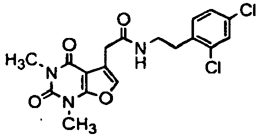

The title compound was prepared according to the general procedure (Method B) by coupling Intermediate 2 (100 mg, 0.420 mmol) with 2-(2,4-dichlorophenyl)ethanamine (79 mg, 0.415 mmol) in the presence of EDCI hydrochloride (96 mg, 0.504 mmol), HOBt (17 mg, 0.126 mmol) and DMAP (5 mg, 0.042 mmol) in 1,2-dichloroethane (5 ml) to give 10 mg of the product as a white solid; ¹H NMR (300 MHz, DMSO-*d*₆) δ 2.81 (t, *J*= 6.6 Hz, 2H), 3.18 (s, 3H), 3.40-3.46 (m, 7H), 7.28 (s, 2H), 7.52 (d, *J*= 8.7 Hz, 2H), 8.02 (br s, 1H); APCI-MS (*m*/*z*) 410.21 (M+H)⁺.

### Example 3

### N1-[4-(Trifluoromethyl)-1,3-thiazol-2-yl]-2-(1,3-Dimethyl-2,4-dioxo-1,2,3,4-tetrahydrofuro[2,3-d]pyrimidin-5-yl)acetamide

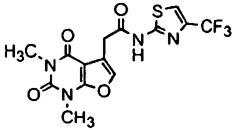

The title compound was prepared according to the general procedure (Method A) by coupling Intermediate 1 (80 mg, 0.300 mmol) with 4-(trifluoromethyl)-1,3-thiazol-2-amine (60 mg, 0.360 mmol) in the presence of sodium hydride (30 mg, 0.750 mmol) in dry toluene (10 ml) to give 40 mg of the product as an off-white solid; ¹H NMR (300 MHz, DMSO-*d*₆) δ 3.17 (s, 3H), 3.42 (s, 3H), 3.87 (s, 2H), 7.63 (s, 1H), 7.95 (s, 1H), 12.77 (br s, 1H); APCI-MS (m/z) 388.99 (M+H)⁺.

### Example 4

### N-(4,6-Difluoro-1,3-benzothiazol-2-yl)-2-(1,3-dimethyl-2,4-dioxo-1,2,3,4-tetrahydrofuro[2,3-d]pyrimidin-5-yl)acetamide

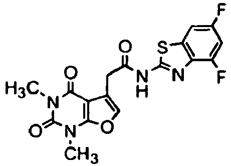

The title compound was prepared according to the general procedure (Method A) by coupling Intermediate 1 (100 mg, 0.375 mmol) with 4,6-difluoro-1,3-benzothiazol-2-amine (83 mg, 0.450 mmol) in the presence of sodium hydride (30 mg, 0.750 mmol) in dry toluene (10 ml) to give 8 mg of the product as an off-white solid; ¹H NMR (300 MHz, DMSO-*d*₆) δ 3.17 (s, 3H), 3.43 (s, 3H), 3.92 (s, 2H), 7.39 (t, *J* = 9.6 Hz, 1H), 7.65 (s, 1H), 7.79 (d, *J=* 7.8 Hz, 1H), 12.85 (br s, 1H); APCI-MS (*m*/*z*) 407.11 (M+H)⁺.

### Example 5

### N1-[4-(4-Isobutylphenyl)-1,3-thiazol-2-yl]-2-(1,3-dimethyl-2,4-dioxo-1,2,3,4-tetrahydrofuro[2,3-d]pyrimidin-5-yl)acetamide

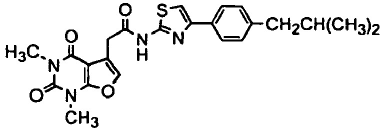

The title compound was prepared according to the general procedure (Method A) by coupling Intermediate 1 (80 mg, 0.300 mmol) with 4-(4-isobutylphenyl)-1,3-thiazol-2-amine (84 mg, 0.360 mmol) in the presence of sodium hydride (30 mg, 0.750 mmol) in dry toluene (10 ml) to give 25 mg of the product as an off-white solid; ¹H NMR (300 MHz, DMSO-*d*₆) δ 0.88 (d, *J* = 6.3 Hz, 6H), 1.82-1.90 (m, 1H), 3.18 (s, 3H), 3.43 (s, 3H), 3.87 (s, 2H), 7.21 (d, *J* = 7.8 Hz, 2H), 7.54 (s, I H), 7.63 (s, 1H), 7.81 (d, *J* = 7.8 Hz, 2H), 12.44 (br s, 1H); APCI-MS (*m*/*z*) 453.16 (M+H)⁺.

### Example 6

### N1-{4-[4-(Trifluoromethyl)phenyl]-1,3-thiazol-2-yl}-2-(1,3-dimethyl-2,4-dioxo-1,2,3,4-tetrahydrofuro[2,3-d]pyrimidin-5-yl)acetamide

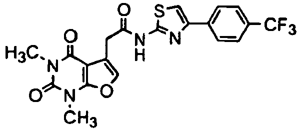

The title compound was prepared according to the general procedure (Method A) by coupling Intermediate 1 (75 mg, 0.282 mmol) with 4-[4-(trifluoromethyl)phenyl]-1,3-thiazol-2-amine (83 mg, 0.338 mmol) in the presence of sodium hydride (28 mg, 0.705 mmol) in dry toluene (10 ml) to give 20 mg of the product as an off-white solid; ¹H NMR (300 MHz, DMSO-*d*₆) δ 3.17 (s, 3H), 3.43 (s, 3H), 3.89 (s, 2H), 7.64 (s, 1H), 7.78-7.96 (m, 3H), 8.09-8.15 (m, 2H), 12.55 (br s, 1H); APCI-MS (*m*/*z*) 465.11 (M+H)⁺.

### Example 7

### N1-{4-[3-(Trifluoromethyl)phenyl)-1,3-thiazol-2-yl}-2-(1,3-dimethyl-2,4-dioxo-1,2,3,4-tetrahydrofuro[2,3-d]pyrimidin-5-yl)acetamide

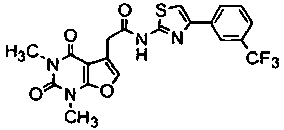

The title compound was prepared according to the general procedure (Method A) by coupling Intermediate 1 (80 mg, 0.300 mmol) with 4-[3-(trifluoromethyl)phenyl]-1,3-thiazol-2-amine (88 mg, 0.360 mmol) in the presence of sodium hydride (30 mg, 0.750 mmol) in dry toluene (10 ml) to give 16 mg of the product as an off white solid; ¹H NMR (300 MHz, DMSO-*d*₆) δ 3.17 (s, 3H), 3.43 (s, 3H), 3.88 (s, 2H), 7.60-7.72 (m, 3H), 7.88 (s, 1H), 8.20-8.26 (m, 2H), 12.54 (br s, I H); APCI-MS (m/z) 465.32 (M+H)⁺.

### Example 8

### N1-[4-(4-Trifluoromethoxyphenyl)-1,3-thiazol-2-yl]-2-(1,3-dimethyl-2,4-dioxo-1,2,3,4-tetrahydrofuro[2,3-d]pyrimidin-5-yl)acetamide

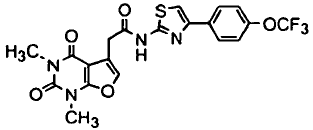

The title compound was prepared according to the general procedure (Method A) by coupling Intermediate 1 (75 mg, 0.282 mmol) with 4-[4-(trifluoromethoxy)phenyl]-1,3-thiazol-2-amine (88 mg, 0.338 mmol) in the presence of sodium hydride (28 mg, 0.705 mmol) in dry toluene (10 ml) to give 11 mg of the product as an off-white solid; ¹H NMR (300 MHz, DMSO-*d*₆) δ 3.18 (s, 3H), 3.43 (s, 3H), 3.88 (s, 2H), 7.43 (d, *J* = 8.4 Hz, 2H), 7.62 (s, 1H), 7.69 (s, 1H), 8.02 (d, *J* = 9.0 Hz, 2H), 12.47 (br s, 1H); APCI-MS (*m*/*z*) 481.14 (M+H)⁺.

### Example 9

### N1-[4-(3-Trifluoromethoxyphenyl)-1,3-thiazol-2-yl]-2-(1,3-dimethyl-2,4-dioxo-1,2,3,4-tetrahydrofuro[2,3-d]pyrimidin-5-yl)acetamide

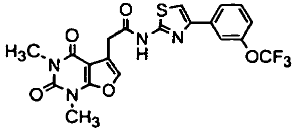

The title compound was prepared according to the general procedure (Method A) by coupling Intermediate 1 (80 mg, 0.300 mmol) with 4-[3-(trifluoromethoxy)phenyl]-1,3-thiazol-2-amine (93 mg, 0.360 mmol) in the presence of sodium hydride (36 mg, 0.900 mmol) in dry toluene (10 ml) to give 60 mg of the product as an off-white solid; ¹H NMR (300 MHz, DMSO-*d*₆) δ 3.17 (s, 3H), 3.43 (s, 3H), 3.88 (s, 2H), 7.33 (d, *J*= 7.8 Hz, 1H), 7.54-7.64 (m, 2H), 7.82 (s, 1H), 7.87 (s, 1H), 7.95 (d, *J* = 7.8 Hz, 1H), 12.52 (br s, 1H); ESI-MS (*m*/*z*) 481.21 (M+H)⁺.

### Example 10

### N1-[4-(3-Bromo-4-fluorophenyl)-1,3-thiazol-2-yl]-2-(1,3-dimethyl-2,4-dioxo-1,2,3,4-tetrahydrofuro[2,3-d]pyrimidin-5-yl)acetamide

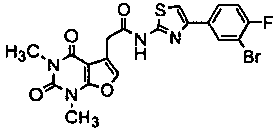

The title compound was prepared according to the general procedure (Method A) by coupling Intermediate 1 (80 mg, 0.300 mmol) with 4-(3-bromo-4-fluorophenyl)-1,3-thiazol-2-amine (98 mg, 0.360 mmol) in the presence of sodium hydride (36 mg, 0.900 mmol) in dry toluene (10 ml) to give 16 mg of the product as an off-white solid; ¹H NMR (300 MHz, DMSO-*d*₆) δ 3.18 (s, 3H), 3.43 (s, 3H), 3.88 (s, 2H), 7.45 (t, *J* = 8.7 Hz, 1H), 7.62 (s, 1H), 7.74 (s, 1H), 7.81-7.88 (m, 1H), 8.23 (d, *J* = 6.9 Hz, I H), 12.45 (br s, 1H); APCI-MS (*m*/*z*) 491.36 (M-H)⁻.

### Example 11

### N1-[4-(3,4-Difluorophenyl)-1,3-thiazol-2-yl]-2-(1,3-dimethyl-2,4-dioxo-1,2,3,4-tetrahydrofuro[2,3-d]pyrimidin-5-yl)acetamide

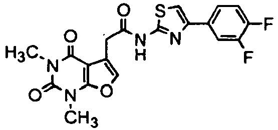

The title compound was prepared according to the general procedure (Method A) by coupling Intermediate 1 (80 mg, 0.300 mmol) with 4-(3,4-difluorophenyl)-1,3-thiazol-2-amine (77 mg, 0.360 mmol) in the presence of sodium hydride (30 mg, 0.750 mmol) in dry toluene (10 ml) to give 40 mg of the product as an off-white solid; ¹H NMR (300 MHz, DMSO-*d*₆) δ 3.17 (s, 3H), 3.43 (s, 3H), 3.88 (s, 2H), 7.51 (q, *J*= 8.7 Hz, 1H), 7.63 (s, 1H), 7.70-7.76 (m, 2H), 7.87-7.96 (m, 1H), 12.49 (br s, 1H); APCI-MS (*m*/*z*) 433.06 (M+H)⁺.

### Example 12

### N1-[4-(2,4-Difluorophenyl)-1,3-thiazol-2-yl]-2-(1,3-dimethyl-2,4-dioxo-1,2,3,4-tetrahydrofuro[2,3-d]pyrimidin-5-yl)acetamide

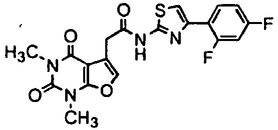

The title compound was prepared according to the general procedure (Method A) by coupling Intermediate 1 (80 mg, 0.300 mmol) with 4-(2,4-difluorophenyl)-1,3-thiazol-2-amine (76 mg, 0.360 mmol) in the presence of sodium hydride (36 mg, 0.900 mmol) in dry toluene (10 ml) to give 29 mg of the product as an off-white solid; ¹H NMR (300 MHz, DMSO-*d*₆) δ 3.18 (s, 3H), 3.40 (s, 3H), 3.86 (s, 2H), 7.20 (t, *J* = 9.6 Hz, 1H), 7.34-7.41 (m, 1H), 7.47 (s, 1H), 7.61 (s, I H), 8.06 (q, *J* = 7.5 Hz, 1H), 12.53 (br s, 1H); APCI-MS (*m*/*z*) 433.05 (M+H)⁺.

### Example 13

### N1-(4-(3,4-Dichlorophenyl)-1,3-thiazol-2-yl]-2-(1,3-dimethyl-2,4-dioxo-1,2,3,4-tetrahydrofuro[2,3-d]pyrimidin-5-yl)acetamide

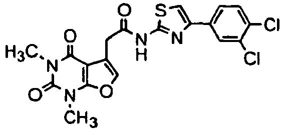

The title compound was prepared according to the general procedure (Method A) by coupling Intermediate I (100 mg, 0.375 mmol) with 4-(3,4-dichlorophenyl)-1,3-thiazol-2-amine (110 mg, 0.450 mmol) in the presence of sodium hydride (30 mg, 0.750 mmol) in dry toluene (10 ml) to give 17 mg of the product as a white solid; ¹H NMR (300 MHz, DMSO-*d*₆) δ 3.18 (s, 3H), 3.43 (s, 3H), 3.88 (s, 2H), 7.63 (s, 1H), 7.70 (d, *J* = 8.1 Hz, 1H), 7.83 (s, 1H), 7.89 (d, *J*= 8.4 Hz, 1H), 8.14 (s, 1H), 12.48 (br s, 1H); APCI-MS (*m*/*z*) 463.20 (M-H)⁻.

### Example 14

### N1-{4-[3-Fluoro-4-(trifluoromethyl)phenyl]-1,3-thiazol-2-yl}-2-(1,3-dimethyl-2,4-dioxo-1,2,3,4-tetrahydrofuro[2,3-d]pyrimidin-5-yl)acetamide

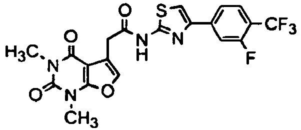

The title compound was prepared according to the general procedure (Method A) by coupling Intermediate 1 (100 mg, 0.375 mmol) with 4-[3-fluoro-4-(trifluoromethyl)phenyl]-1,3-thiazol-2-amine (118 mg, 0.450 mmol) in the presence of sodium hydride (30 mg, 0.750 mmol) in dry toluene (10 ml) to give 25 mg of the product as an off-white solid_{;} ¹H NMR (300 MHz, DMSO-*d*₆) δ 3.18 (s, 3H), 3.43 (s, 3H), 3.89 (s, 2H), 7.63 (s, 1H), 7.86 (t, *J* = 7.8 Hz, 1H), 7.92-8.02 (m, 3H), 12.55 (br s, 1H); APCI-MS (*m*/*z*) 481.25 (M-H)⁻.

### Example 15

### N1-(4-[4-Fluoro-3-(trifluoromethyl)phenyl]-1,3-thiazol-2-yl}-2-(1,3-dimethyl-2,4-dioxo-1,2,3,4-tetrahydrofuro[2,3-d]pyrimidin-5-yl)acetamide

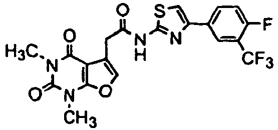

The title compound was prepared according to the general procedure (Method A) by coupling Intermediate I (100 mg, 0.375 mmol) with 4-[4-fluoro-3-(trifluoromethyl)phenyl]-1,3-thiazol-2-amine (136 mg, 0.518 mmol) in the presence of sodium hydride (36 mg, 0.900 mmol) in dry toluene (10 ml) to give 25 mg of the product as an off-white solid; ¹H NMR (300 MHz, DMSO-*d*₆) δ 3.18 (s, 3H), 3.43 (s, 3H), 3.88 (s, 2H), 7.55-7.65 (m, 2H), 7.84 (s, 1H), 8.23-8.29 (m, 2H), 12.51 (br s, 1H); APCI-MS (*m*/*z*) 481.27 (M-H)⁻.

### Example 16

### N1-{4-[4-Fluoro-3-(trifluoromethoxy)phenyl]-1,3-thiazol-2-yl}-2-(1,3-dimethyl-2,4-dioxo-1,2,3,4-tetrahydrofuro[2,3-d]pyrimidin-5-yl)acetamide

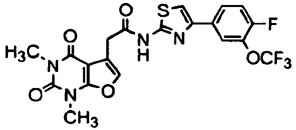

The title compound was prepared according to the general procedure (Method A) by coupling Intermediate 1 (100 mg, 0.375 mmol) with 4-[4-fluoro-3-(trifluoromethoxy)phenyl]-1,3-thiazol-2-amine (125 mg, 0.450 mmol) in the presence of sodium hydride (30 mg, 0.750 mmol) in dry toluene (10 ml) to give 26 mg of the product as a white solid; ¹H NMR (300 MHz, DMSO-*d*₆) δ 3.18 (s, 3H), 3.43 (s, 3H), 3.88 (s, 2H), 7.55-7.63 (m, 2H), 7.79 (s, 1H), 7.97-8.05 (m, 2H), 12.49 (br s, 1H); ESI-MS (*m*/*z*) 497.24 (M-H)⁻.

### Example 17

### N1-{4-[3-Fluoro-4-(trifluoromethuxy)phenyl]-1,3-thiazol-2-yl}-2-(1,3-dimethyl-2,4-dioxo-1,2,3,4-tetrahydrofuro[2,3-d]pyrimidin-5-yl) acetamide

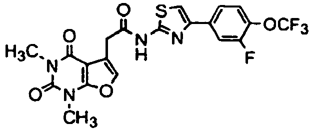

The title compound was prepared according to the general procedure (Method A) by coupling Intermediate 1 (56 mg, 0.210 mmol) with 4-[3-fluoro-4-(trifluoromethoxy)phenyl]-1,3-thiazol-2-amine (70 mg, 0.252 mmol) in the presence of sodium hydride (21 mg, 0.525 mmol) in dry toluene (10 ml) to give 37 mg of the product as a white solid; ¹H NMR (300 MHz, DMSO-*d*₆) δ 3.17 (s, 3H), 3.43 (s, 3H), 3.88 (s, 2H), 7.62-7.69 (m, 2H), 7.84 (s, 2H), 7.95-8.01 (m, 1H), 12.54 (br s, 1H); ESI-MS (*m*/*z*) 497.26 (M+H)'.

### Example 18

### N1-{4-[4-Chloro-3-(trifluoromethyl)phenyl]-1,3-thiazol-2-yl}-2-(1,3-dimethyl-2,4-dioxo-1,2,3,4-tetrahydrofuro[2,3-d]pyrimidin-5-yl)acetamide

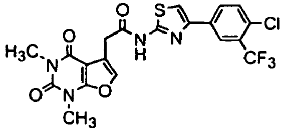

The title compound was prepared according to the general procedure (Method A) by coupling Intermediate 1 (100 mg, 0.375 mmol) with 4-[4-chloro-3-(trifluoromethyl)phenyl]-1,3-thiazol-2-amine (126 mg, 0.450 mmol) in the presence of sodium hydride (30 mg, 0.750 mmol) in dry toluene (10 ml) to give 26 mg of the product as a white solid; ¹H NMR (300 MHz, DMSO-*d*₆) δ 3.17 (s, 3H), 3.43 (s, 3H), 3.88 (s, 2H), 7.58-7.64 (m, 1H), 7.80 (d, J= 8.1 Hz, 1H), 7.92 (s, 1H), 8.20 (d, J= 7.8 Hz, 1H), 8.34 (s, 1H), 12.54 (br s, 1H); APCI-MS (*m*/*z*) 499.00 (M+H)⁺.

### Example 19

### N1-{4-[4-Chloro-3-(trifluoromethoxy)phenyl]-1,3-thiazol-2-yl]-2-(1,3-dimethyl-2,4-dioxo-1,2,3,4-tetrahydrofuro[2,3-d]pyrimidin-5-yl)acetamide

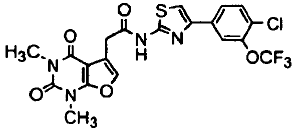

The title compound was prepared according to the general procedure (Method A) by coupling Intermediate 1 (80 mg, 0.300 mmol) with 4-[4-chloro-3-(trifluoromethoxy)phenyl]-1,3-thiazol-2-amine (100 mg, 0.360 mmol) in the presence of sodium hydride (30 mg, 0.750 mmol) in dry toluene (10 ml) to give 32 mg of the product as a white solid; ¹H NMR (300 MHz, DMSO-*d*₆) δ 3.18 (s, 3H), 3.43 (s, 3H), 3.88 (s, 2H), 7.62 (s, 1H), 7.76 (d, *J* = 8.7 Hz, 1H), 7.87 (s, 1H), 7.93-8.04 (m, 2H), 12.51 (br s, 1H); APCI-MS (*m*/*z*) 513.23 (M-H)⁻.

### Example 20

### N1-{4-[2-Fluoro-3-(trifluoromethyl)phenyl]-1,3-thiazol-2-yl}-2-(1,3-dimethyl-2,4-dioxo-1,2,3,4-tetrahydrofuro[2,3-d]pyrimidin-5-yl)acetamide

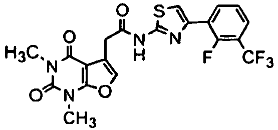

The title compound was prepared according to the general procedure (Method A) by coupling Intermediate 1 (80 mg, 0.300 mmol) with 4-[2-fluoro-3-(trifluoromethyl)phenyl]-1,3-thiazol-2-amine (94 mg, 0.360 mmol) in the presence of sodium hydride (30 mg, 0.750 mmol) in dry toluene (10 ml) to give 50 mg of the product as an off-white solid; ¹H NMR (300 MHz, DMSO-*d*₆) δ 3.18 (s, 3H), 3.43 (s, 3H), 3.89 (s, 2H), 7.53 (d, *J* = 7.8 Hz, 1H), 7.63 (s, 1H), 7.68 (s, 1H), 7.77 (t, *J* = 6.9 Hz, 1H), 8.33 (t, J= 7.5 Hz, I H), 12.55 (br s, I H); APCI-MS (m/z) 481.26 (M-H)⁻.

### Example 21

### N1-{4-[3-Fluoro-5-(trifluoromethyl)phenyl]-1,3-thiazol-2-yl}-2-(1,3-dimethyl-2,4-dioxo-1,2,3,4-tetrahydrofuro[2,3-d]pyrimidin-5-yl)acetamide

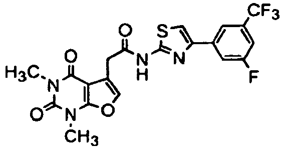

The title compound was prepared according to the general procedure (Method A) by coupling Intermediate 1 (80 mg, 0.300 mmol) with 4-[3-fluoro-5-(trifluoromethyl)phenyl]-1,3-thiazol-2-amine (94 mg, 0.360 mmol) in the presence of sodium hydride (36 mg, 0.900 mmol) in dry toluene (10 ml) to give 25 mg of the product as an off-white solid; ¹H NMR (300 MHz, DMSO-*d*₆) δ 3.17 (s, 3H), 3.43 (s, 3H), 3.88 (s, 2H), 7.60-7.68 (m, 2H), 7.98-8.15 (m, 3H), 12.54 (br s, 1H); APCI-MS (*m*/*z*) 481.24 (M-H)⁻.

### Example 22

### N1-{4-[2-Fluoro-4-(trifluoromethyl)phenyl]-1,3-thiazol-2-yl}-2-(1,3-dimethyl-2,4-dioxo-1,2,3,4-tetrahydrofuro[2,3-d]pyrimidin-5-yl)acetamide

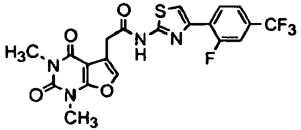

The title compound was prepared according to the general procedure (Method A) by coupling Intermediate 1 (80 mg, 0.300 mmol) with 4-[2-fluoro-4-(trifluoromethyl)phenyl]-1,3-thiazol-2-amine (94 mg, 0.360 mmol) in the presence of sodium hydride (30 mg, 0.750 mmol) in dry toluene (10 ml) to give 30 mg of the product as an off-white solid; ¹H NMR (300 MHz, DMSO-*d*₆) δ 3.18 (s, 3H), 3.43 (s, 3H), 3.90 (s, 2H), 7.63 (s, 1H), 7.73 (s, 2H), 7.78-7.84 (m, 1H), 8.23-8.29 (m, 1H), 12.56 (br s, 1H); APCI-MS (*m*/z) 483.07 (M+H)⁺.

### Example 23

### N1-[4-(3-Fluoro-4-difluoromethoxyphenyl)-1,3-thiazol-2-yl]-2-(1,3-dimethyl-2,4-dioxo-1,2,3,4-tetrahydrofuro[2,3-d]pyrimidin-5-yl)acetamide

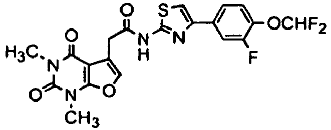

The title compound was prepared according to the general procedure (Method A) by coupling Intermediate 1 (80 mg, 0.300 mmol) with 4-(3-fluoro-4-difluoromethoxyphenyl)-1,3-thiazol-2-amine (95 mg, 0.360 mmol) in the presence of sodium hydride (36 mg, 0.900 mmol) in dry toluene (10 ml) to give 10 mg of the product as an off-white solid; ¹H NMR (300 MHz, DMSO-*d*₆) δ 3.17 (s, 3H), 3.43 (s, 3H), 3.88 (s, 2H), 7.01 (t, *J* = 73.2 Hz, 1H), 7.44 (t, *J* = 8.4 Hz, 1H), 7.63 (s, 1H), 7.73-7.81 (m, 2H), 7.85-7.91 (m, I H), 12.49 (br s, 1H); APCI-MS (*m*/*z*) 479.23 (M-H)⁻.

### Example 24

### N1-[4-(3-Chloro-4-difluoromethoxyphenyl)-1,3-thiazol-2-yl]-2-(1,3-dimethyl-2,4-dioxo-1,2,3,4-tetrahydrofuro[2,3-d]pyrimidin-5-yl)acetamide

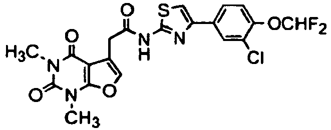

The title compound was prepared according to the general procedure (Method A) by coupling Intermediate 1 (80 mg, 0.300 mmol) with 4-[3-chloro-4-(difluoromethoxy)phenyl]-1,3-thiazol-2-amine (99 mg, 0.360 mmol) in the presence of sodium hydride (30 mg, 0.750 mmol) in dry toluene (10 ml) to give 50 mg of the product as an off-white solid; ¹H NMR (300 MHz, DMSO-*d*₆) δ 3.17 (s, 3H), 3.43 (s, 3H), 3.88 (s, 2H), 7.32 (t, *J*= 73.2 Hz, 1H), 7.45 (d, *J* = 9.0 Hz, 1H), 7.63 (s, 1H), 7.77 (s, 1H), 7.93 (d, *J*= 8.7 Hz, 1H), 8.10 (s, 1H), 12.49 (br s, 1H); APCI-MS (*m*/*z*) 497.16 (M+H)⁺.

### Example 25

### N1-[4-(3-Bromo-4-difluoromethoxyphenyl)-1,3-thiazol-2-yl]-2-(1,3-dimethyl-2,4-dioxo-1,2,3,4-tetrahydrofuro[2,3-d]pyrimidin-5-yl)acetamide

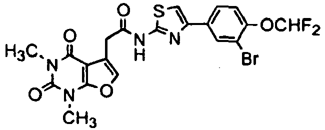

The title compound was prepared according to the general procedure (Method A) by coupling Intermediate 1 (80 mg, 0.300 mmol) with 4-[3-bromo-4-(difluoromethoxy)phenyl]-1,3-thiazol-2-amine (116 mg, 0.360 mmol) in the presence of sodium hydride (24 mg, 0.600 mmol) in dry toluene (10 ml) to give 27 mg of the product as an off-white solid; ¹H NMR (300 MHz, DMSO-*d*₆) δ 3.17 (s, 3H), 3.43 (s, 3H), 3.87 (s, 2H), 7.33 (t, *J* = 73.2 Hz, 1H), 7.40 (d, *J* = 8.1 Hz, 1H), 7.63 (s, 1H), 7.77 (s, 1H), 7.93-7.99 (m, 1H), 8.26 (s, I H), 12.50 (br s, 1H); APCI-MS (*m*/*z*) 541.20 (M+H)⁺.

### Example 26

### N1-{4-[4-(2,2-Dimethylpropoxy)-3-fluorophenyl]-1,3-thiazol-2-yl}-2-(1,3-dimethyl-2,4-dioxo-1,2,3,4-tetrahydrofuro[2,3-d]pyrimidin-5-yl)acetamide

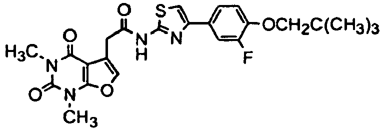

The title compound was prepared according to the general procedure (Method A) by coupling Intermediate 1 (80 mg, 0.300 mmol) with 4-[4-(2,2-dimethylpropoxy)-3-fluorophenyl]-1,3-thiazol-2-amine (101 mg, 0.360 mmol) in the presence of sodium hydride (30 mg, 0.750 mmol) in dry toluene (10 ml) to give 27 mg of the product as an off-white solid; ¹H NMR (300 MHz, DMSO-*d*₆) δ 1.02 (s, 9H), 3.18 (s, 3H), 3.43 (s, 3H), 3.74 (s, 2H), 3.87 (s, 2H), 7.22 (t, *J* = 8.1 Hz, 1H), 7.56 (s, 1H), 7.62 (s, 1H), 7.65-7.70 (m, 1H), 7.73 (s,1H), 12.43 (br s, 1H); APCI-MS (*m*/*z*) 501.15 (M+H)⁺.

### Example 27

### N1-{4-[3-Chloro-4-(2,2-dimethylpropoxy)phenyl]-1,3-thiazol-2-yl}-2-(1,3-dimethyl-2,4-dioxo-1,2,3,4-tetrahydrofuro[2,3-d]pyrimidin-5-yl)acetamide

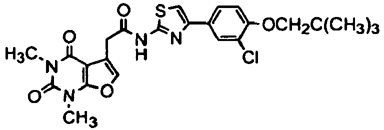

The title compound was prepared according to the general procedure (Method A) by coupling Intermediate 1 (80 mg, 0.300 mmol) with 4-[3-chloro-4-(2,2-dimethylpropoxy)phenyl]-1,3-thiazol-2-amine (107 mg, 0.360 mmol) in the presence of sodium hydride (30 mg, 0.750 mmol) in dry toluene (10 ml) to give 54 mg of the product as an off-white solid; ¹H NMR (300 MHz, DMSO-*d*₆) δ 1.04 (s, 9H), 3.18 (s, 3H), 3.43 (s, 3H), 3.75 (s, 2H), 3.87 (s, 2H), 7.18 (d, *J* = 8.4 Hz, 1H), 7.58 (s, 1H), 7.62 (s, 1H), 7.77-7.85 (m, 1H), 7.95 (s, 1H), 12.42 (br s, 1H); APCI-MS (*m*/*z*) 517.10 (M+H)⁺.

### Example 28

### N1-{4-[3-Bromo-4-(2,2-dimethylpropoxy)phenyl]-1,3-thiazol-2-yl}-2-(1,3-dimethyl-2,4-dioxo-1,2,3,4-tetrahydrofuro[2,3-d]pyrimidin-5-yl)acetamide

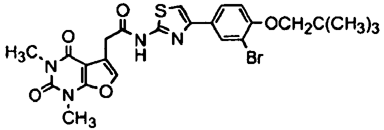

The title compound was prepared according to the general procedure (Method A) by coupling Intermediate 1 (80 mg, 0.300 mmol) with 4-[3-bromo-4-(2,2-dimethylpropoxy)phenyl]-1,3-thiazol-2-amine (122 mg, 0.360 mmol) in the presence of sodium hydride (30 mg, 0.750 mmol) in dry toluene (10 ml) to give 26 mg of the product as an off-white solid; ¹H NMR (300 MHz, DMSO-*d*₆) δ 1.05 (s, 9H), 3.17 (s, 3H), 3.43 (s, 3H), 3.75 (s, 2H), 3.87 (s, 2H), 7.14 (d, *J* = 8.7 Hz, 1H), 7.58 (s, 1H), 7.62 (s, 1H), 7.86 (d, *J* = 7.8 Hz, 1H), 8.12 (s, I H), 12.43 (br s, I H); APCI-MS (*m*/*z*) 561.06 (M)⁺.

### Example 29

### N1-[4-(3-Bromo-4-isobutoxyphenyl)-1,3-thiazol-2-yl]-2-(1,3-dimethyl-2,4-dioxo-1,2,3,4-tetrahydrofuro[2,3-d]pyrimidin-5-yl)acetamide

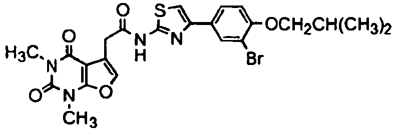

The title compound was prepared according to the general procedure (Method A) by coupling Intermediate 1 (80 mg, 0.300 mmol) with 4-(3-bromo-4-isobutoxyphenyl)-1,3-thiazol-2-amine (118 mg, 0.360 mmol) in the presence of sodium hydride (30 mg, 0.750 mmol) in dry toluene (10 ml) to give 40 mg of the product as an off-white solid; ¹H NMR (300 MHz, DMSO-*d*₆) δ 1.02 (d, *J* = 6.6 Hz, 6H), 2.02-2.08 (m, I H), 3.18 (s, 3H), 3.43 (s, 3H), 3.87 (s, 4H), 7.15 (d, *J* = 9.0 Hz, I H), 7.58 (s, I H), 7.62 (s, 1H), 7.95 (d, *J* = 8.7 Hz, 1H), 8.12 (s, 1H), 12.42 (br s, 1H); APCI-MS (*m*/*z*) 547.09 (M)⁺.

### Example 30

### N1-{4-[3-Fluoro-4-(2,2,2-trifluoroethoxy)phenyl]-1,3-thiazol-2-yl}2-(1,3-dimethyl-2,4-dioxo-1,2,3,4-tetrahydrofuro[2,3-d]pyrimidin-5-yl)acetamide

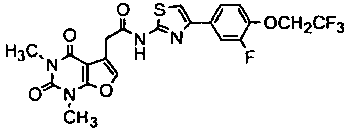

The title compound was prepared according to the general procedure (Method A) by coupling Intermediate 1 (80 mg, 0.300 mmol) with 4-[3-fluoro-4-(2,2,2-trifluoroethoxy)phenyl]-1,3-thiazol-2-amine (105 mg, 0.360 mmol) in the presence of sodium hydride (30 mg, 0.750 mmol) in dry toluene (10 ml) to give 23 mg of the product as a white solid; ¹H NMR (300 MHz, DMSO-*d*₆) δ 3.16 (s, 3H), 3.41 (s, 3H), 3.86 (s, 2H), 4.87 (q, *J* = 8.7 Hz, 2H), 7.34 (t, *J =* 8.7 Hz, 1H), 7.61 (s, 2H), 7.68-7.73 (m, 2H), 12.41 (br s, 1H); APCI-MS (*m*/*z*) 513.07 (M+H)⁺.

### Example 31

### N1-{4-[3-Chloro-4-(2,2,2-trifluoroethoxy)phenyl]-1,3-thiazol-2-yl}2-(1,3-dimethyl-2,4-dioxo-1,2,3,4-tetrahydrofuro[2,3-d]pyrimidin-5-yl)acetamide

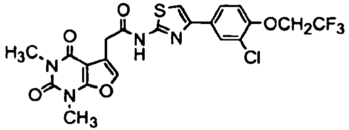

The title compound was prepared according to the general procedure (Method A) by coupling Intermediate 1 (80 mg, 0.300 mmol) with 4-[3-chloro-4-(2,2,2-trifluoroethoxy)phenyl]-1,3-thiazol-2-amine (111 mg, 0.360 mmol) in the presence of sodium hydride (36 mg, 0.900 mmol) in dry toluene (10 ml) to give 34 mg of the product as a white solid; ¹H NMR (300 MHz, DMSO-*d*₆) δ 3.18 (s, 3H), 3.43 (s, 3H), 3.87 (s, 2H), 4.91 (d, *J* = 8.7 Hz, 2H), 7.35 (d, *J* = 8.7 Hz, 1H), 7.62 (s, 1H), 7.66 (s, 1H), 7.86 (d, *J =* 9.0 Hz, 1H), 8.01 (s, 1H), 12.43 (br s, 1H); APCI-MS (*m*/*z*) 529.15 (M+H)⁺.

### Example 32

### N1-{4-[3-Bromo-4-(2,2,2-trifluoroethoxy)phenyl]-1,3-thiazol-2-yl}2-(1,3-dimethyl-2,4-dioxo-1,2,3,4-tetrahydrofuro[2,3-d]pyrimidin-5-yl)acetamide

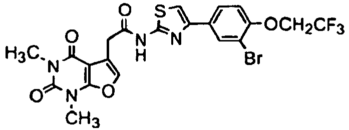

The title compound was prepared according to the general procedure (Method A) by coupling Intermediate 1 (80 mg, 0.300 mmol) with 4-[3-bromo-4-(2,2,2-trifluoroethoxy)phenyl]-1,3-thiazol-2-amine (127 mg, 0.360 mmol) in the presence of sodium hydride (36 mg, 0.900 mmol) in dry toluene (10 ml) to give 53 mg of the product as a white solid; ¹H NMR (300 MHz, DMSO-*d*₆) δ 3.18 (s, 3H), 3.43 (s, 3H), 3.87 (s, 2H), 4.90 (q, *J* = 8.7 Hz, 2H), 7.31 (d, *J* = 8.7 Hz, 1H), 7.62 (s, 1H), 7.66 (s, 1H), 7.91 (d, *J*= 9.3 Hz, 1H), 8.16 (s, 1H), 12.43 (br s, 1H); APCI-MS (*m*/*z*) 571.27 (M-H)⁻.

### Example 33

### N1-[4-(2,4,5,-Trifluorophenyl)-1,3-thiazol-2-yl]-2-(1,3-dimethyl-2,4-dioxo-1,2,3,4-tetrahydrofuro[2,3-d]pyrimidin-5-yl)acetamide

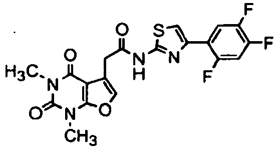

The title compound was prepared according to the general procedure (Method A) by coupling Intermediate 1 (80 mg, 0.300 mmol) with 4-(2,4,5-trifluorophenyl)-1,3-thiazol-2-amine (83 mg, 0.360 mmol) in the presence of sodium hydride (30 mg, 0.750 mmol) in dry toluene (10 ml) to give 20 mg of the product as an off-white solid; ¹H NMR (300 MHz, DMSO-*d*₆) δ 3.17 (s, 3H), 3.43 (s, 3H), 3.89 (s, 2H), 7.59 (s, 1H), 7.63 (s, 1H), 7.70-7.76 (m, 1H), 7.88-8.00 (m, I H), 12.53 (br s, I H); APCI-MS (*m*/*z*) 451.18 (M+H)⁺.

### Example 34

### N1-[4-(2,3,4-Trichlorophenyl)-1,3-thiazol-2-yl]-2-(1,3-dimethyl-2,4-dioxo-1,2,3,4-tetrahydrofuro[2,3-d]pyrimidin-5-yl)acetamide

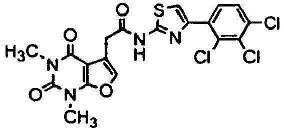

The title compound was prepared according to the general procedure (Method A) by coupling Intermediate 1 (80 mg, 0.300 mmol) with 4-(2,3,4-trichlorophenyl)-1,3-thiazol-2-amine (100 mg, 0.429 mmol) in the presence of sodium hydride (30 mg, 0.750 mmol) in dry toluene (10 ml) to give 30 mg of the product as an off-white solid; ¹H NMR (300 MHz, DMSO-*d*₆) δ 3.18 (s, 3H), 3.43 (s, 3H), 3.88 (s, 2H), 7.63 (s, 1H), 7.68 (s, 1H), 7.90 (d, *J* = 8.7 Hz, 2H), 12.51 (br s, 1H); APCI-MS (*m*/*z*) 499.39 (M-H)⁻.

### Example 35

### N1-{4-[2,4-Difluoro-3-(trifluoromethyl)phenyl]-1,3-thiazol-2-yl}2-(1,3-dimethyl-2,4-dioxo-1,2,3,4-tetrahydrofuro[2,3-d]pyrimidin-5-yl)acetamide

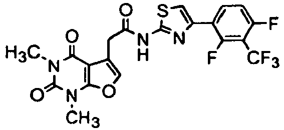

The title compound was prepared according to the general procedure (Method A) by coupling Intermediate 1 (100 mg, 0.375 mmol) with 4-[2,4-difluoro-3-(trifluoromethyl)phenyl]-1,3-thiazol-2-amine (126 mg, 0.450 mmol) in the presence of sodium hydride (30 mg, 0.750 mmol) in dry toluene (10 ml) to give 30 mg of the product as a white solid; ¹H NMR (300 MHz, DMSO-*d*₆) δ 3.18 (s, 3H), 3.43 (s, 3H), 3.89 (s, 2H), 7.47-7.56 (m, 1H), 7.63 (s, 2H), 8.33 (q, *J* = 6.6 Hz, 1H), 12.54 (br s, 1H); APCI-MS (*m*/*z*) 499.39 (M-H)⁻.

### Example 36

### N1-{4-[3,5-Difluoro-4-(trifluoromethyl)phenyl]-1,3-thiazol-2-yl}-2-(1,3-dimethyl-2,4-dioxo-1,2,3,4-tetrahydrofuro[2,3-d]pyrimidin-5-yl)acetamide

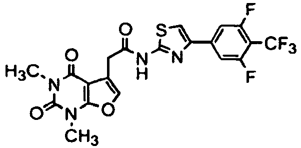

The title compound was prepared according to the general procedure (Method A) by coupling Intermediate 1 (80 mg, 0.300 mmol) with 4-[3,5-difluoro-4-(trifluoromethyl)phenyl]-1,3-thiazol-2-amine (100 mg, 0.360 mmol) in the presence of sodium hydride (30 mg, 0.750 mmol) in dry toluene (10 ml) to give 20 mg of the product as an off-white solid; ¹H NMR (300 MHz, DMSO-*d*₆) δ 3.17 (s, 3H), 3.43 (s, 3H), 3.89 (s, 2H), 7.63 (s, 1H), 7.84 (s, 1H), 7.88 (s, 1H), 8.09 (s, 1H), 12.59 (br s, 1H); APCI-MS (*m*/*z*) 500.97 (M+H)⁺.

### Example 37

### N1-{4-[3,5-Difluoro-4-(difluoromethoxy)phenyl]-1,3-thiazol-2-yl}-2-(1,3-dimethyl-2,4-dioxo-1,2,3,4-tetrahydrofuro[2,3-d]pyrimidin-5-yl)acetamide

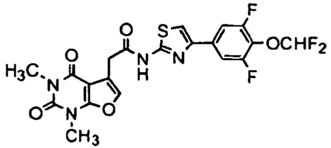

The title compound was prepared according to the general procedure (Method A) by coupling Intermediate 1 (80 mg, 0.300 mmol) with 4-[3,5-difluoro-4-(difluoromethoxy)phenyl]-1,3-thiazol-2-amine (100 mg, 0.360 mmol) in the presence of sodium hydride (30 mg, 0.751 mmol) in dry toluene (10 ml) to give 25 mg of the product as an off-white solid; ¹H NMR (300 MHz, DMSO-*d*₆) δ 3.17 (s, 3H), 3.43 (s, 3H), 3.88 (s, 2H), 7.27 (t, *J* = 71.7 Hz, 1H), 7.62 (s, I H), 7.79 (d, *J* = 9.3 Hz, 2H), 7.88 (s, 1H), 12.51 (br s, 1H); APCI-MS (*m*/*z*) 499.00 (M+H)⁺.

### Example 38

### N1-{4-[3,5-Dichloro-4-(difluoromethoxy)phenyl]-1,3-thiazol-2-yl}-2-(1,3-dimethyl-2,4-dioxo-1,2,3,4-tetrahydrofuro[2,3-d]pyrimidin-5-yl)acetamide

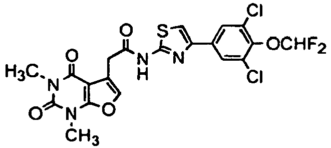

The title compound was prepared according to the general procedure (Method A) by coupling Intermediate 1 (80 mg, 0.300 mmol) with 4-[3,5-dichloro-4-(difluoromethoxy)phenyl]-1,3-thiazol-2-amine (112 mg, 0.360 mmol) in the presence of sodium hydride (36 mg, 0.900 mmol) in dry toluene (10 ml) to give 15 mg of the product as an off-white solid; ¹H NMR (300 MHz, DMSO-*d*₆) δ 3.18 (s, 3H), 3.43 (s, 3H), 3.88 (s, 2H), 7.19 (t, *J* = 72.3 Hz, 1H), 7.63 (s, 1H), 7.94 (s, 1H), 8.12 (s, 2H), 12.51 (br s, 1H); APCI-MS (*m*/*z*) 529.21 (M-H)⁻.

### Example 39

### N1-{4-[4-(Cyclopropylmethoxy)-3,5-difluorophenyl]-1,3-thiazol-2-yl]-2-(1,3-dimethyl-2,4-dioxo-1,2,3,4-tetrahydrofuro[2,3-d]pyrimidin-5-yl)acetamide

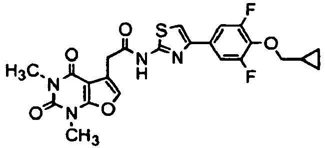

The title compound was prepared according to the general procedure (Method A) by coupling Intermediate 1 (80 mg, 0.300 mmol) with 4-[4-(cyclopropylmethoxy)-3,5-difluorophenyl]-1,3-thiazol-2-amine (102 mg, 0.360 mmol) in the presence of sodium hydride (30 mg, 0.750 mmol) in dry toluene (10 ml) to give 50 mg of the product as a white solid; ¹H NMR (300 MHz, DMSO-*d*₆) δ 0.23-0.30 (m, 2H), 0.50-0.56 (m, 2H), 1.88-1.94 (m, 1H), 3.17 (s, 3H), 3.43 (s, 3H), 3.88 (s, 2H), 3.97 (d, *J* = 7.2 Hz, 2H), 7.60-7.68 (m, 3H), 7.74 (s, 1H), 12.46 (br s, 1H); APCI-MS (*m*/*z*) 503.11 (M+H)⁺.

### Example 40

### N1-{4-(3,5-Difluoro-4-isobutoxyphenyl)-1,3-thiazol-2-yl}2-(1,3-dimethyl-2,4-dioxo-1,2,3,4-tetrahydrofuro[2,3-d]pyrimidin-5-yl)acetamide

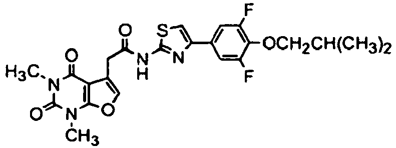

The title compound was prepared according to the general procedure (Method A) by coupling Intermediate 1 (80 mg, 0.300 mmol) with 4-(3,5-difluoro-4-isobutoxyphenyl)-1,3-thiazol-2-amine (102 mg, 0.360 mmol) in the presence of sodium hydride (30 mg, 0.750 mmol) in dry toluene (10 ml) to give 26 mg of the product as a white solid; ¹H NMR (300 MHz, DMSO-*d*₆) δ 0.98 (d, *J*= 6.9 Hz, 6H), 1.93-2.04 (m, 1H), 3.18 (s, 3H), 3.43 (s, 3H), 3.86-3.93 (m, 4H), 7.60-7.68 (m, 3H), 7.73 (s, 1H), 12.45 (br s, 1H); APCI-MS (*m*/*z*) 503.29 (M-H)⁻.

### Example 41

### N1-[4-(3,5-Dichloro-4-isobutoxyphenyl)-1,3-thiazol-2-yl]-2-(1,3-dimethyl-2,4-dioxo-1,2,3,4-tetrahydrofuro[2,3-d]pyrimidin-5-yl)acetamide

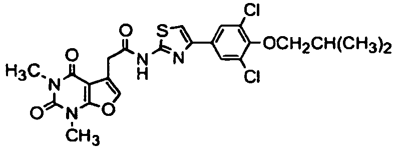

The title compound was prepared according to the general procedure (Method A) by coupling Intermediate 1 (80 mg, 0.300 mmol) with 4-(3,5-dichloro-4-isobutoxyphenyl)-1,3-thiazol -2-amine (114 mg, 0.360 mmol) in the presence of sodium hydride (30 mg, 0.750 mmol) in dry toluene (10 ml) to give 20 mg of the product as an off-white solid; ¹H NMR (300 MHz, DMSO-*d*₆) δ 1.04 (d, *J*= 6.9 Hz, 6H), 2.00-2.14 (m, 1H), 3.17 (s, 3H), 3.43 (s, 3H), 3.79 (d, *J* = 5.7 Hz, 2H), 3.87 (s, 2H), 7.63 (s, 1H), 7.82 (s, 1H), 8.00 (s, 2H), 12.49 (br s, I H); APCI-MS (*m*/*z*) 537.09 (M+H)'.

### Example 42

### N1-{4-[3,5-Difluoro-4-(2,2-dimethylpropoxy)phenyl]-1,3-thiazol-2-yl}2-(1,3-dimethyl-2,4-dioxo-1,2,3,4-tetrahydrofuro[2,3-d]pyrimidin-5-yl)acetamide

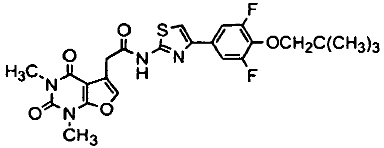

The title compound was prepared according to the general procedure (Method A) by coupling Intermediate 1 (75 mg, 0.282 mmol) with 4-[4-(2,2-dimethylpropoxy)-3,5-difluorophenyl]-1,3-thiazol-2-amine (101 mg, 0.338 mmol) in the presence of sodium hydride (28 mg, 0.704 mmol) in dry toluene (10 ml) to give 38 mg of the product as a white solid; ¹H NMR (300 MHz, DMSO-*d*₆) δ 1.01 (s, 9H), 3.18 (s, 3H), 3.43 (s, 3H), 3.80 (s, 2H), 3.88 (s, 2H), 7.60-7.66 (m, 3H), 7.73 (s, 1H), 12.45 (br s, 1H); APCI-MS (*m*/*z*) 519.14 (M+H)⁺.

### Example 43

### N1-{4-[3,5-Dichloro-4-(2,2-dimethylpropoxy)phenyl]-1,3-thiazol-2-yl}-2-(1,3-dimethyl-2,4-dioxo-1,2,3,4-tetrahydrofuro[2,3-d]pyrimidin-5-yl)acetamide

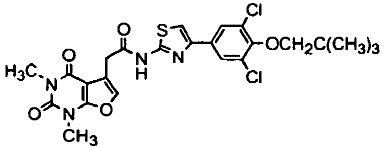

The title compound was prepared according to the general procedure (Method A) by coupling Intermediate 1 (80 mg, 0.300 mmol) with 4-[3,5-dichloro-4-(2,2-dimethylpropoxy)phenyl]-1,3-thiazol-2-amine (119 mg, 0.360 mmol) in the presence of sodium hydride (30 mg, 0.750 mmol) in dry toluene (10 ml) to give 16 mg of the product as an off-white solid; ¹H NMR (300 MHz, DMSO-*d*₆) δ 1.23 (s, 9H), 3.17 (s, 3H), 3.43 (s, 3H), 3.67 (s, 2H), 3.88 (s, 2H), 7.63 (s, 1H), 7.83 (s, 1H), 8.00 (s, 2H), 12.49 (br s, 1H); APCI-MS (*m*/*z*) 551.09 (M+H)⁺.

### Example 44

### N1-{4-[3-Chloro-5-fluoro-4-(2,2-dimethylpropoxy)phenyl]-1,3-thiazol-2-yl}-2-(1,3-dimethyl-2,4-dioxo-1,2,3,4-tetrahydrofuro[2,3-d]pyrimidin-5-yl)acetamide

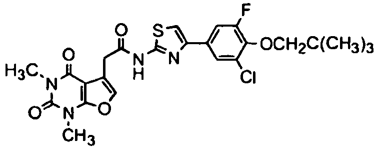

The title compound was prepared according to the general procedure (Method A) by coupling Intermediate 1 (80 mg, 0.300 mmol) with 4-[3-chloro-5-fluoro-4-(2,2-dimethylpropoxy)phenyl]-1,3-thiazol-2-amine (113 mg, 0.360 mmol) in the presence of sodium hydride (36 mg, 0.900 mmol) in dry toluene (10 ml) to give 50 mg of the product as an off-white solid; ¹H NMR (300 MHz, DMSO-*d*₆) δ 1.04 (s, 9H), 3.17 (s, 3H), 3.43 (s, 3H), 3.78 (s, 2H), 3.88 (s, 2H), 7.62 (s, 1H), 7.75-7.81 (m, 2H), 7.85 (s, 1H), 12.46 (brs, 1H); APCI-MS (*m*/*z*) 535.26 (M+H)⁺.

### Example 45

### N1-{4-[3,5-Difluoro-4-(3-methylbutoxy)phenyl]-1,3-thiazol-2-yl}-2-(1,3-dimethyl-2,4-dioxo-1,2,3,4-tetrahydrofuro[2,3-d]pyrimidin-5-yl)acetamide

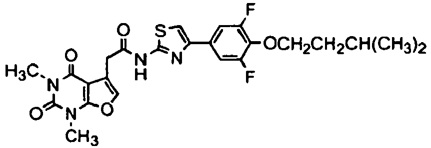

The title compound was prepared according to the general procedure (Method A) by coupling Intermediate 1 (80 mg, 0.300 mmol) with 4-[3,5-difluoro-4-(3-methylbutoxy)phenyl]-1,3-thiazol-2-amine (107 mg, 0.358 mmol) in the presence of sodium hydride (30 mg, 0.750 mmol) in dry toluene (10 ml) to give 40 mg of the product as an off-white solid; ¹H NMR (300 MHz, DMSO-*d*₆) δ 0.92 (d, *J* = 6.3 Hz, 6H), 1.59 (q, *J* = 6.3 Hz, 2H), 1.88-1.93 (m, 1H), 3.17 (s, 3H), 3.43 (s, 3H), 3.88 (s, 2H), 4.15 (d, *J*= 6.6 Hz, 2H), 7.60-7.69 (m, 3H), 7.75 (s, 1H), 12.49 (br s, 1H); ESI-MS (*m*/*z*) 519.11 (M+H)⁺.

### Example 46

### N1-{4-[3,5-Difluoro-4-(2,2,2-trifluoroethoxy)phenyl]-1,3-thiazol-2-yl}-2-(1,3-dimethyl-2,4-dioxo-1,2,3,4-tetrahydrofuro[2,3-d]pyrimidin-5-yl)acetamide

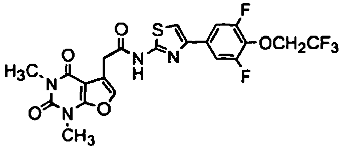

The title compound was prepared according to the general procedure (Method A) by coupling Intermediate 1 (75 mg, 0.282 mmol) with 4-[3,5-difluoro-4-(2,2,2-trifluoroethoxy)phenyl]-1,3-thiazol-2-amine (105 mg, 0.338 mmol) in the presence of sodium hydride (28 mg, 0.704 mmol) in dry toluene (10 ml) to give 28 mg of the product as a white solid; ¹H NMR (300 MHz, DMSO-*d*₆) δ 3.17 (s, 3H), 3.43 (s, 3H), 3.88 (s, 2H), 4.85 (q, *J* = 8.7 Hz, 2H), 7.62 (s, I H), 7.70 (d, *J* = 9.3 Hz, 2H), 7.80 (s, 1H), 12.48 (br s, 1H); APCI-MS (*m*/*z*) 531.08 (M+H)⁺.

### Example 47

### N1-{4-[3,5-Dichloro-4-(2,2,2-trifluoroethoxy)phenyl]-1,3-thiazol-2-yl}2-(1,3-dimethyl-2,4-dioxo-1,2,3,4-tetrahydrofuro[2,3-d]pyrimidin-5-yl)acetamide

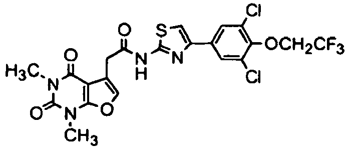

The title compound was prepared according to the general procedure (Method A) by coupling Intermediate 1 (80 mg, 0.300 mmol) with 4-[3,5-dichloro-4-(2,2,2-trifluoroethoxy)phenyl]-1,3-thiazol-2-amine (123 mg, 0.360 mmol) in the presence of sodium hydride (36 mg, 0.900 mmol) in dry toluene (10 ml) to give 17 mg of the product as a white solid; ¹H NMR (300 MHz, DMSO-*d*₆) δ 3.17 (s, 3H), 3.43 (s, 3H), 3.88 (s, 2H), 4.75 (q, *J* = 8.7 Hz, 2H), 7.62 (s, 1H), 7.87 (s, 1H), 8.04 (s, 2H), 12.48 (br s, 1H); APCI-MS (*m*/*z*) 563.06 (M+H)⁺.

### Example 48

### N1-{4-[3-Chloro-5-fluoro-4-(2,2,2-trifluoroethoxy)phenyl]-1,3-thiazol-2-yl}-2-(1,3-dimethyl-2,4-dioxo-1,2,3,4-tetrahydrofuro[2,3-d]pyrimidin-5-yl)acetamide

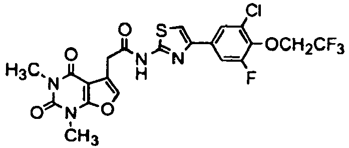

The title compound was prepared according to the general procedure (Method A) by coupling Intermediate 1 (80 mg, 0.300 mmol) with 4-[3-chloro-5-fluoro-4-(2,2,2-trifluoroethoxy)phenyl]-1,3-thiazol-2-amine (117 mg, 0.360 mmol) in the presence of sodium hydride (30 mg, 0.750 mmol) in dry toluene (10 ml) to give 22 mg of the product as an off-white solid; ¹H NMR (300 MHz, DMSO-*d*6) δ 3.17 (s, 3H), 3.43 (s, 3H), 3.88 (s, 2H), 4.83 (q, *J* = 8.7 Hz, 2H), 7.62 (s, 1H), 7.80-7.92 (m, 3H), 12.48 (br s, 1H); APCI-MS (*m*/*z*) 545.16 (M-H)⁻.

### Example 49

### N1-{4-[3,5-Difluoro-4-(3,3,3-trifluoropropoxy)phenyl]-1,3-thiazol-2-yl}-2-(1,3-dimethyl-2,4-dioxo-1,2,3,4-tetrahydrofuro[2,3-d]pyrimidin-5-yl)acetamide

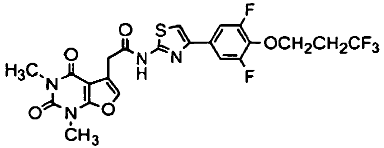

The title compound was prepared according to the general procedure (Method A) by coupling Intermediate 1 (57 mg, 0.216 mmol) with 4-[3,5-difluoro-4-(3,3,3-trifluoropropoxy)phenyl]-1,3-thiazol-2-amine (70 mg, 0.216 mmol) in the presence of sodium hydride (22 mg, 0.540 mmol) in dry toluene (10 ml) to give 17 mg of the product as a white solid; ¹H NMR (300 MHz, DMSO-*d*₆) δ 2.74-2.83 (m, 2H), 3.17 (s, 3H), 3.43 (s, 3H), 3.88 (s, 2H), 4.30-4.38 (m, 2H), 7.60-7.69 (m, 3H), 7.76 (s, 1H), 12.47 (br s, 1H); APCI-MS (*m*/*z*) 543.18 (M-H)⁻.

### Example 50

### N1-{4-[4-(Trifluoromethyl)phenyl]-1,3-thiazol-2-yl}-2-(1,3,6-trimethyl-2,4-dioxo-1,2,3,4-tetrahydrofuro[2,3-d]pyrimidin-5-yl)acetamide

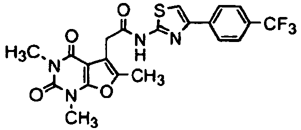

The title compound was prepared according to the general procedure (Method A) by coupling Intermediate 3 (50 mg, 0.177 mmol) with 4-[4-(trifluoromethyl)phenyl]-1,3-thiazol-2-amine (51 mg, 0.212 mmol) in the presence of sodium hydride (11 mg, 0.442 mmol) in dry toluene (10 ml) to give 32 mg of the product as a white solid; ¹H NMR (300 MHz, DMSO-*d*₆) δ 2.32 (s, 3H), 3.16 (s, 3H), 3.41 (s, 3H), 3.84 (s, 2H), 7.78-7.86 (m, 3H), 8.12 (d, *J* = 8.4 Hz, 2H), 12.50 (br s, 1H); APCI-MS (*m*/*z*) 479.14 (M+H)⁺.

### Example 51

### N1-{4-[3-(Trifluoromethyl)phenyl]-1,3-thiazol-2-yl}-2-(1,3,6-trimethyl-2,4-dioxo-1,2,3,4-tetrahydrofuro[2,3-d]pyrimidin-5-yl)acetamide

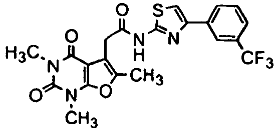

The title compound was prepared according to the general procedure (Method A) by coupling Intermediate 3 (50 mg, 0.177 mmol) with 4-[3-(trifluoromethyl)phenyl]-1,3-thiazol-2-amine (51 mg, 0.212 mmol) in the presence of sodium hydride (17 mg, 0.445 mmol) in dry toluene (10 ml) to give 30 mg of the product as a white solid; ¹H NMR (300 MHz, DMSO-*d*₆) δ 2.32 (s, 3H), 3.16 (s, 3H), 3.42 (s, 3H), 3.83 (s, 2H), 7.66-7.74 (m, 2H), 7.87 (s, 1H), 8.19-8.26 (m, 2H), 12.49 (br s, 1H); APCI-MS (*m*/*z*) 479.16 (M+H)⁺.

### Example 52

### N1-{4-[3-(Trifluoromethoxy)phenyl]-1,3-thiazol-2-yl}-2-(1,3,6-trimethyl-2,4-dioxo-1,2,3,4-tetrahydrofuro[2,3-d]pyrimidin-5-yl)acetamide

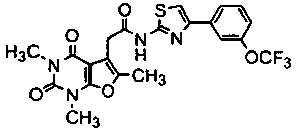

The title compound was prepared according to the general procedure (Method A) by coupling Intermediate 3 (50 mg, 0.177 mmol) with 4-[3-(trifluoromethoxy)phenyl]-1,3-thiazol-2-amine (55 mg, 0.212 mmol) in the presence of sodium hydride (17 mg, 0.445 mmol) in dry toluene (10 ml) to give 12.5 mg of the product as a white solid; ¹H NMR (300 MHz, DMSO-*d*₆) δ 2.32 (s, 3H), 3.16 (s, 3H), 3.41 (s, 3H), 3.83 (s, 2H), 7.30-7.36 (m, 1H), 7.58 (t, *J* = 7.8 Hz, 1H), 7.81 (s, 1H), 7.87 (s, 1H), 7.95 (d, *J* = 7.8 Hz, 1H), 12.48 (br s, 1H); APCI-MS (*m*/*z*) 495.11 (M+H)⁺.

### Example 53

### N1-{4-[4-(Trifluoromethoxy)phenyl]-1,3-thiazol-2-yl}-2-(1,3,6-trimethyl-2,4-dioxo-1,2,3,4-tetrahydrofuro[2,3-d]pyrimidin-5-yl)acetamide

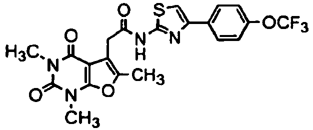

The title compound was prepared according to the general procedure (Method A) by coupling Intermediate 3 (50 mg, 0.178 mmol) with 4-[4-(trifluoromethoxy)phenyl]-1,3-thiazol-2-amine (55 mg, 0.214 mmol) in the presence of sodium hydride (10 mg, 0.445 mmol) in dry toluene (10 ml) to give 30 mg of the product as a white solid; ¹H NMR (300 MHz, DMSO-*d*₆) δ 2.31 (s, 3H), 3.16 (s, 3H), 3.41 (s, 3H), 3.83 (s, 2H), 7.44 (d, *J* = 7.8 Hz, 2H), 7.69 (s, I H), 8.02 (d, *J* = 8.7 Hz, 2H), 12.46 (br s, I H); APCI-MS (*m*/*z*) 495.11 (M+H)⁺.

### Example 54

### N1-[4-(3,4-Dichlorophenyl)-1,3-thiazol-2-yl]-2-(1,3,6-trimethyl-2,4-dioxo-1,2,3,4-tetrahydrofuro[2,3-d]pyrimidin-5-yl)acetamide

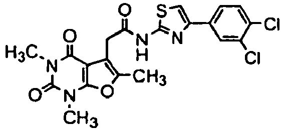

The title compound was prepared according to the general procedure (Method A) by coupling Intermediate 3 (40 mg, 0.142 mmol) with 4-(3,4-dichlorophenyl)-1,3-thiazol-2-amine (42 mg, 0.171 mmol) in the presence of sodium hydride (14 mg, 0.355 mmol) in dry toluene (10 ml) to give 25 mg of the product as an off-white solid; ¹H NMR (300 MHz, DMSO-*d*₆) δ 2.31 (s, 3H), 3.15 (s, 3H), 3.41 (s, 3H), 3.83 (s, 2H), 7.21 (d, *J*= 8.1 Hz, 1H), 7.83 (s, 1H), 7.89 (d, *J* = 8.7 Hz, 1H), 8.15 (s, I H), 12.47 (br s, 1H); APCI-MS (*m*/*z*) 479.09 (M+H)⁺.

### Example 55

### N1-[4-(2,3-Dichlorophenyl)-1,3-thiazol-2-yl]-2-(1,3,6-trimethyl-2,4-dioxo-1,2,3,4-tetrahydrofuro[2,3-d]pyrimidin-5-yl)acetamide

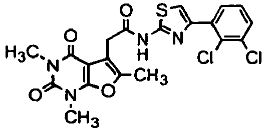

The title compound was prepared according to the general procedure (Method A) by coupling Intermediate 3 (50 mg, 0.178 mmol) with 4-(2,3-dichlorophenyl)-1,3-thiazol-2-amine (52 mg, 0.214 mmol) in the presence of sodium hydride (17 mg, 0.445 mmol) in dry toluene (10 ml) to give 45 mg of the product as a white solid; ¹H NMR (300 MHz, DMSO-*d*₆) δ 2.32 (s, 3H), 3.16 (s, 3H), 3.42 (s, 3H), 3.83 (s, 2H), 7.46 (t, *J* = 7.8 Hz, 1H), 7.61 (s, 1H), 7.67 (d, *J* = 8.4 Hz, 1H), 7.74 (d, *J* = 7.8 Hz, 1H), 12.47 (br s, 1H); APCI-MS (*m*/*z*) 479.10 (M+H)⁺.

### Example 56

### N1-[4-(2,4-Dichlorophenyl)-1,3-thiazol-2-yl]-2-(1,3,6-trimethyl-2,4-dioxo-1,2,3,4-tetrahydrofuro[2,3-d]pyrimidin-5-yl)acetamide

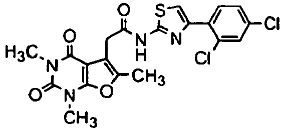

The title compound was prepared according to the general procedure (Method A) by coupling (Intermediate 3 (50 mg, 0.178 mmol) with 4-(2,4-dichlorophenyl)-1,3-thiazol-2-amine (52 mg, 0.214 mmol) in the presence of sodium hydride (17 mg, 0.445 mmol) in dry toluene (10 ml) to give 20 mg of the product as a white solid; ¹H NMR (300 MHz, DMSO-*d*₆) δ 2.31 (s, 3H), 3.16 (s, 3H), 3.41 (s, 3H), 3.83 (s, 2H), 7.54 (dd, *J =* 2.1, 8.7 Hz, 1H), 7.64 (s, 1H), 7.73 (s, 1H), 7.87 (d, *J* = 8.4 Hz, 1H), 12.46 (br s, 1H); APCI-MS (*m*/*z*) 479.14 (M+H)⁺.

### Example 57

### N1-[4-(2,4-Difluorophenyl)-1,3-thiazol-2-yl]-2-(1,3,6-trimethyl-2,4-dioxo-1,2,3,4-tetrahydrofuro[2,3-d]pyrimidin-5-yl)acetamide

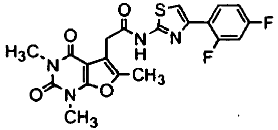

The title compound was prepared according to the general procedure (Method A) by coupling Intermediate 3 (50 mg, 0.178 mmol) with 4-(2,4-difluorophenyl)-1,3-thiazol-2-amine (46 mg, 0.214 mmol) in the presence of sodium hydride (18 mg, 0.445 mmol) in dry toluene (10 ml) to give 46 mg of the product as a white sulid; ¹H NMR (300 MHz, DMSO-*d*₆) δ 2.32 (s, 3H), 3.16 (s, 3H), 3.41 (s, 3H), 3.83 (s, 2H), 7.22 (t, *J* = 8.4 Hz, 1H), 7.38 (t, *J* = 8.7 Hz, 1H), 7.47 (s, 1H), 8.06 (q, *J* = 6.9 Hz, 1H), 12.45 (br s, 1H); APCI-MS (*m*/*z*) 445.26 (M-H)⁻.

### Example 58

### N1-{4-[4-Fluoro-3-(trifluoromethyl)phenyl]-1,3-thiazol-2-yl}-2-(1,3,6-trimethyl-2,4-dioxo-1,2,3,4-tetrahydrofuro[2,3-d]pyrimidin-5-yl)acetamide

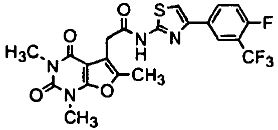

The title compound was prepared according to the general procedure (Method A) by coupling Intermediate 3 (80 mg, 0.285 mmol) with 4-[4-fluoro-3-(trifluoromethyl)phenyl]-1,3-thiazol-2-amine (89 mg, 0.339 mmol) in the presence of sodium hydride (28 mg, 0.708 mmol) in dry toluene (10 ml) to give 70 mg of the product as an off-white solid; ¹H NMR (300 MHz, DMSO-*d*₆) δ 2.32 (s, 3H), 3.15 (s, 3H), 3.41 (s, 3H), 3.83 (s, 2H), 7.61 (t, *J* = 9.6 Hz, 1H), 7.84 (s, I H), 8.25-8.31 (m, 2H), 12.50 (br s, I H); APCI-MS (*m*/*z*) 497.02 (M-H)⁻.

### Example 59

### N1-{4-[3-Fluoro-4-(trifluoromethyl)phenyl]-1,3-thiazol-2-yl}-2-(1,3,6-trimethyl-2,4-dioxo-1,2,3,4-tetrahydrofuro[2,3-d]pyrimidin-5-yl)acetamide

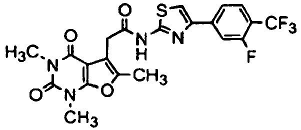

The title compound was prepared according to the general procedure (Method A) by coupling Intermediate 3 (80 mg, 0.285 mmol) with 4-[3-fluoro-4-(trifluoromethyl)phenyl]-1,3-thiazol-2-amine (89 mg, 0.342 mmol) in the presence of sodium hydride (28 mg, 0.713 mmol) in dry toluene (10 ml) to give 61 mg of the product as an off-white solid: ¹H NMR (300 MHz, DMSO-*d*₆) δ 2.32 (s, 3H), 3.15 (s, 3H), 3.41 (s, 3H), 3.84 (s, 2H), 7.87-8.00 (m, 4H), 12.54 (br s, 1H); APCI-MS (*m*/*z*) 533.13 (M+H)⁺.

### Example 60

### N1-{4-[2-Fluoro-4-(trifluoromethyl)phenyl]-1,3-thiazol-2-yl}-2-(1,3,6-trimethyl-2,4-dioxo-1,2,3,4-tetrahydrofuro[2,3-d]pyrimidin-5-yl)acetamide

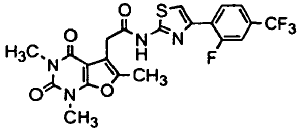

The title compound was prepared according to the general procedure (Method A) by coupling Intermediate 3 (50 mg, 0.178 mmol) with 4-[2-fluoro-4-(trifluoromethyl)phenyl]-1,3-thiazol-2-amine (56 mg, 0.213 mmol) in the presence of sodium hydride (18 mg, 0.445 mmol) in dry toluene (10 ml) to give 20 mg of the product as an off-white solid; ¹H NMR (300 MHz, DMSO-*d*₆) δ 2.32 (s, 3H), 3.16 (s, 3H), 3.42 (s, 3H), 3.84 (s, 2H), 7.70-7.76 (m, 2H), 7.78-7.86 (m, 1H), 8.26 (t, *J*= 7.8 Hz, 1H), 12.55 (br s, I H); APCI-MS (*m*/*z*) 497.07 (M+H)⁺.

### Example 61

### N1-{4-[2-Fluoro-3-(trifluoromethyl)phenyl]-1,3-thiazol-2-yl}-2-(1,3,6-trimethyl-2,4-dioxo-1,2,3,4-tetrahydrofuro[2,3-d]pyrimidin-5-yl)acetamide

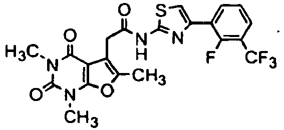

The title compound was prepared according to the general procedure (Method A) by coupling Intermediate 3 (100 mg, 0.357 mmol) with 4-[2-fluoro-3-(trifluoromethyl)phenyl]-1,3-thiazol-2-amine (112 mg, 0.465 mmol) in the presence of sodium hydride (35 mg, 0.875 mmol) in dry toluene (10 ml) to give 85 mg of the product as an off-white solid; ¹H NMR (300 MHz, DMSO-*d*₆) δ 2.31 (s, 3H), 3.15 (s, 3H), 3.41 (s, 3H), 3.84 (s, 2H), 7.53 (t, *J* = 7.8 Hz, 1H), 7.68 (s, 1H), 7.72-7.80 (m, I H), 8.33 (t, *J* = 7.5 Hz, 1H), 12.53 (br s, 1H); APCI-MS (m/z) 497.13 (M+H)⁺.

### Example 62

### N1-{4-[3-Fluoro-5-(trifluoromethyl)phenyl]-1,3-thiazol-2-yl}-2-(1,3,6-trimethyl-2,4-dioxo-1,2,3,4-tetrahydrofuro[2,3-d]pyrimidin-5-yl)acetamide

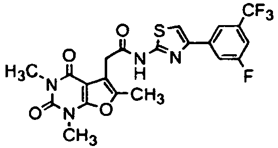

The title compound was prepared according to the general procedure (Method A) by coupling Intermediate 3 (100 mg, 0.356 mmol) with 4-[3-fluoro-5-(trifluoromethyl)phenyl]-1,3-thiazol-2-amine (112 mg, 0.429 mmol) in the presence of sodium hydride (35 mg, 0.891 mmol) in dry toluene (10 ml) to give 70 mg of the product as an off-white solid; ¹H NMR (300 MHz, DMSO-*d*₆) δ 2.32 (s, 3H), 3.15 (s, 3H), 3.41 (s, 3H), 3.84 (s, 2H), 7.65 (d, *J* = 8.1 Hz, 1H), 7.99 (s, 1H), 8.07 (d, *J* = 9.9 Hz, 1H), 8.13 (s, 1H), 12.52 (br s, 1H); APCI-MS (*m*/*z*) 495.49 (M-H)⁻.

### Example 63

### N1-{4-[2-Fluoro-5-(trifluoromethyl)phenyl]-1,3-thiazol-2-yl}-2-(1,3,6-trimethyl-2,4-dioxo-1,2,3,4-tetrahydrofuro[2,3-d]pyrimidin-5-yl)acetamide

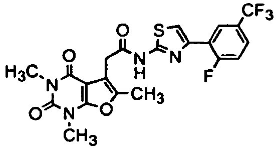

The title compound was prepared according to the general procedure (Method A) by coupling Intermediate 3 (50 mg, 0.178 mmol) with 4-[2-fluoro-5-(trifluoromethyl)phenyl]-1,3-thiazol-2-amine (56 mg, 0.213 mmol) in the presence of sodium hydride (10 mg, 0.416 mmol) in dry toluene (10 ml) to give 17 mg of the product as an off-white solid; ¹H NMR (300 MHz, DMSO-*d*₆) δ 2.32 (s, 3H), 3.15 (s, 3H), 3.41 (s, 3H), 3.84 (s, 2H), 7.55-7.65 (m, 1H), 7.69 (s, 1H), 7.76-7.82 (m, 1H), 8.39-8.45 (m, 1H), 12.55 (br s, I H); APCI-MS (*m*/*z*) 497.06 (M+H)⁺.

### Example 64

### N1-{4-[4-Chloro-3-(trifluoromethyl)phenyl]-1,3-thiazol-2-yl}-2-(1,3,6-trimethyl-2,4-dioxo-1,2,3,4-tetrahydrofuro[2,3-d]pyrimidin-5-yl)acetamide

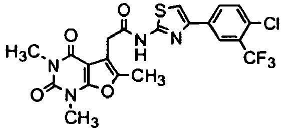

The title compound was prepared according to the general procedure (Method A) by coupling Intermediate 3 (80 mg, 0.285 mmol) with 4-[4-chloro-3-(trifluoromethyl)phenyl]-1,3-thiazol-2-amine (95 mg, 0.342 mmol) in the presence of sodium hydride (29 mg, 0.713 mmol) in dry toluene (10 ml) to give 76 mg of the product as an off-white solid; ¹H NMR (300 MHz, DMSO-*d*₆) δ 2.31 (s, 3H), 3.15 (s, 3H), 3.41 (s, 3H), 3.83 (s, 2H), 7.81 (d, *J*= 8.4 Hz, 1H), 7.92 (s, 1H), 8.20 (d, *J*= 8.1 Hz, 1H), 8.35 (s, 1H), 12.52 (br s, 1H); APCI-MS (*m*/*z*) 513.10 (M+H)⁺.

### Example 65

### N1-{4-[3-Chloro-5-(trifluoromethyl)phenyl]-1,3-thiazol-2-yl}-2-(1,3,6-trimethyl-2,4-dioxo-1,2,3,4-tetrahydrofuro[2,3-d]pyrimidin-5-yl)acetamide

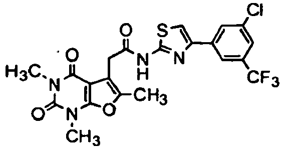

The title compound was prepared according to the general procedure (Method A) by coupling Intermediate 3 (50 mg, 0.178 mmol) with 4-[3-chloro-5-(trifluoromethyl)phenyl]-1,3-thiazol-2-amine (60 mg, 0.214 mmol) in the presence of sodium hydride (18 mg, 0.445 mmol) in dry toluene (10 ml) to give 30 mg of the product as an off-white solid; ¹H NMR (300 MHz, DMSO-*d*₆) δ 2.32 (s, 3H), 3.15 (s, 3H), 3.41 (s, 3H), 3.83 (s, 2H), 7.83 (s, 1H), 8.03 (s, 1H), 8.23 (s, I H), 8.30 (s, I H), 12.52 (br s, I H); APCI-MS (*m*/*z*) 513.07 (M+H)⁺.

### Example 66

### N1-{4-[3-Chloro4-(trifluoromethyl)phenyl]-1,3-thiazol-2-yl}-2-(1,3,6-trimethyl-2,4-dioxo-1,2,3,4-tetrahydrofuro[2,3-d]pyrimidin-5-yl)acetamide

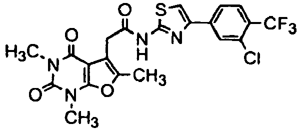

The title compound was prepared according to the general procedure (Method A) by coupling Intermediate 3 (50 mg, 0.178 mmol) with 4-[3-chloro-4-(trifluoromethyl)phenyl]-1,3-thiazol-2-amine (59 mg, 0.214 mmol) in the presence of sodium hydride (17 mg, 0.445 mmol) in dry toluene (10 ml) to give 50 mg of the product as a white solid; ¹H NMR (300 MHz, DMSO-*d*₆) δ 2.32 (s, 3H), 3.15 (s, 3H), 3.41 (s, 3H), 3.84 (s, 2H), 7.93 (d, *J* = 8.1 Hz, 1H), 8.01 (s, I H), 8.06 (d, *J* = 7.8 Hz, 1H), 8.23 (s, 1H), 12.53 (brs, 1H); APCI-MS (*m*/*z*) 513.13 (M)⁺.

### Example 67

### N1-{4-[3-Fluoro-4-(trifluoromethoxy)phenyl]-1,3-thiazol-2-yl}-2-(1,3,6-trimethyl-2,4-dioxo-1,2,3,4-tetrahydrofuro[2,3-d]pyrimidin-5-yl)acetamide

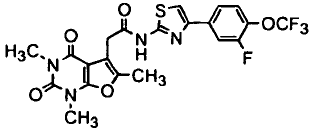

The title compound was prepared according to the general procedure (Method A) by coupling Intermediate 3 (100 mg, 0.356 mmol) with 4-[3-fluoro-4-(trifluoromethoxy)phenyl]-1,3-thiazol-2-amine (119 mg, 0.428 mmol) in the presence of sodium hydride (35 mg, 0.890 mmol) in dry toluene (10 mi) to give 123 mg of the product as an off-white solid; ¹H NMR (300 MHz, DMSO-*d*₆) δ 2.31 (s, 3H), 3.15 (s, 3H), 3.41 (s, 3H), 3.83 (s, 2H), 7.60-7.69 (m, 1H), 7.80-7.90 (m, 2H), 7.95-8.02 (m, 1H), 12.49 (br s, 1H); APCI-MS (*m*/*z*) 513.26 (M+H)⁺.

### Example 68

### N1-{4-[4-Fluoro-3-(trifluoromethoxy)phenyl]-1,3-thiazol-2-yl}-2-(1,3,6-trimethyl-2,4-dioxo-1,2,3,4-tetrahydrofuro[2,3-d]pyrimidin-5-yl)acetamide

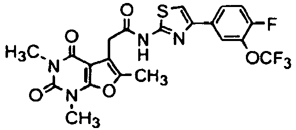

The title compound was prepared according to the general procedure (Method A) by coupling Intermediate 3 (80 mg, 0.285 mmol) with 4-[4-fluoro-3-(trifluoromethoxy)phenyl]-1,3-thiazol-2-amine (95 mg, 0.342 mmol) in the presence of sodium hydride (28 mg, 0.708 mmol) in dry toluene (10 ml) to give 70 mg of the product as an off-white solid; ¹H NMR (300 MHz, DMSO-*d*₆) δ 2.31 (s, 3H), 3.15 (s, 3H), 3.41 (s, 3H), 3.83 (s, 2H), 7.60 (t, *J* = 9.3 Hz, 1H), 7.79 (s, 1H), 8.00-8.06 (m, 2H), 12.48 (br s, 11H); APCI-MS (*m*/*z*) 513.15 (M+H)⁺.

### Example 69

### N1-{4-[4-Chloro-3-(trifluorumethoxy)phenyl]-1,3-thiazol-2-yl}-2-(1,3,6-trimethyl-2,4-dioxo-1,2,3,4-tetrahydrofuro[2,3-d]pyrimidin-5-yl)acetamide

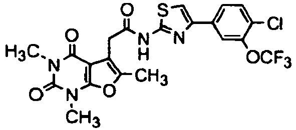

The title compound was prepared according to the general procedure (Method A) by coupling Intermediate 3 (80 mg, 0.285 mmol) with 4-[4-chloro-3-(trifluoromethoxy)phenyl]-1,3-thiazol-2-amine (96 mg, 0.343 mmol) in the presence of sodium hydride (29 mg, 0.713 mmol) in dry toluene (10 ml) to give 62 mg of the product as an off-white solid; ¹H NMR (300 MHz, DMSO-*d*₆) δ 2.31 (s, 3H), 3.15 (s, 3H), 3.41 (s, 3H), 3.83 (s, 2H), 7.77 (d*, J=* 8.4 Hz, 1H), 7.87 (s, 1H), 7.97 (d, *J=* 8.4 Hz, 1H), 8.03 (s, 1H), 12.50 (br s, 1H); APCI-MS (*m*/*z*) 529.05 (M+H)⁺.

### Example 70

### N1-{4-[3-Chloro-4-(trifluoromethoxy)phenyl]-1,3-thiazol-2-yl}-2-(1,3,6-trimethyl-2,4-dioxo-1,2,3,4-tetrahydrofuro[2,3-d]pyrimidin-5-yl)acetamide

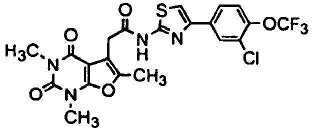

The title compound was prepared according to the general procedure (Method A) by coupling Intermediate 3 (40 mg, 0.142 mmol) with 4-[3-chloro-4-(trifluoromethoxy)phenyl]-1,3-thiazol-2-amine (50 mg, 0.169 mmol) in the presence of sodium hydride (14 mg, 0.333 mmol) in dry toluene (10 ml) to give 17 mg of the product as an off-white solid; ¹H NMR (300 MHz, DMSO-*d*₆) δ 2.29 (s, 3H), 3.13 (s, 3H), 3.39 (s, 3H), 3.81 (s, 2H), 7.63 (d, *J =* 8.4 Hz, I H), 7.83 (s, I H), 7.97 (d, *J* = 8.4 Hz, I H), 8.17 (s, I H), 12.47 (br s, I H); APCI-MS (*m*/*z*) 529.12 (M+H)⁺.

### Example 71

### N1-{4-[3-Chloro-5-(trifluoromethoxyphenyl]-1,3-thiazol-2-yl}-2-(1,3,6-trimethyl-2,4-dioxo-1,2,3,4-tetrahydrofuro[2,3-d]pyrimidin-5-yl)acetamide

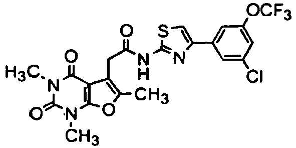

The title compound was prepared according to the general procedure (Method A) by coupling Intermediate 3 (40 mg, 0.142 mmol) with 4-[3-chloro-5-(trifluoromethoxy)phenyl]-1,3-thiazol-2-amine (50 mg, 0.169 mmol) in the presence of sodium hydride (14 mg, 0.333 mmol) in dry toluene (10 ml) to give 29 mg of the product as an off-white solid; ¹H NMR (300 MHz, DMSO-*d*₆) δ 2.31 (s, 3H), 3.15 (s, 3 H), 3.41 (s, 3H), 3.83 (s, 2H), 7.54 (s, 1H), 7.88 (s, 1H), 7.97 (s, 1H), 8.05 (s, 1H), 12.50 (br s, 1H); APCI-MS (*m*/*z*) 529.11 (M+H)⁺.

### Example 72

### N1-{4-[3-Chloro-4-(difluoromethoxy)phenyl]-1,3-thiazol-2-yl}-2-(1,3,6-trimethyl-2,4-dioxo-1,2,3,4-tetrahydrofuro[2,3-d]pyrimidin-5-yl)acetamide

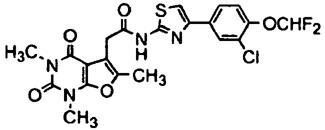

The title compound was prepared according to the general procedure (Method A) by coupling Intermediate 3 (50 mg, 0.178 mmol) with 4-[3-chloro-4-(difluoromethoxy)phenyl]-1,3-thiazol-2-amine (59 mg, 0.214 mmol) in the presence of sodium hydride (17 mg, 0.445 mmol) in dry toluene (10 ml) to give 25 mg of the product as an off-white solid; ¹H NMR (300 MHz, DMSO-*d*₆) δ 2.31 (s, 3H), 3.15 (s, 3H), 3.41 (s, 3H), 3.83 (s, 2H), 7.09 (t, *J*= 72.6 Hz), 7.45 (d, *J*= 8.1 Hz, 1H), 7.76 (s, 1H), 7.91-7.95 (d, *J* = 8.7 Hz, 1H), 8.11 (s, 1H), 12.46 (br s, 1H); ESI-MS (*m*/*z*) 511.08 (M+H)⁺.

### Example 73

### N1-{4-[3-Fluoro-4-(2,2,2-trifluoroethoxy)phenyl]-1,3-thiazol-2-yl}-2-(1,3,6-trimethyl-2,4-dioxo-1,2,3,4-tetrahydrofuro[2,3-d]pyrimidin-5-yl)acetamide

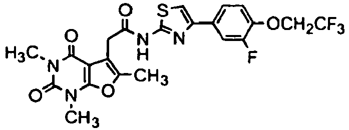

The title compound was prepared according to the general procedure (Method A) by coupling Intermediate 3 (50 mg, 0.178 mmol) with 4-[3-fluoro-4-(2,2,2-trifluoroethoxy)phenyl]-1,3-thiazol-2-amine (62 mg, 0.214 mmol) in the presence of sodium hydride (17 mg, 0.445 mmol) in dry toluene (10 ml) to give 75 mg of the product as an off-white solid; ¹H NMR (300 MHz, DMSO-*d*₆) δ 2.31 (s, 3H), 3.15 (s, 3H), 3.41 (s, 3H), 3.82 (s, 2H), 4.89 (q, *J =* 9.0 Hz, 2H), 7.37 (t, *J* = 9.0 Hz, 1H), 7.63 (s, 1H), 7.70-7.76 (m, 1H), 7.80 (s, 1H), 12.42 (br s, 1H); APCI-MS (*m*/*z*) 527.15 (M+H)⁺.

### Example 74

### N1-{4-[3-Chloro-4-(2,2,2-trifluoroethoxy)phenyl]-1,3-thiazol-2-yl}-2-(1,3,6-trimethyl-2,4-dioxo-1,2,3,4-tetrahydrofuro[2,3-d]pyrimidin-5-yl)acetamide

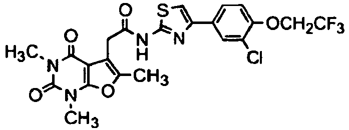

The title compound was prepared according to the general procedure (Method A) by coupling Intermediate 3 (50 mg, 0.178 mmol) with 4-[3-chloro-4-(2,2,2-trifluoroethoxy)phenyl]-1,3-thiazol-2-amine (66 mg, 0.214 mmol) in the presence of sodium hydride (17 mg, 0.445 mmol) in dry toluene (10 ml) to give 21 mg of the product as an off-white solid; ¹H NMR (300 MHz, DMSO-*d*₆) δ 2.31 (s, 3H), 3.15 (s, 3H), 3.41 (s, 3H), 3.82 (s, 2H), 4.92 (q, *J* = 9.0 Hz, 2H), 7.35 (d, *J* = 8.7 Hz, 1H), 7.66 (s, 1H), 7.84-7.90 (m, 1H), 8.01 (s, 1H), 12.42 (br s, 1H); APCI-MS (*m*/*z*) 543.92 (M+H)⁺.

### Example 75

### N1-[4-(2,4,5-Trifluorophenyl)-1,3-thiazol-2-yl]-2-(1,3,6-trimethyl-2,4-dioxo-1,2,3,4-tetrahydrofuro[2,3-d]pyrimidin-5-yl)acetamide

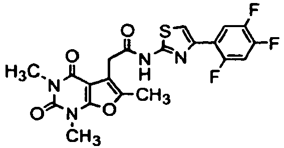

The title compound was prepared according to the general procedure (Method A) by coupling Intermediate 3 (50 mg, 0.178 mmol) with 4-(2,4,5-Trifluorophenyl)-1,3-thiazol-2-amine (49 mg, 0.212 mmol) in the presence of sodium hydride (10 mg, 0.416 mmol) in dry toluene (10 ml) to give 21 mg of the product as an off-white solid; ¹H NMR (300 MHz, DMSO-*d*₆) δ 2.32 (s, 3H), 3.15 (s, 3H), 3.41 (s, 3H), 3.84 (s, 2H), 7.57 (s, 1H), 7.67-7.77 (s, 1H), 7.90-7.99 (m, 1H), 12.49 (br s, 1H); APCI-MS (*m*/*z*) 465.06 (M+H)⁺.

### Example 76

### N1-[4-(3,4,5-Trifluorophenyl)-1,3-thiazol-2-yl]-2-(1,3,6-trimethyl-2,4-dioxo-1,2,3,4-tetrahydrofuro[2,3-d]pyrimidin-5-yl)acetamide

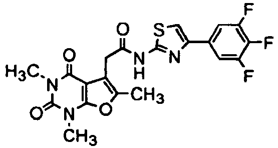

The title compound was prepared according to the general procedure (Method A) by coupling Intermediate 3 (40 mg, 0.142 mmol) with 4-(3,4,5-trifluorophenyl)-1,3-thiazol-2-amine (39 mg, 0.171 mmol) in the presence of sodium hydride (14 mg, 0.355 mmol) in dry toluene (10 ml) to give 25 mg of the product as an off-white solid; ¹H NMR (300 MHz, DMSO-*d*₆) δ 2.31 (s, 3H), 3.15 (s, 3H), 3.41 (s, 3H), 3.83 (s, 2H), 7.76-7.85 (m, 3H), 12.48 (br s, 1H); APCI-MS (*m*/*z*) 465.09 (M+H)⁺.

### Example 77

### N1-[4-(2,3,4-Trifluorophenyl)-1,3-thiazol-2-yl]-2-(1,3,6-trimethyl-2,4-dioxo-1,2,3,4-tetrahydrofuro[2,3-d]pyrimidin-5-yl)acetamide

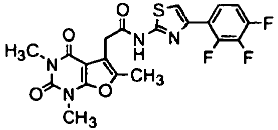

The title compound was prepared according to the general procedure (Method A) by coupling Intermediate 3 (50 mg, 0.178 mmol) with 4-(2,3,4-trifluorophenyl)-1,3-thiazol-2-amine (49 mg, 0.214 mmol) in the presence of sodium hydride (18 mg, 0.445 mmol) in dry toluene (10 ml) to give 45 mg of the product as an off-white solid; ¹H NMR (300 MHz, DMSO-*d*₆) δ 2.32 (s, 3H), 3.16 (s, 3H), 3.41 (s, 3H), 3.84 (s, 2H), 7.43-7.46 (m, 1H), 7.57 (s, 1H), 7.82-7.85 (m, 1H), 12.50 (br s, 1H); ESI-MS (*m*/*z*) 465.06 (M+H)⁺.

### Example 78

### N1-[4-(3-Chloro-2,4-difluorophenyl)-1,3-thiazol-2-yl]-2-(1,3,6-trimethyl-2,4-dioxo-1,2,3,4-tetrahydrofuro[2,3-d]pyrimidin-5-yl)acetamide

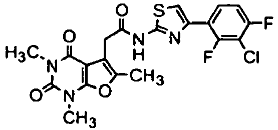

The title compound was prepared according to the general procedure (Method A) by coupling Intermediate 3 (40 mg, 0.142 mmol) with 4-(3-chloro-2,4-difluorophenyl)-1,3-thiazol-2-amine (42 mg, 0.170 mmol) in the presence of sodium hydride (14 mg, 0.333 mmol) in dry toluene (10 ml) to give 20 mg of the product as an off-white solid; ¹H NMR (300 MHz, DMSO-*d*₆) δ 2.31 (s, 3H), 3.15 (s, 3H), 3.41 (s, 3H), 3.83 (s, 2H), 7.45 (t, *J* = 8.1 Hz, 1H), 7.58 (s, 1H), 8.02 (q, *J* = 6.9 Hz, 1H), 12.50 (br s, 1H); APCI-MS (*m*/*z*) 481.09 (M+H)⁺.

### Example 79

### N1-[4-(2,4-Dichloro-5-fluorophenyl)-1,3-thiazol-2-yl]-2-(1,3,6-trimethyl-2,4-dioxo-1,2,3,4-tetrahydrofuro[2,3-d]pyrimidin-5-yl)acetamide

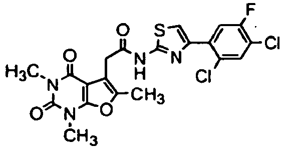

The title compound was prepared according to the general procedure (Method A) by coupling Intermediate 3 (50 mg, 0.178 mmol) with 4-(2,4-dichloro-5-fluorophenyl)-1,3-thiazol-2-amine (56 mg, 0.214 mmol) in the presence of sodium hydride (21 mg, 0.534 mmol) in dry toluene (10 ml) to give 24 mg of the product as an off-white solid; ¹H NMR (300 MHz, DMSO-*d*₆) δ 2.27 (s, 3H), 3.15 (s, 3H), 3.41 (s, 3H), 3.83 (s, 2H), 7.77 (s, 1H), 7.85-7.96 (m, 2H), 12.50 (br s, 1H); APCI-MS (*m*/*z*) 497.06 (M+H)⁺.

### Example 80

### N1-{4-(2,2-Dimethylpropoxy)-3,5-difluorophenyl]-1,3-thiazol-2-yl}-2-(1,3,6-trimethyl-2,4-dioxo-1,2,3,4-tetrahydrofuro[2,3-d]pyrimidin-5-yl)acetamide

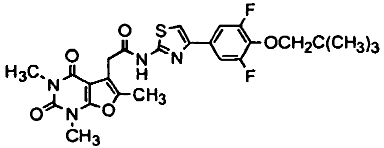

The title compound was prepared according to the general procedure (Method A) by coupling Intermediate 3 (80 mg, 0.285 mmol) with 4-[4-(2,2-dimethylpropoxy)-3,5-difluorophenyl]-1,3-thiazol-2-amine (102 mg, 0.342 mmol) in the presence of sodium hydride (28 mg, 0.713 mmol) in dry toluene (10 ml) to give 41 mg of the product as an off-white solid; ¹H NMR (300 MHz, DMSO-*d*₆) δ 1.01 (s, 9H), 2.31 (s, 3H), 3.15 (s, 3H), 3.41 (s, 3H), 3.80 (s, 2H), 3.82 (s, 2H), 7.64 (d, *J* = 9.3 Hz, 2H), 7.74 (s, I H), 12.44 (br s, 1H); APCI-MS (*m*/*z*) 497.07 (M+H)⁺.

### Example 81

### N1-[4-(3,5-Difluoro-4-isobutoxyphenyl)-1,3-thiazol-2-yl]-2-(1,3,6-trimethyl-2,4-dioxo-1,2,3,4-tetrahydrofuro[2,3-d]pyrimidin-5-yl)acetamide

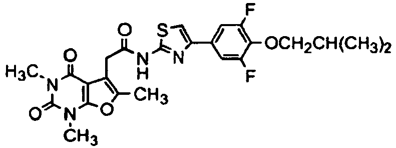

The title compound was prepared according to the general procedure (Method A) by coupling Intermediate 3 (50 mg, 0.178 mmol) with 4-(3,5-difluoro-4-isobutoxyphenyl)-1,3-thiazol-2-amine (60 mg, 0.214 mmol) in the presence of sodium hydride (28 mg, 0.445 mmol) in dry toluene (10 ml) to give 38 mg of the product as an off-white solid; ¹H NMR (300 MHz, DMSO-*d*₆) δ 0.98 (d, *J*= 6.6 Hz, 6H), 1.92-2.03 (m, 1H), 2.31 (s, 3H), 3.15 (s, 3H), 3.41 (s, 3H), 3.82 (s, 2H), 3.91 (d, *J*= 6.3 Hz, 2H), 7.63 (d, *J*= 9.3 Hz, 2H), 7.73 (s, 1H), 12.44 (br s, 1H); APCI-MS (*m*/*z*) 519.19 (M+H)⁺.

### Example 82

### N1-[4-(3,5-Dichloro-4-isobutoxyphenyl)-1,3-thiazol-2-yl]-2-(1,3,6-trimethyl-2,4-dioxo-1,2,3,4-tetrahydrofuro[2,3-d]pyrimidin-5-yl)acetamide

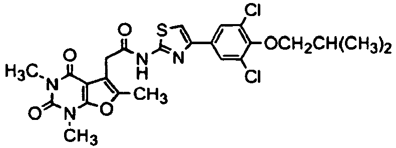

The title compound was prepared according to the general procedure (Method A) by coupling Intermediate 3 (50 mg, 0.178 mmol) with 4-(3,5-dichloro-4-isobutoxyphenyl)-1,3-thiazol-2-amine (68 mg, 0.214 mmol) in the presence of sodium hydride (18 mg, 0.445 mmol) in dry toluene (10 ml) to give 35 mg of the product as an off-white solid; ¹H NMR (300 MHz, DMSO-*d*₆) δ 1.05 (d, *J* = 6.9 Hz, 6H), 2.27 (s, 3H), 3.15 (s, 3H), 3.41 (s, 3H), 3.78-3.83 (m, 4H), 7.82 (s, 1H), 8.01 (s, 2H), 12.45 (br s, 1H); ESI-MS (*m*/*z*) 551.10 (M+H)⁺.

### Example 83

### N1-{4-[2,4-Difluoro-3-(trifluoromethyl)phenyl]-1,3-thiazol-2-yl}-2-(1,3,6-trimethyl-2,4-dioxo-1,2,3,4-tetrahydrofuro[2,3-d]pyrimidin-5-yl)acctamide

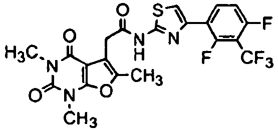

The title compound was prepared according to the general procedure (Method A) by coupling Intermediate 3 (80 mg, 0.285 mmol) with 4-[2,4-difluoro-3-(trifluoromethyl)phenyl]-1,3-thiazol-2-amine (96 mg, 0.343 mmol) in the presence of sodium hydride (29 mg, 0.713 mmol) in dry toluene (10 ml) to give 85 mg of the product as an off-white solid; ¹H NMR (300 MHz, DMSO-*d*₆) δ 2.32 (s, 3H), 3.15 (s, 3H), 3.41 (s, 3H), 3.84 (s, 2H), 7.53 (t, *J* = 9.3 Hz, 1H), 7.64 (s, I H), 8.33-8.35 (m, 1H), 12.53 (br s, 1H); APCI-MS (*m*/*z*) 515.24 (M+H)⁺.

### Example 84

### N1-{4-[3,5-Difluoro-4-(trifluoromethyl)phenyl]-1,3-thiazol-2-yl}-2-(1,3,6-trimethyl-2,4-dioxo-1,2,3,4-tetrahydrofuro[2,3-d]pyrimidin-5-yl)acetamide

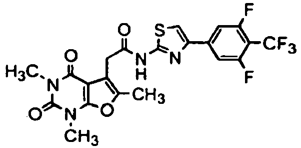

The title compound was prepared according to the general procedure (Method A) by coupling Intermediate 3 (80 mg, 0.285 mmol) with 4-[3,5-difluoro-4-(trifluoromethyl)phenyl]-1,3-thiazol-2-amine (95 mg, 0.342 mmol) in the presence of sodium hydride (28.5 mg, 0.712 mmol) in dry toluene (15 ml) to give 86 mg of the product as an off-white solid; ¹H NMR (300 MHz, DMSO-*d*₆) δ 2.32 (s, 3H), 3.15 (s, 3H), 3.41 (s, 3H), 3.84 (s, 2H), 7.84 (s, 1H), 7.88 (s, 1H), 8.08 (s, 1H), 12.56 (br s, 1H); APCI-MS (*m*/*z*) 515.04 (M+H)⁺.

### Example 85

### N1-{4-[3,5-Difluoro-4-(difluoromethoxy)phenyl]-1,3-thiazol-2-yl}-2-(1,3,6-trimethyl-2,4-dioxo-1,2,3,4-tetrahydrofuro[2,3-d]pyrimidin-5-yl)acetamide

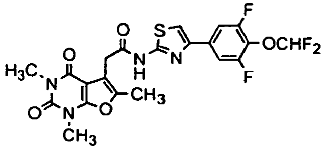

The title compound was prepared according to the general procedure (Method A) by coupling Intermediate 3 (50 mg, 0.178 mmol) with 4-[3,5-difluoro-4-(difluoromethoxy)phenyl]-1,3-thiazol-2-amine (59 mg, 0.212 mmol) in the presence of sodium hydride (17 mg, 0.445 mmol) in dry toluene (10 ml) to give 30 mg of the product as an off-white solid; ¹H NMR (300 MHz, DMSO-*d*₆) δ 2.31 (s, 3H), 3.15 (s, 3H), 3.41 (s, 3H), 3.83 (s, 2H), 7.28 (t, *J*= 72.3 Hz, 1H), 7.79 (d, *J*= 9.9 Hz, 2H), 7.88 (s, 1H), 12.50 (br s, 1H); APCI-MS (*m*/*z*) 513.11 (M+H)⁺.

Using the similar procedure as described in method A or B, additional examples of furopyrimindiedione acetamides with multiple fluorine substitutions (as depicted in Table 3) can be prepared by coupling 2,4-dioxofuropyrimidinyl acetic acid or their ester with an appropriate fluorinated 2-amino-4-arylthiazole selected from Table 2.

**Table 3: Additional examples of fluorinated furopyrimindiedionc acetamide derivatives**

| S No. | Molecular structure | Chemical name | Mol. Formula | Mol Wt. |
|---|---|---|---|---|
| 1 | 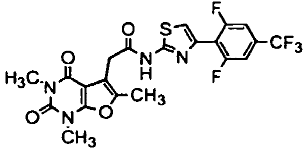 | *N*1-{4-[2,6-Difluoro-4-(trifluoromethyl)phenyl]-1,3-thiazol-2-yl}-2-(1,3,6-trimethyl-2,4-dioxo-1,2,3,4-tetrahydrofuro[2,3-*d*]pyrimidin-5-yl)acetamide | C₂₁H₁₅F₅ N₄O₄S | 514.43 |
| 2 | 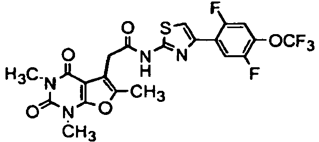 | *N*1-{4-[2,5-Difluuro-4-(trifluoromethoxy)phenyl]-1,3-thiazol-2-yl}-2-(1,3,6-trimethyl-2,4-dioxo-1,2,3,4-tetrahydrofuro[2,3-*d*]pyrimidin-5-yl)acetamide | C₂₁H₁₅F₅ N₄O₅S | 530.43 |
| 3 | 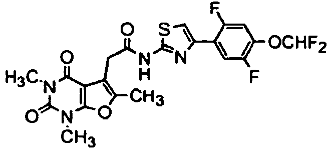 | *N*1-{4-[2,5-Difluoro-4-(difluoromethoxy)phenyl]-1,3-thiazol-2-yl}-2-(1,3,6-trimethyl-2,4-dioxo-1,2,3,4-tetrahydrofuro[2,3-*d*]pyrimidin-5-yl)acetamide | C₂₁H₁₆F₄ N₄O₅S | 512.44 |
| 4 | 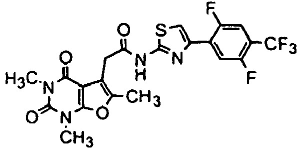 | *N*1-{4-[2,5-Difluoro-4-(trifluorumethyl)phenyl]-1,3-thiazol-2-yl}-2-(1,3,6-trimethyl-2,4-dioxo-1,2,3,4-tetrahydrofuro[2,3-*d*]pyrimidin-5-yl)acetamide | C₂₁H₁₅F₅ N₄O₄S | 514.43 |
| 5 | 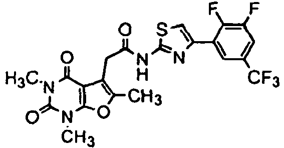 | *N*1-{4-[2,3-Difluoro-5-(trifluoromethyl)phenyl]-1,3-thiazol-2-yl}-2-(1,3,6-trimethyl-2,4-dioxo-1,2,3,4-tetrahydrofuro[2,3-*d*]pyrimidin-5-yl)acetamide | C₂₁H₁₅F₅ N₄O₄S | 514.43 |
| 6 | 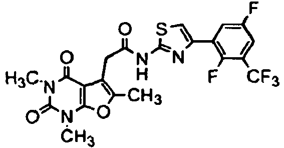 | *N*1-{4-[2,5-Difluoro-3-(trifluoromethyl)phenyl]-1,3-thiazol-2-yl}-2-(1,3,6-trimethyl-2,4-dioxo-1,2,3,4-tetrahydrofuro[2,3-*d*]pyrimidin-5-yl)acetamide | C₂₁H₁₅F₅ N₄O₄S | 514.43 |
| 7 | 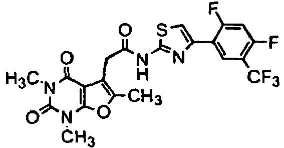 | *N*1-{4-[2,4-Difluoro-5-(trifluoromethyl)phenyl]-1,3-thiazol-2-yl} -2-(1,3,6-trimethyl-2,4-dioxo-1,2,3,4-tetrahydrofuro[2,3-*d*]pyrimidin-5-yl)acetamide | C₇₁H₁₅F₅ N₄O₄S | 514.43 |
| 8 | 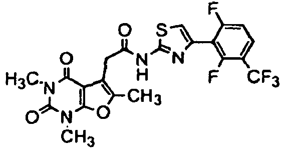 | *N*1-{4-(2,6-Difluoro-5-(trifluoromethyl)phenyl]-1,3-thiazol-2-yl}-2-(1,3,6-trimethyl-2,4-dioxo-1,2,3,4-tetrahydrofuro[2,3-*d*]pyrimidin-5-yl)acetamide | C₂₁H₁₅F₅ N₄O₄S | 514.43 |
| 9 | 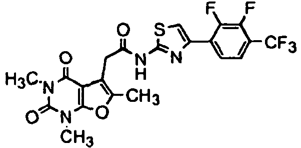 | *N*1-{4-[2,3-Difluoro-4-(trifluoromethyl)phenyl]-1,3-thiazol-2-yl}-2-(1,3,6-trimethyl-2,4-dioxo-1,2,3,4-tetrahydrofuro[2,3-*d*]pyrimidin-5-yl)acetamide | C₂₁H₁₅F₅ N₄O₄S | 514.43 |
| 10 | 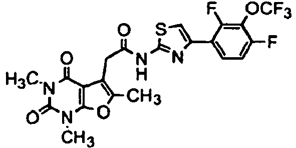 | *N*1-{4-[2,4-Difluoro-3-(trifluoromethoxy)phenyl]-1,3-thiazol-2-yl}-2-(1,3,6-trimethyl-2,4-dioxo-1,2,3,4-tetrahydrofuro[2,3-*d*]pyrimidin-5-yl)acetamide | C₂₁H₁₅F₅ N₄O₅S | 530.43 |
| 11 | 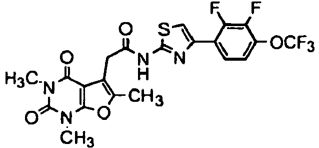 | *N*1-{4-[2,3-Difluoro-4-(trifluoromethoxy)phenyl]-1,3-thiazol-2-yl}-2-(1,3,6-trimethyl-2,4-dioxo-1,2,3,4-tetrahydrofuro[2,3-*d*]pyrimidin-5-yl)acetamide | C₂₁H₁₅F₅ N₄O₅S | 530.43 |
| 12 | 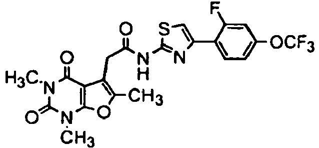 | *N*1-{4-[2-Fluoro-4-(trifluoromethoxy)phenyl)-1,3-thiazol-2-yl}-2-(1,3,6-trimethyl-2,4-dioxo-1,2,3,4-tetrahydrofuro[2,3-*d*]pyrimidin-5-yl)acetamide | C₂₁H₁₆F₄ N₄O₅S | 512.44 |
| 13 | 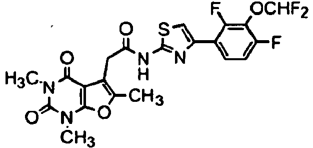 | *N*1-{4-[2,4-Difluoro-3-(difluoromethoxy)phenyl]-1,3-thiazol-2-yl}-2-(1,3,6-trimethyl-2,4-dioxo-1,2,3,4-tetrahydrofuro[2,3-*d*]pyrimidin-5-yl)acetamide | C₂₁H₁₆F₄ N₄O₅S | 512.44 |
| 14 | 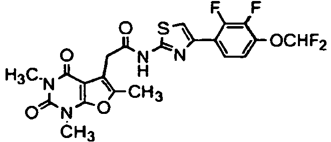 | *N*1-{4-[2,3-Difluoro-4-(difluoromethoxy)phenyl]-1,3-thiazol-2-yl}-2-(1,3,6-trimethyl-2,4-dioxo-1,2,3,4-tetrahydrofuro[2,3-*d*]pyrimidin-5-yl)acetamide | C₂₁H₁₆F₄ N₄O₅S | 512.44 |
| 15 | 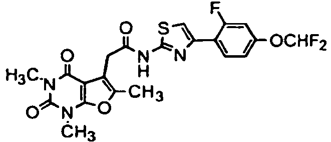 | *N*1-{4-2-Fluoro-4-(difluoromethoxy)phenyl]-1,3-thiazol-2-yl}-2-(1,3,6-trimethyl-2,4-dioxo-1,2,3,4-tetrahydrofuro[2,3-*d*]pyrimidin-5-yl)acetamide | C₂₁H₁₇F₃ N₄O₅S | 494.44 |

### Example 86

### N1-{4-[3,5-Dichloro-4-(difluoromethoxy)phenyl]-1,3-thiazol-2-yl}-2-(,3,6-trimethyl-2,4-dioxo-1,2,3,4-tetrahydrofuro[2,3-d]pyrimidin-5-yl)acetamide

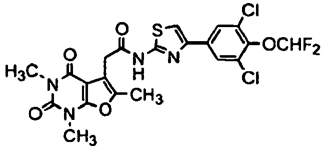

The title compound was prepared according to the general procedure (Method A) by coupling Intermediate 3 (50 mg, 0.170 mmol) with 4-[3,5-dichloro-4-(difluoromethoxy)phenyl]-1,3-thiazol-2-amine (62 mg, 0.200 mmol) in the presence of sodium hydride (16 mg, 0.420 mmol) in dry toluene (10 ml) to give 60 mg of the product as an off-white solid; ¹H NMR (300 MHz, DMSO-*d*₆) δ 2.31 (s, 3H), 3.15 (s, 3H), 3.41 (s, 3H), 3.83 (s, 2H), 7.20 (t, *J*= 72.0 Hz, 1H), 7.94 (s, 1H), 8.12 (s, 2H), 12.49 (br s, 1H); APCI-MS (*m*/*z*) 545.12 (M+H)⁺.

### Example 87

### N1-{4-[3,5-Difluoro-4-(2,2,2-trifluoroethoxy)phenyl]-1,3-thiazol-2-yl-2-(1,3,6-trimethyl-2,4-dioxo-1,2,3,4-tetrahydrofuro[2,3-d]pyrimidin-5-yl)acetamide

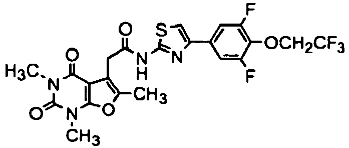

The title compound was prepared according to the general procedure (Method A) by coupling Intermediate 3 (80 mg, 0.285 mmol) with 4-[3,5-difluoro-4-(2,2,2-trifluoroethoxy)phenyl]-1,3-thiazol-2-amine (106 mg, 0.343 mmol) in the presence of sodium hydride (29 mg, 0.713 mmol) in dry toluene (10 ml) to give 64 mg of the product as an off-white solid; ¹H NMR (300 MHz, DMSO-*d*₆) δ 2.31 (s, 3H), 3.15 (s, 3H), 3.41 (s, 3H), 3.83 (s, 2H), 4.87 (q, *J* = 8.7 Hz, 2H), 7.69 (s, 1H), 7.72 (s, 1H), 7.80 (s, 1H), 12.47 (br s, 1H); APCI-MS (*m*/*z*) 545.07 (M+H)⁺.

### Example 88

### N1-{4-[3,5-Dichloro-4-(2,2,2-trifluoroethoxy)phenyl]-1,3-thiazol-2-yl-2-(1,3,6-trimethyl-2,4-dioxo-1,2,3,4-tetrahydrofuro[2,3-d]pyrimidin-5-yl)acetamide

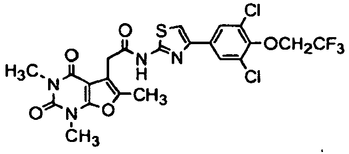

The title compound was prepared according to the general procedure (Method A) by coupling Intermediate 3 (42 mg, 0.150 mmol) with 4-[3,5-dichloro-4-(2,2,2-trifluoroethoxy)phenyl]-1,3-thiazol-2-amine (62 mg, 0.180 mmol) in the presence of sodium hydride (14 mg, 0.370 mmol) in dry toluene (10 ml) to give 17 mg of the product as an off-white solid; ¹H NMR (300 MHz, DMSO-*d*₆) δ 2.31 (s, 3H), 3.15 (s, 3H), 3.41 (s, 3H), 3.83 (s, 2H), 4.76 (q, *J* = 8.7 Hz, 2H), 7.87 (s, 1H), 8.05 (s, 2H), 12.47 (br s, 1H); APCI-MS (*m*/*z*) 577.45 (M+H)⁺.

### Example 89

### N1-{4-[4-(Cyclopropylmethoxy)-3,5-difluorophenyl]-1,3-thiazol-2-yl]-2-(1,3,-trimethyl-2,4-dioxo-1,2,3,4-tetrahydrofuro[2.3-d]pyrimidin-5-yl)acetamide

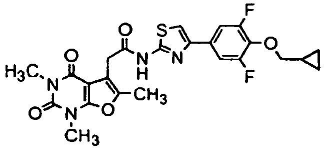

The title compound was prepared according to the general procedure (Method A) by coupling Intermediate 3 (50 mg, 0.178 mmol) with 4-[4-(cyclopropylmethoxy)-3,5-difluorophenyl]-1,3-thiazol-2-amine (60 mg, 0.214 mmol) in the presence of sodium hydride (17 mg, 0.445 mmol) in dry toluene (10 ml) to give 19 mg of the product as a white solid; ¹H NMR (300 MHz, DMSO-*d*₆) δ 0.23-0.30 (m, 2H), 0.50-0.56 (m, 2H), 1.18-1.24 (m, 1H), 2.31 (s, 3H), 3.15 (s, 3H), 3.41 (s, 3H), 3.83 (s, 2H), 3.97 (d, *J*= 7.2 Hz, 2H), 7.62 (s, 1H), 7.74 (s, 1H), 12.45 (br s, 1H); APCI-MS (*m*/*z*) 517.14 (M+H)⁺.

### Example 90

### N1-{4-[4-Fluoro-3-(trifluuromethyl)phenyl]-5-methyl-1,3-thiazol-2-yl}-2-(1,3,6-trimethyl-2,4-dioxo-1,2,3,4-tetrahydrofuro[2,3-d]pyrimidin-5-yl)acetamide

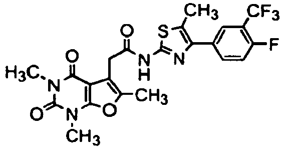

The title compound was prepared according to the general procedure (Method A) by coupling Intermediate 3 (50 mg, 0.178 mmol) with 4-[4-fluoro-3-(trifluoromethyl)phenyl]-5-methyl-1,3-thiazol-2-amine (59 mg, 0.214 mmol) in the presence of sodium hydride (17 mg, 0.445 mmol) in dry toluene (10 ml) to give 26 mg of the product as a white solid; ¹H NMR (300 MHz, CDCl₃) δ 2.41 (s, 3H), 2.47 (s, 3H), 3.47 (s, 3H), 3.54 (s, 3H), 3.70 (s, 2H), 7.18-7.26 (m, 1H), 7.76-7.83 (m, 1H), 7.85-7.90 (m, I H), 11.42 (br s, 1H); APCI-MS (*m*/*z*) 511.08 (M+H)⁺.

### Example 91

### N1-[5-(4-Bromophenyl)-1,3,4-thiadiazol-2-yl]-2-(1,3,6-trimethyl-2,4-dioxo-1,2,3,4-tetrahydrofuro[2,3-d]pyrimidin-5-yl)acetamide

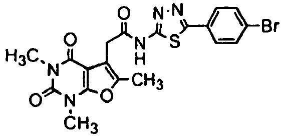

The title compound was prepared according to the general procedure (Method B) by coupling intermediate 4 (45 mg, 0.178 mmol) with 5-(4-bromophenyl)-1,3,4-thiadiazol-2-amine (45 mg, 0.178 mmol) in the presence of EDCI hydrochloride (40 mg, 0.214 mmol), HOBt (7 mg, 0.053 mmol) and DMAP (2 mg, 0.017 mmol) in 1,2-dichloroethane (5 ml) to give 13 mg of the product as a white solid; ¹H NMR (300 MHz, DMSO-*d*₆) δ 2.32 (s, 3H), 3.15 (s, 3H), 3.41 (s, 3H), 4.03 (s, 2H), 7.66-7.75 (m, 2H), 7.84-7.90 (m, 2H), 12.89 (br s, 1H); APCI-MS (*m*/*z*) 490.09 (M)⁺.

### Pharmacological activity

The illustrative examples of the present invention are screened for TRPA1 activity according to a modified procedure described in (a) Tóth, A. et al. Life Sciences, 2003, 73, 487-498. (b) McNamara C, R. et al, Proc. Natl. Acad. Sci. U.S.A., 2007, 104, 13525-13530. The screening of the compounds can be carried out by other methods and procedures known to persons skilled in the art.

### Screening for TRPA1 antagonist using the ⁴⁵Calcium uptake assay:

The inhibition of TRPA1 receptor activation was measured as inhibition of allyl isothiocyanate (AITC) induced cellular uptake of radioactive calcium.

Test compounds were dissolved in 100% DMSO to prepare 10 mM stock and then diluted using plain medium with 0.1% BSA and 1.8 mM CaCl₂ to get the desired concentration. The final concentration of DMSO in the reaction was 0.5% (v/v). Human TRPA1 expressing CHO cells were grown in F-12 DMEM medium with 10% FBS, 1% penicillin-streptomycin solution, and 400 µg / ml of G-418. Rat TRPA I expressing CHO cells were grown in F-12 DMEM medium with 10% FBS, 1% penicillin-streptomycin solution, and 400 µg / ml of Zeocin. Cells were seeded 24 h prior to the assay in 96 well plates so as to get - 50,000 cells per well on the day of experiment. Cells were treated with the test compounds for 10 minutes followed by the addition of AITC at a final concentration of 30 µM (for human TRPA1) and / or 10 µM (for rat TRPA1) and 5 µCi/ml ⁴⁵Ca⁺² for 3 minutes. Cells were washed and lysed using a buffer containing 1% Triton X-100, 0.1 % deoxycholate and 0.1% SDS. Radioactivity in the lysate was measured in a Packard TopCount after addition of liquid scintillant. (Toth et al, Life Sciences (2003) 73, 487-498; McNamara CR et al, Proceedings of the National Academy of Sciences, (2007) 104 , 13525-13530).

Concentration response curves were plotted as a % of maximal response obtained in the absence of test antagonist. IC₅₀ values can be calculated from concentration response curve by nonlinear regression analysis using GraphPad PRISM software.

The compounds prepared were tested using the above assay procedure and the results obtained are given in Table 4. Percentage inhibition at concentrations of 1.0 µM and 10.0 µM are given in the table along with IC₅₀ (nM) details for selected examples. The IC₅₀ (nM) values of the compounds are set forth in Table 4 wherein "A" refers to an IC₅₀ value of less than 50 nM, "B" refers to IC₅₀ value in range of 50.01 to 100.0 nM and "C" refers to IC₅₀ value of more than 100.0 nM

**Table 4: In-vitro screening results of compounds of invention:**

| | **human TRPA1** | | | **rat TRPA1** | | |
|---|---|---|---|---|---|---|
| Examples | Percentage inhibition | | IC₅₀ value (nM) | Percentage inhibition | | IC₅₀ value (nM) |
| | at 1.0 µM | at 10.0 µM | | at 1.0 µM | at 10.0 µM | |
| Example 1 | -- | -- | C | 96.65 | 100.00 | -- |
| Example 6 | 100 | 99.58 | A | -- | -- | -- |
| Example 7 | 98.76 | 99.75 | A | -- | -- | -- |
| Example 8 | 93.15 | 98.82 | A | 100.00 | 100.00 | A |
| Example 9 | 99.33 | 100 | A | -- | -- | -- |
| Example 10 | 98.54 | 97.71 | A | 97.55 | 100.00 | B |
| Example 13 | 98.53 | 99.36 | A | -- | -- | C |
| Example 14 | 99.39 | 99.74 | A | 100.00 | 100.00 | A |
| Example 15 | 99.9 | 99.85 | A | 100.00 | 98.95 | A |
| Example 16 | 98.51 | 99.17 | A | 99.96 | 98.63 | A |
| Example 17 | 99.94 | 99.97 | A | 97.71 | 99.83 | B |
| Example 18 | 99.9 | 100 | A | 99.90 | 94.08 | B |
| Example 19 | 99.9 | 100 | A | 98.88 | 98.22 | B |
| Example 20 | 98.48 | 97.55 | A | 96.80 | 99.79 | A |
| Example 21 | 99.85 | 99.8 | A | 98.88 | 99.90 | A |
| Example 22 | 99.26 | 98.47 | A | 100.00 | 100.00 | -- |
| Example 23 | 96.35 | 99.02 | A | 92.57 | 95.54 | C |
| Example 24 | 97.4 | 99.85 | A | 100.00 | 9.62 | C |
| Example 25 | 99.33 | 99.83 | A | -- | -- | -- |
| Example 26 | 97.57 | 99.31 | A | 98.59 | 99.87 | -- |
| Example 27 | 97.14 | 99.2 | C | 93.13 | 100.00 | -- |
| Example 28 | 96.71 | 99.31 | C | 98.46 | 100.00 | -- |
| Example 30 | 97.43 | 100 | A | 97.09 | 100.00 | C |
| Example 31 | 98.32 | 99.82 | A | 96.75 | 100.00 | B |
| Example 32 | 99.8 | 99.54 | A | 85.16 | 100.00 | A |
| Example 33 | 97.87 | 98.1 | A | -- | -- | -- |
| Example 34 | 97.05 | 99.72 | A | 91.01 | 99.27 | B |
| Example 35 | 99.85 | 98.38 | A | -- | -- | A |
| Example 36 | 99.73 | 99.6 | A | 100.00 | 100.00 | B |
| Example 37 | 99.9 | 99.74 | A | 100.00 | 100.00 | B |
| Example 38 | 99.79 | 99.9 | A | 67.01 | 100.00 | C |
| Example 39 | 94.18 | 98.68 | A | -- | -- | C |
| Example 40 | 93.2 | 99.17 | A | 99.20 | 99.57 | A |
| Example 41 | 99.56 | 100 | A | 99.53 | 100.00 | A |
| Example 42 | 96.13 | 99.9 | A | -- | -- | A |
| Example 43 | 99.23 | 99.7 | A | 89.21 | 100.00 | B |
| Example 44 | 100 | 99.51 | A | 71.04 | 99.25 | A |
| Example 45 | 90.67 | 98.14 | A | -- | -- | -- |
| Example 46 | 99.85 | 100 | A | 96.12 | 99.54 | B |
| Example 47 | 99.18 | 99.8 | A | 86.03 | 100.00 | A |
| Example 48 | 96.59 | 95.12 | A | 99.58 | 99.16 | A |
| Example 49 | 95.58 | 98.26 | A | 86.62 | 97.70 | A |
| Example 50 | 99.64 | 98.35 | -- | 99.87 | 100 | -- |
| Example 51 | 99.77 | 99.96 | -- | 100 | 100 | -- |
| Example 52 | 99.08 | 99.15 | -- | 100 | 100 | -- |
| Example 53 | 99.73 | 97.28 | -- | 100 | 100 | -- |
| Example 54 | -- | -- | A | -- | -- | B |
| Example 55 | 95.83 | 99.03 | -- | 97.66 | 100 | -- |
| Example 58 | -- | -- | A | -- | -- | A |
| Example 59 | -- | -- | A | -- | -- | B |
| Example 60 | -- | -- | A | 99.32 | 99.65 | B |
| Example 61 | -- | -- | A | -- | -- | A |
| Example 62 | -- | -- | A | -- | -- | A |
| Example 63 | -- | -- | B | 97.63 | 99.80 | -- |
| Example 64 | -- | -- | A | -- | -- | A |
| Example 65 | -- | -- | A | 99.79 | 99.80 | B |
| Example 66 | 99.38 | 99.85 | | 99.85 | 98.50 | -- |
| Example 67 | -- | -- | A | -- | -- | A |
| Example 68 | -- | -- | A | -- | -- | A |
| Example 69 | -- | -- | A | -- | -- | A |
| Example 70 | -- | -- | A | -- | -- | A |
| Example 71 | -- | -- | A | -- | -- | B |
| Example 72 | -- | -- | A | 100 | 100 | C |
| Example 73 | -- | -- | A | 100 | 100 | -- |
| Example 74 | -- | -- | A | 100 | 100 | A |
| Example 77 | -- | -- | A | 99.15 | 98.55 | -- |
| Example 78 | -- | - | A | 94.5 | 94.8 | -- |
| Example 80 | -- | -- | A | -- | -- | A |
| Example 81 | -- | -- | A | 100 | 99.25 | B |
| Example 82 | -- | -- | A | 99.96 | 100 | A |
| Example 83 | -- | -- | A | -- | -- | A |
| Example 84 | -- | -- | A | -- | -- | A |
| Example 85 | -- | -- | A | 100 | 100 | -- |
| Example 86 | -- | -- | A | 99.12 | 95.41 | A |
| Example 87 | -- | -- | A | -- | -- | A |
| Example 88 | -- | -- | A | 98.26 | 100 | -- |
| Example 89 | -- | -- | A | -- | 98.81 | B |
| Example 90 | -- | -- | A | 97.98 | 99.52 | -- |

## Claims

1. A compound having the formula (Ia): 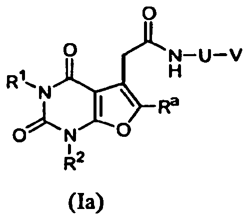 or a pharmaceutically acceptable salt thereof,
wherein,
R¹ and R², which may be the same or different, are independently selected from hydrogen, substituted or unsubstituted alkyl, alkenyl, alkynyl, cycloalkyl, cycloalkylalkyl, arylalkyl, (CR^{x}R^{y})ₙOR^{x}, C(O)R^{x}, C(O)OR^{x}, C(O)NR^{x}R^{y}, (CH₂)ₙNR^{x}R^{y}, (CH₂)ₙCHR^{x}R^{y}, NR^{x}(CR^{x}R^{y})ₙCONR^{x}R^{y}, (CH₂)ₙNR^{x}R^{y} and (CH₂)ₙNHC(O)R^{x};
R^{a} is selected from hydrogen, cyano, substituted or unsubstituted alkyl, alkenyl, alkynyl, cycloalkyl, cycloalkylalkyl, aryl, arylalkyl, biaryl, heteroaryl, heteroarylalkyl, heterocyclic ring, heterocyclylalkyl, OR^{x}, (CR^{x}R^{y})ₙOR^{x}, C(O)R^{x}, C(O)OR^{x}, C(O)NR^{x}R^{y}, S(O)ₘNR^{x}R^{y}, NR^{x}R^{y}, NR^{x}(CR^{x}R^{y})ₙOR^{x}, (CH₂)ₙNR^{x}R^{y}, (CH₂)ₙCHR^{x}R^{y}, NR^{x}(CR^{x}R^{y})ₙC(O)NR^{x}R^{y}, (CH₂)ₙNHC(O)R^{x}, (CH=)ₙNH(CH₂)ₙSO₂R^{x}, (CH₂)ₙNHSO₂R^{x}, SR^{x} and OR^{x};
U is selected from -(CR^{x}R^{y})ₙ-, substituted or unsubstituted aryl, substituted or unsubstituted five membered heterocycles selected from the group consisting of thiazole, isothiazole, oxazole, isoxazole, thiadiazole, oxadiazole, pyrazole, imidazole, furan, thiophene, pyrrole, 1,2,3-triazole and 1, 2, 4-triazole, or substituted or unsubstituted six membered heterocycle selected from the group consisting of pyrimidine, pyridine and pyridazine;
V is selected from hydrogen, cyano, nitro, -NR^{x}R^{y}, halogen, hydroxyl, substituted or unsubstituted alkyl, alkenyl, alkynyl, cycloalkyl, cycloalkylalkyl, cycloalkenyl, haloalkyl, haloalkoxy, cycloalkylalkoxy, aryl, arylalkyl, biaryl, heteroaryl, heteroarylalkyl, heterocyclic ring and heterocyclylalkyl, -C(O)OR^{x}, -OR^{x}, -C(O)NR^{x}R^{y}, -C(O)R^{x} and -SO₂NR^{x}R^{y}; or
alternatively U and V together may form an optionally substituted 3 to 7 membered saturated or unsaturated cyclic ring that may optionally include one or more heteroatoms selected from O, S and N;
at each occurrence, R^{x} and R^{y} are independently selected from hydrogen, hydroxyl, halogen, substituted or unsubstituted alkyl, alkenyl, alkynyl, cycloalkyl, cycloalkylalkyl, cycloalkenyl, aryl, arylalkyl, heteroaryl, heteroarylalkyl, heterocyclic ring and heterocyclylalkyl and
at each occurrence, 'm' and 'n' are independently selected from 0 to 2, both inclusive.

2. The compound according to claim 1, wherein R¹ and R² are (C₁-C₄)alkyl.

3. The compound according to claim 2, wherein (C₁-C₄)alkyl is methyl.

4. The compound according to any of claims 1 to 3, wherein R^{a} is hydrogen or (C₁-C₄)alkyl.

5. The compound according to any of claims 1 to 4, wherein U is thiazole, imidazole, isoxazole, pyrazole, thiadiazole or pyrimidine.

6. The compound according to any of claims 1 to 5, wherein V is substituted or unsubstituted phenyl.

7. The compound according to claim 1 having the structure: 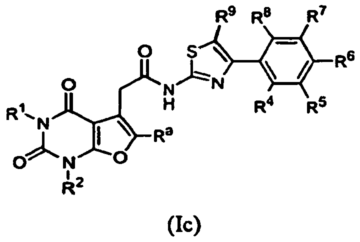 or a pharmaceutically acceptable salt thereof,
wherein,
R¹, R² and R^{a}, which may be the same or different, are each independently hydrogen or (C₁-C₄)alkyl; and
R⁴, R⁵, R⁶, R⁷, R⁸ and R⁹, which may be same or different, are each independently selected from the group comprising of hydrogen, halogen, cyano, hydroxyl, nitro, amino, substituted or unsubstituted alkyl, alkoxy, haloalkyl, haloalkoxy, cycloalkyl, cycloalkylalkyl, cycloalkenyl, cycloalkylalkoxy, aryl, arylalkyl, biaryl, heteroaryl, heteroarylalkyl, heterocyclic ring and heterocyclylalkyl.

8. The compound according to claim 7, wherein R^{a}, R¹ and R² are independently selected from hydrogen and methyl.

9. The compound according to any of claims 7 to 8, wherein R⁴, R⁵, R⁶ and R⁷ are independently selected from the group consisting of hydrogen, fluoro, trifluoromethyl and trifluoromethoxy, R⁸ is hydrogen, and R⁹ is hydrogen or methyl.

10. The compound according to claim 1 selected from:
*N*1-[4-(4-Trifluoromethylphenoxy)phenyl]-2-(1,3-dimethyl-2,4-dioxo-1,2,3,4-tetrahydrofuro[2,3-*d*]pyrimidin-5-yl)acetamide;
*N*1-[2-(2,4-Dichlorophenyl)ethyl]-2-(1,3-dimethyl-2,4-dioxo-1,2,3,4-tetrahydrofuro[2,3-*d*]pyrimidin-5-yl)acetamide;
*N*1-[4-(Trifluoromethyl)-1,3-thiazol-2-yl]-2-(1,3-Dimethyl-2,4-dioxo-1,2,3,4-tetrahydrofuro[2,3-*d*]pyrimidin-5-yl)acetamide;
*N*-(4,6-Difluoro-1,3-benzothiazol-2-yl)-2-(1,3-dimethyl-2,4-dioxo-1,2,3,4-tetrahydrofuro[2,3-*d*]pyrimidin-5-yl)acetamide;
*N*1-[4-(4-Isobutylphenyl)-1,3-thiazol-2-yl]-2-(1,3-dimethyl-2,4-dioxo-1,2,3,4-tetrahydrofuro[2,3-*d*]pyrimidin-5-yl)acetamide;
*N*1-{4-[4-(Trifluoromethyl)phenyl]-1,3-thiazol-2-yl}-2-(1,3-dimethyl-2,4-dioxo-1,2,3,4-tetrahydrofuro[2,3-*d*]pyrimidin-5-yl)acetamide;
*N*1-{4-[3-(Trifluoromethyl)phenyl]-1,3-thiazol-2-yl}-2-(1,3-dimethyl-2,4-dioxo-1,2,3,4-tetrahydrofuro[2,3-*d*]pyrimidin-5-yl)acetamide;
*N*1-[4-(4-Trifluorormethoxyphenyl)-1,3-thiazol-2-yl]-2-(1,3-dimethyl-2,4-dioxo-1,2,3,4-tetrahydrofuro[2,3-*d*]pyrimidin-5-yl)acetamide;
*N*1-[4-(3-Trifluorormethoxyphenyl)-1,3-thiazol-2-yl]-2-(1,3-dimethyl-2,4-dioxo-1,2,3,4-tetrahydrofuro[2,3-*d*]pyrimidin-5-yl)acetamide;
*N*1-[4-(3-Bromo-4-fluorophenyl)-1,3-thiazol-2-yl]-2-(1,3-dimethyl-2,4-dioxo-1,2,3,4-tetrahydrofuro[2,3-*d*]pyrimidin-5-yl)acetamide;
*N*1-[4-(3,4-Difluorophenyl)-1,3-thiazol-2-yl]-2-(1,3-dimethyl-2,4-dioxo-1,2,3,4-tetrahydrofuro[2,3-*d*]pyrimidin-5-yl)acetamide;
*N*1-[4-(2,4-Difluorophenyl)-1,3-thiazol-2-yl]-2-(1,3-dimethyl-2,4-dioxo-1,2,3,4-tetrahydrofuro[2,3-*d*]pyrimidin-5-yl)acetamide;
*N*1-[4-(3,4-Dichlorophenyl)-1,3-thiazol-2-yl]-2-(1,3-dimethyl-2,4-dioxo-1,2,3,4-tetrahydrofuro[2,3-*d*]pyrimidin-5-yl)acetamide;
*N*1-{4-[3-Fluoro-4-(trifluoromethyl)phenyl]-1,3-thiazol-2-yl}-2-(1,3-dimethyl-2,4-dioxo-1,2,3,4-tetrahydrofuro[2,3-*d*]pyrimidin-5-yl)acetamide;
*N*1-{4-[4-Fluoro-3-(trifluoromethyl)phenyl]-1,3-thiazol-2-yl}-2-(1,3-dimethyl-2,4-dioxo-1,2,3,4-tetrahydrofuro[2,3-*d*]pyrimidin-5-yl)acetamide;
*N*1-{4-[4-Fluoro-3-(trifluoromethoxy)phenyl]-1,3-thiazol-2-yl}-2-(1,3-dimethyl-2,4-dioxo-1,2,3,4-tetrahydrofuro[2,3-*d*]pyrimidin-5-yl)acetamide;
*N*1-{4-[3-Fluoro-4-(trifluoromethoxy)phenyl]-1,3-thiazol-2-yl}-2-(1,3-dimethyl-2,4-dioxo-1,2,3,4-tetrahydrofuro[2,3-*d*]pyrimidin-5-yl) acetamide;
*N*1-{4-[4-Chloro-3-(trifluoromethyl)phenyl]-1,3-thiazol-2-yl}-2-(1,3-dimethyl-2,4-dioxo-1,2,3,4-tetrahydrofuro[2,3-*d*]pyrimidin-5-yl)acetamide;
*N*1-{4-[4-Chloro-3-(trifluoromethoxy)phenyl]-1,3-thiazol-2-yl]-2-(1,3-dimethyl-2,4-dioxo-1,2,3,4-tetrahydrofuro[2,3-*d*]pyrimidin-5-yl)acetamide;
*N*1-{4-[2-Fluoro-3-(trifluoromethyl)phenyl]-1,3-thiazol-2-yl}-2-(1,3-dimethyl-2,4-dioxo-1,2,3,4-tetrahydrofuro[2,3-*d*]pyrimidin-5-yl)acetamide;
*N*1-{4-[3-Fluoro-5-(trifluoromethyl)phenyl]-1,3-thiazol-2-yl}-2-(1 ,3-dimethyl-2,4-dioxo-1,2,3,4-tetrahydrofuro[2,3-*d*]pyrimidin-5-yl)acetamide;
*N*1-{4-[2-Fluoro-4-(trifluoromethyl)phenyl]-1,3-thiazol-2-yl}-2-(1,3-dimethyl-2,4-dioxo-1,2,3,4-tetrahydrofuro[2,3-*d*]pyrimidin-5-yl)acetamide;
*N*1-[4-(3-Fluoro-4-difluoromethoxyphenyl)-1,3-thiazol-2-yl]-2-(1,3-dimethyl-2,4-dioxo-1,2,3,4-tetrahydrofuro[2,3-*d*]pyrimidin-5-yl)acetamide;
*N*1-[4-(3-Chloro-4-difluoromethoxyphenyl)-1,3-thiazol-2-yl]-2-(1,3-dimethyl-2,4-dioxo-1,2,3,4-tetrahydrofuro[2,3-*d*]pyrimidin-5-yl)acetamide; and
*N*1-[4-(3-Bromo-4-difluoromethoxyphenyl)-1,3-thiazol-2-yl]-2-(1,3-dimethyl-2,4-dioxo-1,2,3,4-tetrahydrofuro[2,3-*d*]pyrimidin-5-yl)acetamide;
or a pharmaceutically acceptable salt thereof,

11. The compound according to claim 1 selected from:
*N*1-{4-[4-(2,2-Dimethylpropoxy)-3-fluorophenyl]-1,3-thiazol-2-yl}-2-(1,3-dimethyl-2,4-dioxo-1,2,3,4-tetrahydrofuro[2,3-*d*]pyrimidin-5-yl)acetamide;
*N*1-{4-[3-Chloro-4-(2,2-dimethylpropoxy)phenyl]-1,3-thiazol-2-yl}-2-(1,3-dimethyl-2,4-dioxo-1,2,3,4-tetrahydrofuro[2,3-*d*]pyrimidin-5-yl)acetamide;
*N*1-{4-[3-Bromo-4-(2,2-dimethylpropoxy)phenyl]-1,3-thiazol-2-yl}-2-(1,3-dimethyl-2,4-dioxo-1,2,3,4-tetrahydrofuro[2,3-*d*]pyrimidin-5-yl)acetamide;
*N*1-[4-(3-Bromo-4-isobutoxyphenyl)-1,3-thiazol-2-yl]-2-(1,3-dimethyl-2,4-dioxo-1,2,3,4-tetrahydrofuro[2,3-*d*]pyrimidin-5-yl)acetamide;
*N*1-{4-[3-Fluoro-4-(2,2,2-trifluoroethoxy)phenyl]-1,3-thiazol-2-yl}2-(1,3-dimethyl-2,4-dioxo-1,2,3,4-tetrahydrofuro[2,3-*d*]pyrimidin-5-yl)acetamide;
*N*1-{4-[3-Chloro-4-(2,2,2-trifluoroethoxy)phenyl]-1,3-thiazol-2-yl}2-(1,3-dimethyl-2,4-dioxo-1,2,3,4-tetrahydrofuro[2,3-*d*]pyrimidin-5-yl)acetamide;
*N*1-{4-[3-Bromo-4-(2,2,2-trifluoroethoxy)phenyl]-1,3-thiazol-2-yl}2-(1,3-dimethyl-2,4-dioxo-1,2,3,4-tetrahydrofuo[2,3-*d*]pyrimidin-5-yl)acetamide;
*N*1-[4-(2,4,5,-Trifluorophenyl)-1,3-thiazol-2-yl]-2-(1,3-dimethyl-2,4-dioxo-1,2,3,4-tetrahydrofuro[2,3-*d*]pyrimidin-5-yl)acetamide;
*N*1-[4-(2,3,4-Trichlorophenyl)-1,3-thiazol-2-yl]-2-(1,3-dimethyl-2,4-dioxo-1,2,3,4-tetrahydrofuro[2,3*-d*]pyrimidin-5-yl)acetamide;
*N*1-{4-[2,4-Difluoro-3-(trifluommethyl)phenyl]-1,3-thiazol-2-yl}2-(1,3-dimethyl-2,4-dioxo-1,2,3,4-tetrahydrofuro[2,3-*d*]pyrimidin-5-yl)acetamide;
*N*1-{4-[3,5-Difluoro-4-(trifluoromethyl)phenyl]-1,3-thiazol-2-yl}-2-(1,3-dimethyl-2,4-dioxo-1,2,3,4-tetrahydrofuro[2,3-*d*]pyrimidin-5-yl)acetamide;
*N*1-{4-[3,5-Difluoro-4-(difluoromethoxy)phenyl]-1,3-thiazol-2-yl}-2-(1,3-dimethyl-2,4-dioxo-1,2,3,4-tetrahydrofuro[2,3-*d*]pyrimidin-5-yl)acetamide;
*N*1-{4-[3,5-Dichloro-4-(difluoromethoxy)phenyl]-1,3-thiazol-2-yl}-2-(1,3-dimethyl-2,4-dioxo-1,2,3,4-tetrahydrofuro[2,3-*d*]pyrimidin-5-yl)acetamide;
*N*1-{4-[4-(Cyclopropylmethoxy)-3,5-difluorophenyl]-1,3-thiazol-2-yl]-2-(1,3-dimethyl-2,4-dioxo-1,2,3,4-tetrahydrofuro[2,3-*d*]pyrimidin-5-yl)acetamide;
*N*1-{4-(3,5-Difluoro-4-isobutoxyphenyl)-1,3-thiazol-2-yl}2-(1,3-dimethyl-2,4-dioxo-1,2,3,4-tetrahydrofuro[2,3-*d*]pyrimidin-5-yl)acetamide;
*N*1-[4-(3,5-Dichloro-4-isobutoxyphenyl)-1,3-thiazol-2-yl]-2-(1,3-dimethyl-2,4-dioxo-1,2,3,4-tetrahydrofuro[2,3-*d*]pyrimidin-5-yl)acetamide;
*N*1-{4-[3,5-Difluoro-4-(2,2-dimethylpropoxy)phenyl]-1,3-thiazol-2-yl}2-(1,3-dimethyl-2,4-dioxo-1,2,3,4-tetrahydrofuro[2,3-*d*]pyrimidin-5-yl)acetamide;
*N*1-(4-[3,5-Dichloro-4-(2,2-dimethylpropoxy)phenyl]-1,3-thiazol-2-yl}-2-(1,3-dimethyl-2,4-dioxo-1,2,3,4-tetrahydrofuro[2,3-*d*]pyrimidin-5-yl)acetamide;
*N*1-(4-[3-Chloro-5-fluoro-4-(2,2-dimethylpropoxy)phenyl]-1,3-thiazol-2-yl}-2-(1,3-dimethyl-2,4-dioxo-1,2,3,4-tetrahydrofuro[2,3-*d*]pyrimidin-5-yl)acetamide;
*N*1-(4-[3,5-Difluoro-4-(3-methylbutoxy)phenyl]-1,3-thiazol-2-yl}-2-(1,3-dimethyl-2,4-dioxo-1,2,3,4-tetrahydrofuro[2,3-d]pyrimidin-5-yl)acetamide;
*N*1-{4-[3,5-Difluoro-4-(2,2,2-trifluoroethoxy)phenyl]-1,3-thiazol-2-yl}-2-(1,3-dimethyl-2,4-dioxo-1,2,3,4-tetrahydrofuro[2,3-*d*]pyrimidin-5-yl)acetamide;
*N*1-{4-[3,5-Dichloro-4-(2,2,2-trifluoroethoxy)phenyl]-1,3-thiazol-2-yl}2-(1,3-dimethyl-2,4-dioxo-1,2,3,4-tetrahydrofuro[2,3-*d*]pyrimidin-5-yl)acetamide;
*N*1-{4-[3-Chloro-5-fluoro-4-(2,2,2-trifluoroethoxy)phenyl]-1,3-thiazol-2-yl}-2-(1,3-dimethyl-2,4-dioxo-1,2,3,4-tetrahydrofuro[2,3-*d*]pyrimidin-5-yl)acetamide; and
*N*1-{4-[3,5-Difluoro-4-(3,3,3-trifluoropropoxy)phenyl]-1,3-thiazol-2-yl}-2-(1,3-dimethyl-2,4-dioxo-1,2,3,4-tetrahydrofuro[2,3-*d*]pyrimidin-5-yl)acetamide;
or a pharmaceutically acceptable salt thereof,

12. The compound according to claim 1 selected from:
*N*1-{4-[4-(Trifluoromethyl)phenyl]-1,3-thiazol-2-yl}-2-(1,3,6-trimethyl-2,4-dioxo-1,2,3,4-tetrahydrofuro[2,3-*d*]pyrimidin-5-yl)acetamide;
*N*1-{4-[3-(Trifluoromethyl)phenyl]-1,3-thiazol-2-yl}-2-(1,3,6-trimethyl-2,4-dioxo-1,2,3,4-tetrahydrofuro[2,3-*d*]pyrimidin-5-yl)acetamide;
*N*1-{4-[3-(Trifluoromethoxy)phenyl]-1,3-thiazol-2-yl}-2-(1,3,6-trimethyl-2,4-dioxo-1,2,3,4-tetrahydrofuro[2,3-*d*]pyrimidin-5-yl)acetamide;
*N*1-{4-[4-(Trifluoromethoxy)phenyl]-1,3-thiazol-2-yl}-2-(1,3,6-trimethyl-2,4-dioxo-1,2,3,4-tetrahydrofuro[2,3-*d*]pyrimidin-5-yl)acetamide;
*N*1-[4-(3,4-Dichlorophenyl)-1,3-thiazol-2-yl]-2-(1,3,6-trimethyl-2,4-dioxo-1,2,3,4-tetrahydrofuro[2,3-*d*]pyrimidin-5-yl)acetamide;
*N*1-[4-(2,3-Dichlorophenyl)-1,3-thiazol-2-yl]-2-(1,3,6-trimethyl-2,4-dioxo-1,2,3,4-tetrahydrofuro[2,3-*d*]pyrimidin-5-yl)acetamide;
*N*1-[4-(2,4-Dichlorophenyl)-1,3-thiazol-2-yl]-2-(1,3,6-trimethyl-2,4-dioxo-1,2,3,4-tetrahydrofuro[2,3-*d*]pyrimidin-5-yl)acetamide;
*N*1-[4-(2,4-Difluorophenyl)-1,3-thiazol-2-yl]-2-(1,3,6-trimethyl-2,4-dioxo-1,2,3,4-tetrahydrofuro[2,3-*d*]pyrimidin-5-yl)acetamide;
*N*1-{4-[4-Fluoro-3-(trifluoromethyl)phenyl]-1,3-thiazol-2-yl}-2-(1,3,6-trimethyl-2,4-dioxo-1,2,3,4-tetrahydrofuro[2,3-*d*]pyrimidin-5-yl)acetamide;
*N*1-{4-[3-Fluoro-4-(trifluoromethyl)phenyl]-1,3-thiazol-2-yl}-2-(1,3,6-trimethyl-2,4-dioxo-1,2,3,4-tetrahydrofuro[2,3-*d*]pyrimidin-5-yl)acetamide;
*N*1-{4-[2-Fluoro-4-(trifluoromethyl)phenyl]-1,3-thiazol-2-yl}-2-(1,3,6-trimethyl-2,4-dioxo-1,2,3,4-tetrahydrofuro[2,3-*d*]pyrimidin-5-yl)acetamide;
*N*1-{4-[2-Fluoro-3-(trifluoromethyl)phenyl]-1,3-thiazol-2-yl}-2-(1,3,6-trimethyl-2,4-dioxo-1,2,3,4-tetrahydrofuro[2,3-*d*]pyrimidin-5-yl)acetamide;
*N*1-{4-[3-Fluoro-5-(trifluoromethyl)phenyl]-1,3-thiazol-2-yl}-2-(1,3,6-trimethyl-2,4-dioxo-1,2,3,4-tetrahydrofuro[2,3-*d*]pyrimidin-5-yl)acetamide;
*N*1-{4-[2-Fluoro-5-(trifluoromethyl)phenyl]-1,3-thiazol-2-yl}-2-(1,3,6-trimethyl-2,4-dioxo-1,2,3,4-tetrahydrofuro[2,3-*d*]pyrimidin-5-yl)acetamide;
*N*1-{4-[4-Chloro-3-(trifluoromethyl)phenyl]-1,3-thiazol-2-yl}-2-(1,3,6-trimethyl-2,4-dioxo-1,2,3,4-tetrahydrofuro[2,3-*d*]pyrimidin-5-yl)acetamide;
*N*1-{4-[3-Chloro-(trifluoromethyl)phenyl]-1,3-thiazol-2-yl}-2-(1,3,6-trimethyl-2,4-dioxo-1,2,3,4-tetrahydrofuro[2,3-d]pyrimidin-5-yl)acetamide;
*N*1-{4-[3-Chloro-4-(trifluoromethyl)phenyl]-1,3-thiazol-2-yl}-2-(1,3,6-trimethyl-2,4-dioxo-1,2,3,4-tetrahydrofuro[2,3-*d*]pyrimidin-5-yl)acetamide;
*N*1-{4-[3-Fluoro-4-(trifluoromethoxy)phenyl]-1,3-thiazol-2-yl}-2-(1,3,6-trimethyl-2,4-dioxo-1,2,3,4-tetrahydrofuro[2,3-*d*]pyrimidin-5-yl)acetamide;
*N*1-{4-[4-Fluoro-3-(trifluoromethoxy)henyl]-1,3-thiazol-2-yl}-2-(1,3,6-trimethyl-2,4-dioxo-1,2,3,4-tetrahydrofuro[2,3-*d*]pyrimidin-5-yl)acetamide;
*N*1-{4-[4-Chloro-3-(trifluoromethoxy)phenyl]-1,3-thiazol-2-yl}-2-(1,3,6-trimethyl-2,4-dioxo-1,2,3,4-tetrahydrofuro[2,3-*d*]pyrimidin-5-yl)acetamide;
*N*1--{4-[3-Chloro-4-(trifluoromethoxy)phenyl]-1,3-thiazol-2-yl}-2-(1,3,6-trimethyl-2,4-dioxo-1,2,3,4-tetrahydrofuro[2,3-*d*]pyrimidin-5-yl)acetamide; and
*N*1-{4-[3-Chloro-5-(trifluoromethoxyphenyl]-1,3-thiazol-2-yl}-2-(1,3,6-trimethyl-2,4-dioxo-1,2,3,4-tetrahydrofuro[2,3-*d*]pyrimidin-5-yl)acetamide;
or a pharmaceutically acceptable salt thereof,

13. The compound according to claim 1 selected from:
*N*1-{4-[3-Chloro-4-(difluoromethoxy)phenyl]-1,3-thiazol-2-yl}-2-(1,3,6-trimethyl-2,4-dioxo-1,2,3,4-tetrahydrofuro[2,3-*d*]pyrimidin-5-yl)acetamide;
*N*1-{4-[3-Fluoro-4-(2,2,2-trifluoroethoxy)phenyl]-1,3-thiazol-2-yl}-2-(1,3,6-trimethyl-2,4-dioxo-1,2,3,4-tetrahydrofuro[2,3-*d*]pyrimidin-5-yl)acetamide;
*N*1-{4-[3-Chloro-4-(2,2,2-trifluoroethoxy)phenyl]-1,3-thiazol-2-yl}-2-(1,3,6-trimethyl-2,4-dioxo-1,2,3,4-tetrahydrofuro[2,3-*d*]pyrimidin-5-yl)acetamide;
*N*1-[4-(2,4,5-Trifluorophenyl)-1,3-thiazol-2-yl]-2-(1,3,6-trimethyl-2,4-dioxo-1,2,3,4-tetrahydrofuro[2,3-*d*]pyrimidin-5-yl)acetamide;
*N*1-[4-(3,4,5-Trifluorophenyl)-1,3-thiazol-2-yl]-2-(1,3,6-trimethyl-2,4-dioxo-1,2,3,4-tetrahydrofuro[2,3-*d*]pyrimidin-5-yl)acetamide;
*N*1-[4-(2,3,4-Trifluorophenyl)-1,3-thiazol-2-yl]-2-(1,3,6-trimethyl-2,4-dioxo-1,2,3,4-tetrahydrofuro[2,3-*d*]pyrimidin-5-yl)acetamide;
*N*1-[4-(3-Chloro-2,4-difluorophenyl)-1,3-thiazol-2-yl]-2-(1,3,6-trimethyl-2,4-dioxo-1,2,3,4-tetrahydrofuro[2,3-*d*]pyrimidin-5-yl)acetamide;
*N*1-[4-(2,4-Dichloro-5-fluorophenyl)-1,3-thiazol-2-yl]-2-(1,3,6-trimethyl-2,4-dioxo-1,2,3,4-tetrahydrofuro[2,3-*d*]pyrimidin-5-yl)acetamide;
*N*1-{4-(2,2-Dimethylpropoxy)-3,5-difluorophenyl]-1,3-thiazol-2-yl}-2-(1,3,6-trimethyl-2,4-dioxo-1,2,3,4-tetrahydrofuro[2,3-*d*]pyrimidin-5-yl)acetamide;
*N*1-[4-(3,5-Difluoro-4-isobutoxyphenyl)-1,3-thiazol-2-yl]-2-(1,3,6-trimethyl-2,4-dioxo-1,2,3,4-tetrahydrofuro[2,3-*d*]pyrimidin-5-yl)acetamide;
*N*1-[4-(3,5-Dichloro-4-isobutoxyphenyl)-1,3-thiazol-2-yl]-2-(1,3,6-trimethyl-2,4-dioxo-1,2,3,4-tetrahydrofuro[2,3-*d*]pyrimidin-5-yl)acetamide;
*N*1-{4-[2,4-Difluoro-3-(trifluoromethyl)phenyl]-1,3-thiazol-2-yl}-2-(1,3,6-trimethyl-2,4-dioxo-1,2,3,4-tetrahydrofuro[2,3-*d*]pyrimidin-5-yl)acetamide;
*N*1-{4-[3,5-Difluoro-4-(trifluoromethyl)phenyl]-1,3-thiazol-2-yl}-2-(1,3,6-trimethyl-2,4-dioxo-1,2,3,4-tetrahydrofuro[2,3-*d*]pyrimidin-5-yl)acetamide;
*N*1-{4-[3,5-Difluoro-4-(difluoromethoxy)phenyl]-1,3-thiazol-2-yl}-2-(1,3,6-trimethyl-2,4-dioxo-1,2,3,4-tetrahydrofuro[2,3-*d*]pyrimidin-5-yl)acetamide;
*N*1-{4-[3,5-Dichloro-4-(difluoromethoxy)phenyl]-1,3-thiazol-2-yl}-2-(1,3,6-trimethyl-2,4-dioxo-1,2,3,4-tetrahydrofuro[2,3-*d*]pyrimidin-5-yl)acetamide;
*N*1-{4-[3,5-Difluoro-4-(2,2,2-trifluoroethoxy)phenyl]-1,3-thiazol-2-yl}-2-(1,3,6-trimethyl-2,4-dioxo-1,2,3,4-tetrahydrofuro[2,3-*d*]pyrimidin-5-yl)acetamide;
*N*1-{4-[3,5-Dichloro-4-(2,2,2-trifluoroethoxy)phenyl]-1,3-thiazol-2-yl}-2-(1,3,6-trimethyl-2,4-dioxo-1,2,3,4-tetrahydrofuro[2,3,*d*]pyrimidin-5-yl)acetamide;
*N*1-{4-[4-(Cyclopropylmethoxy)-3,5-difluorophenyl]-1,3-thiazol-2-yl]-2-(1,3,6-trimethyl-2,4-dioxo-1,2,3,4-tetrahydrofuro[2,3-*d*]pyrimidin-5-yl)acetamide;
*N*1-{4-[4-Fluoro-3-(trifluoromethyl)phenyl]-5-methyl-1,3-thiazol-2-yl}-2-(1,3,6-trimethyl-2,4-dioxo-1,2,3,4-tetrahydrofuro[2,3-*d*]pyrimidin-5-yl)acetamide; and
*N*1-[5-(4-Bromophenyl)-1,3,4-thiadiazol-2-yl]-2-(1,3,6-trimethyl-2,4-dioxo-1,2,3,4-tetrahydrofuro[2,3-*d*]pyrimidin-5-yl)acetamide;
or a pharmaceutically acceptable salt thereof.

14. The compound according to claim 1 having the structure 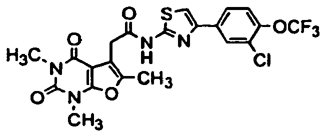 or pharmaceutically acceptable salt thereof.

15. The compound according to claim 1 having the structure 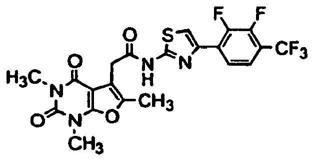 or pharmaceutically acceptable salt thereof.

16. A pharmaceutical composition comprising one or more compounds according to any one of claims 1 to 15, and one or more pharmaceutically acceptable excipients, carriers, diluents or mixture thereof.

17. The compound according to any one of claims 1 to 15, for use in prevention or treatment of a disease or condition associated with TRPA1 function in a subject.

18. The compound according to any one of claims 1 to 15, for use in prevention or treatment of the symptoms of a disease or condition associated with TRPA1 function which is selected from pain, chronic pain, complex regional pain syndrome, neuropathic pain, postoperative pain, rheumatoid arthritic pain, osteoarthritic pain, back pain, visceral pain, cancer pain, algesia, neuralgia, migraine, neuropathies, diabetic neuropathy, sciatica, HIV-related neuropathy, post-herpetic neuralgia, fibromyalgia, nerve injury, ischaemia, neurodegeneration, stroke, post stroke pain, multiple sclerosis, respiratory diseases, asthma, cough, COPD, inflammatory disorders, oesophagitis, gastroeosophagal reflux disorder (GERD), irritable bowel syndrome, inflammatory bowel disease, pelvic hypersensitivity, urinary incontinence, cystitis, burns, psoriasis, eczema, emesis, stomach duodenal ulcer and pruritus.

19. The compound according to any one of claims 1 to 15 for use in the treatment of pain, wherein the compound is in therapeutically effective amount.

20. The compound according to any one of claims 1 to 15 for use in the treatment of pain, wherein the pain is chronic pain, neuropathic pain, rheumatoid pain or osteoarthritic pain.

21. The compound having the structures:

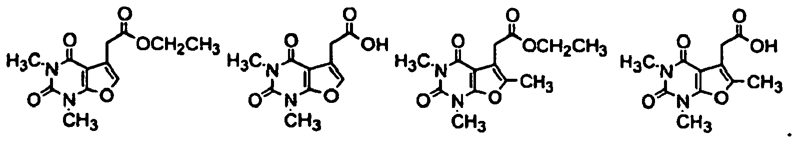

## Patentansprüche

1. Verbindung mit der Formel (la): 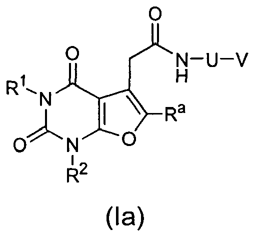 oder ein pharmazeutisch verträgliches Salz davon, worin
R¹ und R², welche gleich oder unterschiedlich sein können, unabhängig voneinander aus Wasserstoff, substituiertem oder unsubstituiertem Alkyl, Alkenyl, Alkinyl, Cycloalkyl, Cycloalkylalkyl, Arylalkyl, (CR^{x}R^{y})ₙOR^{x}, C(O)R^{x}, C(O)OR^{x}, C(O)NR^{x}R^{y}; (CH₂)ₙNR^{x}R^{y}, (CH₂)ₙCHR^{x}R^{y}, NR^{x}(CR^{x}R^{y})ₙCONR^{x}R^{y}, (CH₂)ₙNR^{x}R^{y} und (CH₂)ₙNHC(O)R^{x} ausgewählt ist,
R^{a} aus Wasserstoff, Cyan, substituiertem oder unsubstituiertem Alkyl, Alkenyl, Alkinyl, Cycloalkyl, Cycloalkylalkyl, Aryl, Arylalkyl, Biaryl, Heteroaryl, Heteroarylalkyl, heterocyclischem Ring, Heterocyclylalkyl, OR^{x}, (CR^{x}R^{y})ₙOR^{x}, C(O)R^{x}, C(O)OR^{x}, C(O)NR^{x}R^{y}, S(O)ₘNR^{x}R^{y}, NR^{x}R^{y}, NR^{x}(CR^{x}R^{y})ₙOR^{x}, (CH₂)ₙR^{x}R^{y}, (CH2)ₙCHR^{x}R^{y}, NR^{x}(CR^{x}R^{y})ₙC(O)NR^{x}R^{y}, (CH₂)ₙNHC(O)R^{x}, (CH₂)ₙNH(CH₂)ₙSO₂R^{x}, (CH₂)ₙNHSO₂R^{x}, SR^{x} und OR^{x} ausgewählt ist,
U aus -(CR^{x}R^{y})ₙ-, substituiertem oder unsubstituiertem Aryl, substituierten oder unsubstituierten fünfgliedrigen Heterocyclen, ausgewählt aus der Gruppe, bestehend aus Thiazol, Isothiazol, Oxazol, Isoxazol, Thiadiazol, Oxadiazol, Pyrazol, Imidazol, Furan, Thiophen, Pyrrol, 1,2,3-Triazol und 1,2,4-Triazol, oder substituierten oder unsubstituierten sechsgliedrigen Heterocyclen, ausgewählt aus der Gruppe, bestehend aus Pyrimidin, Pyridin und Pyridazin, ausgewählt ist,
V aus Wasserstoff, Cyan, Nitro, -NR^{x}R^{y}, Halogen, Hydroxyl, substituiertem oder unsubstituiertem Alkyl, Alkenyl, Alkinyl, Cycloalkyl, Cycloalkylalkyl, Cycloalkenyl, Haloalkyl, Haloalkoxy, Cycloalkylalkoxy, Aryl, Arylalkyl, Biaryl, Heteroaryl, Heteroarylalkyl, heterocyclischem Ring und Heterocyclylalkyl, -C(O)OR^{x}, -OR^{x}, - C(O)NR^{x}R^{y}, -(O)R^{x} und -SO₂NR^{x}R^{y} ausgewählt ist, oder alternativ U und V miteinander einen gegebenenfalls substituierten 3- bis 7-gliedrigen gesättigten oder ungesättigten cyclischen Ring bilden können, der gegebenenfalls ein oder mehrere Heteroatome, ausgewählt aus O, S und N, einschließen kann,
bei jedem Auftreten R^{x} und R^{y} unabhängig voneinander aus Wasserstoff, Hydroxyl, Halogen, substituiertem oder unsubstituiertem Alkyl, Alkenyl, Alkinyl, Cycloalkyl, Cycloalkylalkyl, Cycloalkenyl, Aryl, Arylalkyl, Heteroaryl, Heteroarylalkyl, heterocyclischem Ring oder Heterocyclylalkyl ausgewählt sind und
bei jedem Auftreten "m" und "n" unabhängig voneinander aus 0 bis 2, beide einschließend, ausgewählt sind.

2. Verbindung gemäß Anspruch 1, wobei R¹ und R² (C₁-C₄)Alkyl sind.

3. Verbindung gemäß Anspruch 2, wobei (C₁-C₄)Alkyl Methyl ist.

4. Verbindung gemäß einem der Ansprüche 1 bis 3, wobei R^{a} Wasserstoff oder (C₁-C₄)Alkyl ist.

5. Verbindung gemäß einem der Ansprüche 1 bis 4, wobei U Thiazol, Imidazol, Isoxazol, Pyrazol, Thiadiazol oder Pyrimidin ist.

6. Verbindung gemäß einem der Ansprüche 1 bis 5, wobei V substituiertes oder unsubstituiertes Phenyl ist.

7. Verbindung gemäß Anspruch 1 mit der Struktur: 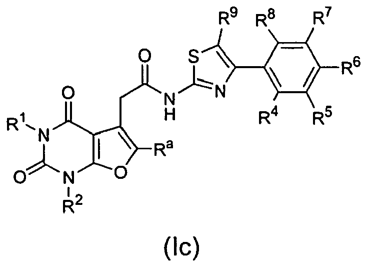 oder ein pharmazeutisch verträgliches Salz davon, wobei
R¹, R² und R^{a}, welche gleich oder unterschiedlich sein können, jeweils unabhängig voneinander Wasserstoff oder (C₁-C₄)Alkyl sind, und
R⁴, R⁵, R⁶, R⁷, R⁸ und R⁹, welche gleich oder unterschiedlich sein können, jeweils unabhängig voneinander aus der Gruppe, umfassend Wasserstoff, Halogen, Cyan, Hydroxyl, Nitro, Amino, substituiertes oder unsubstituiertes Alkyl, Alkoxy, Haloalkyl, Haloalkoxy, Cycloalkyl, Cycloalkylalkyl, Cycloalkenyl, Cycloalkylalkoxy, Aryl, Arylalkyl, Biaryl, Heteroaryl, Heteroarylalkyl, heterocyclischen Ring und Heterocyclylalkyl, ausgewählt sind.

8. Verbindung gemäß Anspruch 7, wobei R^{a}, R¹ und R² unabhängig voneinander aus Wasserstoff und Methyl ausgewählt sind.

9. Verbindung gemäß einem der Ansprüche 7 bis 8, wobei R⁴, R⁵, R⁶ und R⁷ unabhängig voneinander aus der Gruppe, bestehend aus Wasserstoff, Fluor, Trifluormethyl und Trifluormethoxy, ausgewählt sind, R⁸ Wasserstoff ist und R⁹ Wasserstoff oder Methyl ist.

10. Verbindung gemäß Anspruch 1, ausgewählt aus:
*N*1-[4-(4-Trifluoromethylphenoxy)phenyl]-2-(1,3-dimethyl-2,4-dioxo-1,2,3,4-tetrahydrofuro[2,3-*d*]pyrimidin-5-yl)acetamid;
*N*1-[2-(2,4-Dichlorophenyl)ethyl]-2-(1,3-dimethyl-2,4-dioxo-1,2,3,4-tetrahydrofuro[2,3-*d*]pyrimidin-5-yl)acetamid;
*N*1-[4-(Trifluoromethyl)-1,3-thiazol-2-yl]-2-(1,3-dimethyl-2,4-dioxo-1,2,3,4-tetrahydrofuro[2,3-*d*]pyrimidin-5-yl)acetamid;
*N*1-(4,6-Difluoro-1,3-benzothiazol-2-yl)-2-(1,3-dimethyl-2,4-dioxo-1,2,3,4-tetrahydrofuro[2,3-*d*]pyrimidin-5-yl)acetamid;
*N*1-[4-(4-Isobutylphenyl)-1,3-thiazol-2-yl]-2-(1,3-dimethyl-2,4-dioxo-1,2,3,4-tetrahydrofuro[2,3-*d*]pyrimidin-5-yl)acetamid;
*N*1-{4-[4-(Trifluoromethyl)phenyl]-1,3-thiazol-2-yl}-2-(1,3-dimethyl-2,4-dioxo-1,2,3,4-tetrahydrofuro[2,3-*d*]pyrimidin-5-yl)acetamid;
*N*1-{4-[3-(Trifluoromethyl)phenyl]-1,3-thiazol-2-yl}-2-(1,3-dimethyl-2,4-dioxo-1,2,3,4-tetrahydrofuro[2,3-*d*]pyrimidin-5-yl)acetamid;
*N*1-[4-(4-Trifluoromethoxyphenyl)-1,3-thiazol-2-yl]-2-(1,3-dimethyl-2,4-dioxo-1,2,3,4-tetrahydrofuro[2,3-*d*]pyrimidin-5-yl)acetamid;
*N*1-[4-(3-Trifluorormethoxyphenyl)-1,3-thiazol-2-yl]-2-(1,3-dimethyl-2,4-dioxo-1,2,3,4-tetrahydrofuro[2,3-*d*]pyrimidin-5-yl)acetamid;
*N*1-[4-(3-Bromo-4-fluorophenyl)-1,3-thiazol-2-yl]-2-(1,3-dimethyl-2,4-dioxo-1,2,3,4-tetrahydrofuro[2,3-*d*]pyrimidin-5-yl)acetamid;
*N*1-[4-(3,4-Difluorophenyl)-1,3-thiazol-2-yl]-2-(1,3-dimethyl-2,4-dioxo-1,2,3,4-tetrahydrofuro[2,3-*d*]pyrimidin-5-yl)acetamid;
*N*1-[4-(2,4-Difluorophenyl)-1,3-thiazol-2-yl]-2-(1,3-dimethyl-2,4-dioxo-1,2,3,4-tetrahydrofuro[2,3-*d*]pyrimidin-5-yl)acetamid;
*N*1-[4-(3,4-Dichlorophenyl)-1,3-thiazol-2-yl]-2-(1,3-dimethyl-2,4-dioxo-1,2,3,4-tetrahydrofuro[2,3-*d*]pyrimidin-5-yl)acetamid;
*N*1-{4-[3-Fluoro-4-(trifluoromethyl)phenyl]-1,3-thiazol-2-yl}-2-(1,3-dimethyl-2,4-dioxo-1,2,3,4-tetrahydrofuro[2,3-*d*]pyrimidin-5-yl)acetamid;
*N*1-{4-[4-Fluoro-3-(trifluoromethyl)phenyl]-1,3-thiazol-2-yl}-2-(1,3-dimethyl-2,4-dioxo-1,2,3,4-tetrahydrofuro[2,3-*d*]pyrimidin-5-yl)acetamid;
*N*1-{ 4-[4-Fluoro-3-(trifluoromethoxy)phenyl]-1,3-thiazol-2-yl}-2-(1,3-dimethyl-2,4-dioxo-1,2,3,4-tetrahydrofuro[2,3-*d*]pyrimidin-5-yl)acetamid;
*N*1-{4-[3-Fluoro-4-(trifluoromethoxy)phenyl]-1,3-thiazol-2-yl}-2-(1,3-dimethyl-2,4-dioxo-1,2,3,4-tetrahydrofuro[2,3-*d*]pyrimidin-5-yl)acetamid;
*N*1-{4-[4-Chloro-3-(trifluoromethyl)phenyl]-1,3-thiazol-2-yl}-2-(1,3-dimethyl-2,4-dioxo-1,2,3,4-tetrahydrofuro[2,3-*d*]pyrimidin-5-yl)acetamid;
*N*1-{4-[4-Chloro-3-(trifluoromethoxy)phenyl]-1,3-thiazol-2-yl]-2-(1,3-dimethyl-2,4-dioxo-1,2,3,4-tetrahydrofuro[2,3-*d*]pyrimidin-5-yl)acetamid;
*N*1-{4-[2-Fluoro-3-(trifluoromethyl)phenyl]-1,3-thiazol-2-yl}-2-(1,3-dimethyl-2,4-dioxo-1,2,3,4-tetrahydrofuro[2,3-*d*]pyrimidin-5-yl)acetamid;
*N*1-{4-[3-Fluoro-5-(trifluoromethyl)phenyl]-1,3-thiazol-2-yl}-2-(1,3-dimethyl-2,4-dioxo-1,2,3,4-tetrahydrofuro[2,3-*d*]pyrimidin-5-yl)acetamid;
*N*1-{4-[2-Fluoro-4-(trifluoromethyl)phenyl]-1,3-thiazol-2-yl}-2-(1,3-dimethyl-2,4-dioxo-1,2,3,4-tetrahydrofuro[2,3-*d*]pyrimidin-5-yl)acetamid;
*N*1-[4-(3-Fluoro-4-difluoromethoxyphenyl)-1,3-thiazol-2-yl]-2-(1,3-dimethyl-2,4-dioxo-1,2,3,4-tetrahydrofuro[2,3-*d*]pyrimidin-5-yl)acetamid;
*N*1-[4-(3-Chloro-4-difluoromethoxyphenyl)-1,3-thiazol-2-yl]-2-(1,3-dimethyl-2,4-dioxo-1,2,3,4-tetrahydrofuro[2,3-*d*]pyrimidin-5-yl)acetamid; und
N1-[4-(3-Bromo-4-difluoromethoxyphenyl)-1,3-thiazol-2-yl]-2-(1,3-dimethyl-2,4-dioxo-1,2,3,4-tetrahydrofuro[2,3-*d*]pyrimidin-5-yl)acetamid; oder ein pharmazeutisch verträgliches Salz davon.

11. Verbindung gemäß Anspruch 1, ausgewählt aus
*N*1-{4-[4-(2,2-Dimethylpropoxy)-3-fluorophenyl]-1,3-thiazol-2-yl}-2-(1,3-dimethyl-2,4-dioxo-1,2,3,4-tetrahydrofuro[2,3-*d*]pyrimidin-5-yl)acetamid;
*N*1-{4-[3-Chloro-4-(2,2-dimethylpropoxy)phenyl]-1,3-thiazol-2-yl}-2-(1,3-dimethyl-2,4-dioxo-1,2,3,4-tetrahydrofuro[2,3-*d*]pyrimidin-5-yl)acetamid;
*N*1-{4-[3-Bromo-4-(2,2-dimethylpropoxy)phenyl]-1,3-thiazol-2-yl}-2-(1,3-dimethyl-2,4-dioxo-1,2,3,4-tetrahydrofuro[2,3-*d*]pyrimidin-5-yl)acetamid;
*N*1-[4-(3-Bromo-4-isobutoxyphenyl)-1,3-thiazol-2-yl]-2-(1,3-dimethyl-2,4-dioxo-1,2,3,4-tetrahydrofuro[2,3-*d*]pyrimidin-5-yl)acetamid;
*N*1-{4-[3-Fluoro-4-(2,2,2-trifluoroethoxy)phenyl]-1,3-thiazol-2-yl}2-(1,3-dimethyl-2,4-dioxo-1,2,3,4-tetrahydrofuro[2,3-*d*]pyrimidin-5-yl)acetamid;
*N*1-{4-[3-Chloro-4-(2,2,2-trifluoroethoxy)phenyl]-1,3-thiazol-2-yl}2-(1,3-dimethyl-2,4-dioxo-1,2,3,4-tetrahydrofuro[2,3-*d*]pyrimidin-5-yl)acetamid;
*N*1-{4-[3-Bromo-4-(2,2,2-trifluoroethoxy)phenyl]-1,3-thiazol-2-yl}2-(1,3-dimethyl-2,4-dioxo-1,2,3,4-tetrahydrofuro[2,3-*d*]pyrimidin-5-yl)acetamid;
*N*1-[4-(2,4,5,-Trifluorophenyl)-1,3-thiazol-2-yl]-2-(1,3-dimethyl-2,4-dioxo-1,2,3,4-tetrahydrofuro[2,3-*d*]pyrimidin-5-yl)acetamid;
*N*1-[4-(2,3,4-Trichlorophenyl)-1,3-thiazol-2-yl]-2-(1,3-dimethyl-2,4-dioxo-1,2,3,4-tetrahydrofuro[2,3-*d*]pyrimidin-5-yl)acetamid;
*N*1-{4-[2,4-Difluoro-3-(trifluoromethyl)phenyl]-1,3-thiazol-2-yl}2-(1,3-dimethyl-2,4-dioxo-1,2,3,4-tetrahydrofuro[2,3-*d*]pyrimidin-5-yl)acetamid;
*N*1-{4-[3,5-Difluoro-4-(trifluoromethyl)phenyl]-1,3-thiazol-2-yl}-2-(1,3-dimethyl-2,4-dioxo-1,2,3,4-tetrahydrofuro[2,3-*d*]pyrimidin-5-yl)acetamid;
*N*1-{4-[3,5-Difluoro-4-(difluoromethoxy)phenyl]-1,3-thiazol-2-yl}-2-(1,3-dimethyl-2,4-dioxo-1,2,3,4-tetrahydrofuro[2,3-*d*]pyrimidin-5-yl)acetamid;
*N*1-{4-[3,5-Dichloro-4-(difluoromethoxy)phenyl]-1,3-thiazol-2-yl}-2-(1,3-dimethyl-2,4-dioxo-1,2,3,4-tetrahydrofuro[2,3-*d*]pyrimidin-5-yl)acetamid;
*N*1-{4-[4-(Cyclopropylmethoxy)-3,5-difluorophenyl]-1,3-thiazol-2-yl]-2-(1,3-dimethyl-2,4-dioxo-1,2,3,4-tetrahydrofuro[2,3-*d*]pyrimidin-5-yl)acetamid;
*N*1-{4-(3,5-Difluoro-4-isobutoxyphenyl)-1,3-thiazol-2-yl}2-(1,3-dimethyl-2,4-dioxo-1,2,3,4-tetrahydrofuro[2,3-*d*]pyrimidin-5-yl)acetamid;
*N*1-[4-(3,5-Dichloro-4-isobutoxyphenyl)-1,3-thiazol-2-yl]-2-(1,3-dimethyl-2,4-dioxo-1,2,3,4-tetrahydrofuro[2,3-*d*]pyrimidin-5-yl)acetamid;
*N*1-{4-[3,5-Difluoro-4-(2,2-dimethylpropoxy)phenyl]-1,3-thiazol-2-yl}2-(1,3-dimethyl-2,4-dioxo-1,2,3,4-tetrahydrofuro[2,3-*d*]pyrimidin-5-yl)acetamid;
*N*1-{4-[3,5-Dichloro-4-(2,2-dimethylpropoxy)phenyl]-1,3-thiazol-2-yl}-2-(1,3-dimethyl-2,4-dioxo-1,2,3,4-tetrahydrofuro[2,3-*d*]pyrimidin-5-yl)acetamid;
*N*1-{4-[3-Chloro-5-fluoro-4-(2,2-dimethylpropoxy)phenyl]-1,3-thiazol-2-yl}-2-(1,3-dimethyl-2,4-dioxo-1,2,3,4-tetrahydrofuro[2,3-*d*]pyrimidin-5-yl)acetamid;
*N*1-{4-[3,5-Difluoro-4-(3-methylbutoxy)phenyl]-1,3-thiazol-2-yl}-2-(1,3-dimethyl-2,4-dioxo-1,2,3,4-tetrahydrofuro[2,3-*d*]pyrimidin-5-yl)acetamid;
*N*1-{4-[3,5-Difluoro-4-(2,2,2-trifluoroethoxy)phenyl]-1,3-thiazol-2-yl}-2-(1,3-dimethyl-2,4-dioxo-1,2,3,4-tetrahydrofuro[2,3-*d*]pyrimidin-5-yl)acetamid;
*N*1-{4-[3,5-Dichloro-4-(2,2,2-trifluoroethoxy)phenyl]-1,3-thiazol-2-yl}2-(1,3-dimethyl-2,4-dioxo-1,2,3,4-tetrahydrofuro[2,3-*d*]pyrimidin-5-yl)acetamid;
*N*1-{4-[3-Chloro-5-fluoro-4-(2,2,2-trifluoroethoxy)phenyl]-1,3-thiazol-2-yl}-2-(1,3-dimethyl-2,4-dioxo-1,2,3,4-tetrahydrofuro[2,3-*d*]pyrimidin-5-yl)acetamid; und
*N*1-{4-[3,5-Difluoro-4-(3,3,3-trifluoropropoxy)phenyl]-1,3-thiazol-2-yl}-2-(1,3-dimethyl-2,4-dioxo-1,2,3,4-tetrahydrofuro[2,3-*d*]pyrimidin-5-yl)acetamid,
oder ein pharmazeutisch verträgliches Salz davon.

12. Verbindung gemäß Anspruch 1, ausgewählt aus
*N*1-{4-[4-(Trifluoromethyl)phenyl]-1,3-thiazol-2-yl}-2-(1,3,6-trimethyl-2,4-dioxo-1,2,3,4-tetrahydrofuro[2,3-*d*]pyrimidin-5-yl)acetamid;
*N*1-{4-[3-(Trifluoromethyl)phenyl]-1,3-thiazol-2-yl}-2-(1,3,6-trimethyl-2,4-dioxo-1,2,3,4-tetrahydrofuro[2,3-*d*]pyrimidin-5-yl)acetamid;
*N*1-{4-[3-(Trifluoromethoxy)phenyl]-1,3-thiazol-2-yl}-2-(1,3,6-trimethyl-2,4-dioxo-1,2,3,4-tetrahydrofuro[2,3-*d*]pyrimidin-5-yl)acetamid;
*N*1-{4-[4-(Trifluoromethoxy)phenyl]-1,3-thiazol-2-yl}-2-(1,3,6-trimethyl-2,4-dioxo-1,2,3,4-tetrahydrofuro[2,3-*d*]pyrimidin-5-yl)acetamid;
*N*1-[4-(3,4-Dichlorophenyl)-1,3-thiazol-2-yl]-2-(1,3,6-trimethyl-2,4-dioxo-1,2,3,4-tetrahydrofuro[2,3-*d*]pyrimidin-5-yl)acetamid;
*N*1-[4-(2,3-Dichlorophenyl)-1,3-thiazol-2-yl]-2-(1,3,6-trimethyl-2,4-dioxo-1,2,3,4-tetrahydrofuro[2,3-*d*]pyrimidin-5-yl)acetamid;
*N*1-[4-(2,4-Dichlorophenyl)-1,3-thiazol-2-yl]-2-(1,3,6-trimethyl-2,4-dioxo-1,2,3,4-tetrahydrofuro[2,3-*d*]pyrimidin-5-yl)acetamid;
*N*1-[4-(2,4-Difluorophenyl)-1,3-thiazol-2-yl]-2-(1,3,6-trimethyl-2,4-dioxo-1,2,3,4-tetrahydrofuro[2,3-*d*]pyrimidin-5-yl)acetamid;
*N*1-{4-[4-Fluoro-3-(trifluoromethyl)phenyl]-1,3-thiazol-2-yl}-2-(1,3,6-trimethyl-2,4-dioxo-1,2,3,4-tetrahydrofuro[2,3-*d*]pyrimidin-5-yl)acetamid;
*N*1-{4-[3-Fluoro-4-(trifluoromethyl)phenyl]-1,3-thiazol-2-yl}-2-(1,3,6-trimethyl-2,4-dioxo-1,2,3,4-tetrahydrofuro[2,3-*d*]pyrimidin-5-yl)acetamid;
N1-{4-[2-Fluoro-4-(trifluoromethyl)phenyl]-1,3-thiazol-2-yl}-2-(1,3,6-trimethyl-2,4-dioxo-1,2,3,4-tetrahydrofuro[2,3-*d*]pyrimidin-5-yl)acetamid;
*N*1-{4-[2-Fluoro-3-(trifluoromethyl)phenyl]-1,3-thiazol-2-yl}-2-(1,3,6-trimethyl-2,4-dioxo-1,2,3,4-tetrahydrofuro[2,3-*d*]pyrimidin-5-yl)acetamid;
*N*1-{4-[3-Fluoro-5-(trifluoromethyl)phenyl]-1,3-thiazol-2-yl}-2-(1,3,6-trimethyl-2,4-dioxo-1,2,3,4-tetrahydrofuro[2,3-*d*]pyrimidin-5-yl)acetamid;
*N*1-{4-[2-Fluoro-5-(trifluoromethyl)phenyl]-1,3-thiazol-2-yl}-2-(1,3,6-trimethyl-2,4-dioxo-1,2,3,4-tetrahydrofuro[2,3-*d*]pyrimidin-5-yl)acetamid;
*N*1-{4-[4-Chloro-3-(trifluoromethyl)phenyl]-1,3-thiazol-2-yl}-2-(1,3,6-trimethyl-2,4-dioxo-1,2,3,4-tetrahydrofuro[2,3-*d*]pyrimidin-5-yl)acetamid;
*N*1-{4-[3-Chloro-5-(trifluoromethyl)phenyl]-1,3-thiazol-2-yl}-2-(1,3,6-trimethyl-2,4-dioxo-1,2,3,4-tetrahydrofuro[2,3-*d*]pyrimidin-5-yl)acetamid;
*N*1-{4-[3-Chloro-4-(trifluoromethyl)phenyl]-1,3-thiazol-2-yl}-2-(1,3,6-trimethyl-2,4-dioxo-1,2,3,4-tetrahydrofuro[2,3-*d*]pyrimidin-5-yl)acetamid;
*N*1-{4-[3-Fluoro-4-(trifluoromethoxy)phenyl]-1,3-thiazol-2-yl}-2-(1,3,6-trimethyl-2,4-dioxo-1,2,3,4-tetrahydrofuro[2,3-*d*]pyrimidin-5-yl)acetamid;
*N*1-{4-[4-Fluoro-3-(trifluoromethoxy)phenyl]-1,3-thiazol-2-yl}-2-(1,3,6-trimethyl-2,4-dioxo-1,2,3,4-tetrahydrofuro[2,3-*d*]pyrimidin-5-yl)acetamid;
*N*1-{4-[4-Chloro-3-(trifluoromethoxy)phenyl]-1,3-thiazol-2-yl}-2-(1,3,6-trimethyl-2,4-dioxo-1,2,3,4-tetrahydrofuro[2,3-*d*]pyrimidin-5-yl)acetamid;
*N*1-{4-[3-Chloro-4-(trifluoromethoxy)phenyl]-1,3-thiazol-2-yl}-2-(1,3,6-trimethyl-2,4-dioxo-1,2,3,4-tetrahydrofuro[2,3-*d*]pyrimidin-5-yl)acetamid; und
*N*1-{4-[3-Chloro-5-(trifluoromethoxyphenyl]-1,3-thiazol-2-yl}-2-(1,3,6-trimethyl-2,4-dioxo-1,2,3,4-tetrahydrofuro[2,3-*d*]pyrimidin-5-yl)acetamid;
oder ein pharmazeutisch verträgliches Salz davon.

13. Verbindung gemäß Anspruch 1, ausgewählt aus
*N*1-{4-[3-Chloro-4-(difluoromethoxy)phenyl]-1,3-thiazol-2-yl}-2-(1,3,6-trimethyl-2,4-dioxo-1,2,3,4-tetrahydrofuro[2,3-*d*]pyrimidin-5-yl)acetamid;
*N*1-{4-[3-Fluoro-4-(2,2,2-trifluoroethoxy)phenyl]-1,3-thiazol-2-yl}-2-(1,3,6-trimethyl-2,4-dioxo-1,2,3,4-tetrahydrofuro[2,3-*d*]pyrimidin-5-yl)acetamide;
*N*1-{4-[3-Chloro-4-(2,2,2-trifluoroethoxy)phenyl]-1,3-thiazol-2-yl}-2-(1,3,6-trimethyl-2,4-dioxo-1,2,3,4-tetrahydrofuro[2,3-*d*]pyrimidin-5-yl)acetamid;
*N*1-[4-(2,4,5-Trifluorophenyl)-1,3-thiazol-2-yl]-2-(1,3,6-trimethyl-2,4-dioxo-1,2,3,4-tetrahydrofuro[2,3-*d*]pyrimidin-5-yl)acetamid;
*N*1-[4-(3,4,5-Trifluorophenyl)-1,3-thiazol-2-yl]-2-(1,3,6-trimethyl-2,4-dioxo-1,2,3,4-tetrahydrofuro[2,3-*d*]pyrimidin-5-yl)acetamid;
*N*1-[4-(2,3,4-Trifluorophenyl)-1,3-thiazol-2-yl]-2-(1,3,6-trimethyl-2,4-dioxo-1,2,3,4-tetrahydrofuro[2,3-*d*]pyrimidin-5-yl)acetamid;
*N*1-[4-(3-Chloro-2,4-difluorophenyl)-1,3-thiazol-2-yl]-2-(1,3,6-trimethyl-2,4-dioxo-1,2,3,4-tetrahydrofuro[2,3-*d*]pyrimidin-5-yl)acetamid;
*N*1-[4-(2,4-Dichloro-5-fluorophenyl)-1,3-thiazol-2-yl]-2-(1,3,6-trimethyl-2,4-dioxo-1,2,3,4-tetrahydrofuro[2,3-*d*]pyrimidin-5-yl)acetamid;
*N*1-{4-(2,2-Dimethylpropoxy)-3,5-difluorophenyl]-1,3-thiazol-2-yl}-2-(1,3,6-trimethyl-2,4-dioxo-1,2,3,4-tetrahydrofuro[2,3-*d*]pyrimidin-5-yl)acetamid;
*N*1-[4-(3,5-Difluoro-4-isobutoxyphenyl)-1,3-thiazol-2-yl]-2-(1,3,6-trimethyl-2,4-dioxo-1,2,3,4-tetrahydrofuro[2,3-*d*]pyrimidin-5-yl)acetamid;
*N*1-[4-(3,5-Dichloro-4-isobutoxyphenyl)-1,3-thiazol-2-yl]-2-(1,3,6-trimethyl-2,4-dioxo-1,2,3,4-tetrahydrofuro[2,3-*d*]pyrimidin-5-yl)acetamid;
*N*1-{4-[2,4-Difluoro-3-(trifluoromethyl)phenyl]-1,3-thiazol-2-yl}-2-(1,3,6-trimethyl-2,4-dioxo-1,2,3,4-tetrahydrofuro[2,3-*d*]pyrimidin-5-yl)acetamid;
*N*1-{4-[3,5-Difluoro-4-(trifluoromethyl)phenyl]-1,3-thiazol-2-yl}-2-(1,3,6-trimethyl-2,4-dioxo-1,2,3,4-tetrahydrofuro[2,3-*d*]pyrimidin-5-yl)acetamid;
*N*1-{4-[3,5-Difluoro-4-(difluoromethoxy)phenyl]-1,3-thiazol-2-yl}-2-(1,3,6-trimethyl-2,4-dioxo-1,2,3,4-tetrahydrofuro[2,3-*d*]pyrimidin-5-yl)acetamid;
*N*1-{4-[3,5-Dichloro-4-(difluoromethoxy)phenyl]-1,3-thiazol-2-yl}-2-(1,3,6-trimethyl-2,4-dioxo-1,2,3,4-tetrahydrofuro[2,3-*d*]pyrimidin-5-yl)acetamid;
*N*1-{4-[3,5-Difluoro-4-(2,2,2-trifluoroethoxy)phenyl]-1,3-thiazol-2-yl}-2-(1,3,6-trimethyl-2,4-dioxo-1,2,3,4-tetrahydrofuro[2,3-*d*]pyrimidin-5-yl)acetamid;
*N*1-{4-[3,5-Dichloro-4-(2,2,2-trifluoroethoxy)phenyl]-1,3-thiazol-2-yl}-2-(1,3,6-trimethyl-2,4-dioxo-1,2,3,4-tetrahydrofuro[2,3-*d*]pyrimidin-5-yl)acetamid;
*N*1-{4-[4-(Cyclopropylmethoxy)-3,5-difluorophenyl]-1,3-thiazol-2-yl]-2-(1,3,6-trimethyl-2,4-dioxo-1,2,3,4-tetrahydrofuro[2,3-*d*]pyrimidin-5-yl)acetamid;
*N*1-{4-[4-Fluoro-3-(trifluoromethyl)phenyl]-5-methyl-1,3-thiazol-2-yl}-2-(1,3,6-trimethyl-2,4-dioxo-1,2,3,4-tetrahydrofuro[2,3-*d*]pyrimidin-5-yl)acetamid; and
*N*1-[5-(4-Bromophenyl)-1,3,4-thiadiazol-2-yl]-2-(1,3,6-trimethyl-2,4-dioxo-1,2,3,4-tetrahydrofuro[2,3-*d*]pyrimidin-5-yl)acetamid;
oder ein pharmazeutisch verträgliches Salz davon.

14. Verbindung gemäß Anspruch 1 mit der Struktur 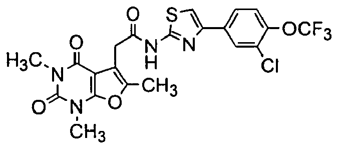 oder ein pharmazeutisch verträgliches Salz davon.

15. Verbindung gemäß Anspruch 1 mit der Struktur 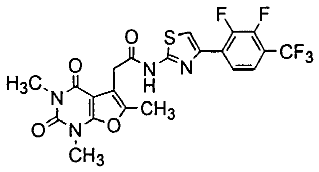 oder ein pharmazeutisch verträgliches Salz davon.

16. Pharmazeutische Zusammensetzung, umfassend eine oder mehrere Verbindungen gemäß einem der Ansprüche 1 bis 15 und ein oder mehrere pharmazeutisch verträgliche Exzipienten, Träger, Verdünnungsmittel oder ein Gemisch davon.

17. Verbindung gemäß einem der Ansprüche 1 bis 15 zur Verwendung in der Verhinderung oder Behandlung einer Krankheit oder Bedingung, verknüpft mit TRPA1 Funktion, in einem Subjekt.

18. Verbindung gemäß einem der Ansprüche 1 bis 15 zur Verwendung in der Verhinderung oder Behandlung der Symptome einer Krankheit oder Bedingung, verknüpft mit TRPA1 Funktion, welche aus Schmerzen, chronischen Schmerzen, komplexem regionalem Schmerzsyndrom, neuropathischem Schmerz, postoperativem Schmerz, rheumatoidem arthritischen Schmerz, osteoarthritischem Schmerz, Rückenschmerzen, viszeralen Schmerzen, Krebsschmerzen, Algesie, Neuralgie, Migräne, Neuropathien, diabetischer Neuropathie, Ischiassyndrom, HIV-bezogener Neuropathie, post-herpetischer Neuralgie, Fibromyalgie, Nervenkrankheit, Ischämie, Neurodegeneration, Schlaganfall, Schmerzen nach Schlaganfall, multipler Sklerose, respiratorischen Krankheiten, Asthma, Husten, COPD, Entzündungsstörungen, Ösophagitis, gastroeosophagal reflux disorder (GERD), Reizdarmsyndrom, chronisch entzündlicher Darmerkrankung, Beckenhypersensitivität, Harninkontinenz, Blasenentzündung, Verbrennungen, Psoriasis, Hautausschlägen, Erbrechen, Zwölffingerdarmgeschwür und Hautjucken, ausgewählt ist.

19. Verbindung gemäß einem der Ansprüche 1 bis 15 zur Verwendung in der Behandlung von Schmerzen, wobei die Verbindung in einer therapeutisch wirksamen Menge vorliegt.

20. Verbindung gemäß einem der Ansprüche 1 bis 15 zur Verwendung in der Behandlung von Schmerzen, wobei die Schmerzen chronische Schmerzen, neuropathische Schmerzen, rheumatoide Schmerzen oder osteoarthritische Schmerzen sind.

21. Verbindung mit den Strukturen:

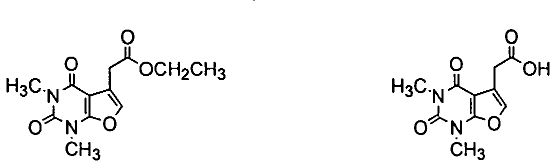

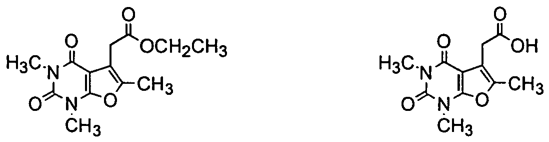

## Revendications

1. Composé ayant la formule (Ia) : 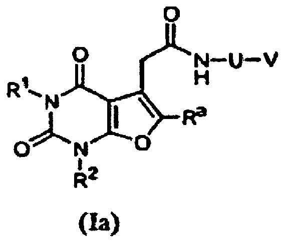 ou un sel pharmaceutiquement acceptable de celui-ci, où,
R¹ et R², qui peuvent être identiques ou différents, sont choisis indépendamment parmi un atome d'hydrogène, les groupes alkyle substitué ou non substitué, alcényle, alcynyle, cycloalkyle, cycloalkylalkyle, arylalkyle, (CR^{x}R^{y})ₙOR^{x}, C(O)R^{x}, C(O)OR^{x}, C(O)NR^{x}R^{y}, (CH₂)NR^{x}R^{y}, (CH₂)ₙCHR^{x}R^{y}, NR^{x}(CR^{x}R^{y})CONR^{x}R^{y}, (CH₂)ₙNR^{x}R^{y} et (CH₂)ₙNHC(O)R^{x} ;
R^{a} est choisi parmi un atome d'hydrogène, les groupes cyano, alkyle substitué ou non substitué, alcényle, alcynyle, cycloalkyle, cycloalkylalkyle, aryle, arylalkyle, biaryle, hétéroaryle, hétéroarylalkyle, cycle hétérocyclique, hétérocyclylalkyle, OR^{x}, (CR^{x}R^{y})ₙOR^{x}, C(O)R^{x}, C(O)OR^{x}, C(O)NR^{x}R^{y}, S(O)ₘNR^{x}R^{y}, NR^{x}R^{y}, NR^{x}(CR^{x}R^{y})ₙOR^{x}, (CH₂)ₙNR^{x}R^{y}, (CH₂)ₙCHR^{x}R^{y}, NR^{x}(CR^{x}R^{y})ₙC(O)NR^{x}R^{y}, (CH₂)ₙNHC(O)R^{x}, (CH₂)ₙNH(CH₂)ₙSO₂R^{x}, (CH₂)ₙNHSO₂R^{x}, SR^{x} et OR^{x} ;
U est choisi parmi les groupes -(CR^{x}R^{y})ₙ-, aryle substitué ou non substitué, des hétérocycles de cinq chaînons substitués ou non substitués choisis dans le groupe constitué de thiazole, isothiazole, oxazole, isoxazole, thiadiazole, oxadiazole, pyrazole, imidazole, furane, thiophène, pyrrole, 1,2,3-triazole et 1,2,4-triazole, ou des hétérocycles de six chaînons substitués ou non substitués choisis dans le groupe constitué de pyrimidine, pyridine et pyridazine ;
V est choisi parmi un atome d'hydrogène, les groupes cyano, nitro, -NR^{x}R^{y}, halogène, hydroxyle, alkyle substitué ou non substitué, alcényle, alcynyle, cycloalkyle, cycloalkylalkyle, cycloalcényle, halogénoalkyle, halogénoalcoxy, cycloalkylalcoxy, aryle, arylalkyle, biaryle, hétéroaryle, hétéroarylalkyle, cycle hétérocyclique et hétérocyclylalkyle, -C(O)OR^{x}, -OR^{x}, -C(O)NR^{x}R^{y}, -C(O)R^{x} et -SO₂NR^{x}R^{y} ; ou
en variante U et V ensemble peuvent former un cycle saturé ou insaturé de 3 à 7 chaînons éventuellement substitué qui peut éventuellement inclure un ou plusieurs hétéroatomes choisis parmi O, S et N ;
à chaque apparition, R^{x} et R^{y} sont choisis indépendamment parmi un atome d'hydrogène, les groupes hydroxyle, halogène, alkyle substitué ou non substitué, alcényle, alcynyle, cycloalkyle, cycloalkylalkyle, cycloalcényle, aryle, arylalkyle, hétéroaryle, hétéroarylalkyle, cycle hétérocyclique et hétérocyclylalkyle et
à chaque apparition, m et n sont choisis indépendamment parmi 0 à 2, les deux compris.

2. Composé selon la revendication 1, où R¹ et R² représentent un groupe alkyle en C₁ à C₄.

3. Composé selon la revendication 2, où le groupe alkyle en C₁ à C₄ est un groupe méthyle.

4. Composé selon l'une quelconque des revendications 1 à 3, où R^{a} représente un atome d'hydrogène ou un groupe alkyle en C₁ à C₄.

5. Composé selon l'une quelconque des revendications 1 à 4, où U représente un cycle thiazole, imidazole, isoxazole, pyrazole, thiadiazole ou pyrimidine.

6. Composé selon l'une quelconque des revendications 1 à 5, où V représente un cycle phényle substitué ou non substitué.

7. Composé selon la revendication 1 ayant la structure : 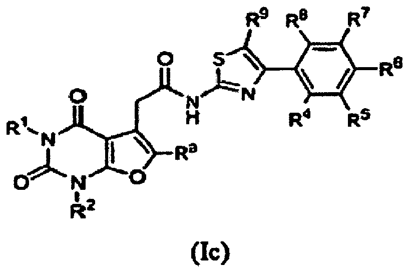 ou un sel pharmaceutiquement acceptable de celui-ci, où
R¹, R² et R^{a}, qui peuvent être identiques ou différents, représentent chacun indépendamment un atome d'hydrogène ou un groupe alkyle en C₁ à C₄ ; et
R⁴, R⁵, R⁶, R⁷, R⁸ et R⁹, qui peuvent être identiques ou différents, sont choisis chacun indépendamment dans le groupe comprenant un atome d'hydrogène, d'halogène, les groupes cyano, hydroxyle, nitro, amino, alkyle substitué ou non substitué, alcoxy, halogénoalkyle, halogénoalcoxy, cycloalkyle, cycloalkylalkyle, cycloalcényle, cycloalkylalcoxy, aryle, arylalkyle, biaryle, hétéroaryle, hétéroarylalkyle, cycle hétérocyclique et hétérocyclylalkyle.

8. Composé selon la revendication 7, où R^{a}, R¹ et R² sont choisis indépendamment parmi un atome d'hydrogène et un groupe méthyle.

9. Composé selon l'une quelconque des revendications 7 ou 8, où R⁴, R⁵, R⁶ et R⁷ sont choisis indépendamment dans le groupe constitué d'un atome d'hydrogène, des groupes fluoro, trifluorométhyle et trifluorométhoxy, R⁸ représente un atome d'hydrogène, et R⁹ représente un atome d'hydrogène ou un groupe méthyle.

10. Composé selon la revendication 1 choisi parmi :
le *N*1-[4-(4-trifluorométhylphénoxy)phényl]-2-(1,3-diméthyl-2,4-dioxo-1,2,3,4-tétrahydrofuro[2,3-*d*]-pyrimidin-5-yl)acétamide ;
le *N*1-[2-(2,4-dichlorophényl)éthyl]-2-(1,3-diméthyl-2,4-dioxo-1,2,3,4-tétrahydrofuro[2,3-*d*]-pyrimidin-5-yl)-acétamide ;
le *N*1-[4-(trifluorométhyl)-1,3-thiazol-2-yl]-2-(1,3-diméthyl-2,4-dioxo-1,2,3,4-tétrahydrofuro[2,3-*d*]-pyrimidin-5-yl)acétamide ;
le *N*-(4,6-difluoro-1,3-benzothiazol-2-yl)-2-(1,3-diméthyl-2,4-dioxo-1,2,3,4-tétrahydrofuro[2,3-*d*]-pyrimidin-5-yl)acétamide ;
le *N*1-[4-(4-isobutylphényl)-1,3-thiazol-2-yl]-2-(1,3-diméthyl-2,4-dioxo-1,2,3,4-tétrahydrofuro[2,3-*d*]-pyrimidin-5-yl)acétamide ;
le *N*1-{4-[4-(trifluorométhyl)phényl]-1,3-thiazol-2-yl}-2-(1,3-diméthyl-2,4-dioxo-1,2,3,4-tétrahydrofuro[2,3-d]-pyrimidin-5-yl)acétamide ;
le *N*1-{4-[3-(trifluorométhyl)phényl]-1,3-thiazol-2-yl}-2-(1,3-diméthyl-2,4-dioxo-1,2,3,4-tétrahydrofuro-[2,3-*d*]pyrimidin-5-yl)acétamide ;
le *N*1-[4-(4-trifluorométhoxyphényl)-1,3-thiazol-2-yl]-2-(1,3-diméthyl-2,4-dioxo-1,2,3,4-tétrahydrofuro-[2,3-*d*]pyrimidin-5-yl)acétamide ;
le *N*1-[4-(3-trifluorométhoxyphényl)-1,3-thiazol-2-yl]-2-(1,3-diméthyl-2,4-dioxo-1,2,3,4-tétrahydrofuro-[2,3-d]pyrimidin-5-yl)acétamide ;
le *N*1-[4-(3-bromo-4-fluorophényl)-1,3-thiazol-2-yl]-2-(1,3-diméthyl-2,4-dioxo-1,2,3,4-tétrahydrofuro-[2,3-d]pyrimidin-5-yl)acétamide ;
le *N*1-[4-(3,4-difluorophényl)-1,3-thiazol-2-yl]-2-(1,3-diméthyl-2,4-dioxo-1,2,3,4-tétrahydrofuro[2,3-*d*]-pyrimidin-5-yl)acétamide ;
le *N*1-[4-(2,4-difluorophényl)-1,3-thiazol-2-yl]-2-(1,3-diméthyl-2,4-dioxo-1,2,3,4-tétrahydrofuro[2,3-*d*]-pyrimidin-5-yl)acétamide ;
le *N*1-[4-(3,4-dichlorophényl)-1,3-thiazol-2-yl]-2-(1,3-diméthyl-2,4-dioxo-1,2,3,4-tétrahydrofuro[2,3-*d*]-pyrimidin-5-yl)acétamide ;
le *N*1-{4-[3-fluoro-4-(trifluorométhyl)phényl]-1,3-thiazol-2-yl}-2-(1,3-diméthyl-2,4-dioxo-1,2,3,4-tétrahydrofuro[2,3-*d*]pyrimidin-5-yl)acétamide ;
le *N*1-{4-[4-fluoro-3-(trifluorométhyl)phényl]-1,3-thiazol-2-yl}-2-(1,3-diméthyl-2,4-dioxo-1,2,3,4-tétrahydrofuro[2,3-*d*]pyrimidin-5-yl)acétamide ;
le *N*1-{4-[4-fluoro-3-(trifluorométhoxy)phényl]-1,3-thiazol-2-yl}-2-(1,3-diméthyl-2,4-dioxo-1,2,3,4-tétrahydrofuro[2,3-*d*]pyrimidin-5-yl)acétamide ;
le *N*1-{4-[3-fluoro-4-(trifluorométhoxy)phényl]-1,3-thiazol-2-yl}-2-(1,3-diméthyl-2,4-dioxo-1,2,3,4-tétrahydrofuro[2,3-*d*]pyrimidin-5-yl)acétamide ;
le *N*1-{4-[4-chloro-3-(trifluorométhyl)phényl]-1,3-thiazol-2-yl}-2-(1,3-diméthyl-2,4-dioxo-1,2,3,4-tétrahydrofuro[2,3-*d*]pyrimidin-5-yl)acétamide ;
le *N*1-{4-[4-chloro-3-(trifluorométhoxy)phényl]-1,3-thiazol-2-yl}-2-(1,3-diméthyl-2,4-dioxo-1,2,3,4-tétrahydrofuro[2,3-*d*]pyrimidin-5-yl)acétamide ;
le *N*1-{4-[2-fluoro-3-(trifluorométhyl)phényl]-1,3-thiazol-2-yl}-2-(1,3-diméthyl-2,4-dioxo-1,2,3,4-tétrahydrofuro[2,3-*d*]pyrimidin-5-yl)acétamide ;
le *N*1-{4-[3-fluoro-5-(trifluorométhyl)phényl]-1,3-thiazol-2-yl}-2-(1,3-diméthyl-2,4-dioxo-1,2,3,4-tétrahydrofuro[2,3-*d*]pyrimidin-5-yl)acétamide ;
le *N*1-{4-[2-fluoro-4-(trifluorométhyl)phényl]-1,3-thiazol-2-yl}-2-(1,3-diméthyl-2,4-dioxo-1,2,3,4-tétrahydrofuro[2,3-*d*]pyrimidin-5-yl)acétamide ;
le *N*1-[4-(3-fluoro-4-difluorométhoxyphényl)-1,3-thiazol-2-yl]-2-(1,3-diméthyl-2,4-dioxo-1,2,3,4-tétrahydrofuro[2,3-*d*]pyrimidin-5-yl)acétamide ;
le *N*1-[4-(3-chloro-4-difluorométhoxyphényl)-1,3-thiazol-2-yl]-2-(1,3-diméthyl-2,4-dioxo-1,2,3,4-tétrahydrofuro[2,3-*d*]pyrimidin-5-yl)acétamide ; et
le *N*1-[4-(3-bromo-4-difluorométhoxyphényl)-1,3-thiazol-2-yl]-2-(1,3-diméthyl-2,4-dioxo-1,2,3,4-tétrahydrofuro[2,3-*d*]pyrimidin-5-yl)acétamide ;
ou un sel pharmaceutiquement acceptable de celui-ci.

11. Composé selon la revendication 1 choisi parmi :
le *N*1-{4-[4-(2,2-diméthylpropoxy)-3-fluorophényl]-1,3-thiazol-2-yl}-2-(1,3-diméthyl-2,4-dioxo-1,2,3,4-tétrahydrofuro[2,3-*d*]pyrimidin-5-yl)acétamide ;
le *N*1-{4-[3-chloro-4-(2,2-diméthylpropoxy)phényl]-1,3-thiazol-2-yl}-2-(1,3-diméthyl-2,4-dioxo-1,2,3,4-tétrahydrofuro[2,3-*d*]pyrimidin-5-yl)acétamide ;
le *N*1-{4-[3-bromo-4-(2,2-diméthylpropoxy)phényl]-1,3-thiazol-2-yl}-2-(1,3-diméthyl-2,4-dioxo-1,2,3,4-tétrahydrofuro[2,3-*d*]pyrimidin-5-yl)acétamide ;
le *N*1-[4-(3-bromo-4-isobutoxyphényl)-1,3-thiazol-2-yl]-2-(1,3-diméthyl-2,4-dioxo-1,2,3,4-tétrahydrofuro-[2,3-d]pyrimidin-5-yl)acétamide ;
le N1-{4-[3-fluoro-4-(2,2,2-trifluoroéthoxy)phényl]-1,3-thiazol-2-yl}-2-(1,3-diméthyl-2,4-dioxo-1,2,3,4-tétrahydrofuro[2,3-*d*]pyrimidin-5-yl)acétamide ;
le *N*1-{4-[3-chloro-4-(2,2,2-trifluoroéthoxy)phényl]-1,3-thiazol-2-yl}-2-(1,3-diméthyl-2,4-dioxo-1,2,3,4-tétrahydrofuro[2,3-*d*]pyrimidin-5-yl)acétamide ;
le *N*1-{4-[3-bromo-4-(2,2,2-trifluoroéthoxy)phényl]-1,3-thiazol-2-yl}-2-(1,3-diméthyl-2,4-dioxo-1,2,3,4-tétrahydrofuro[2,3-*d*]pyrimidin-5-yl)acétamide ;
le *N*1-[4-(2,4,5-trifluorophényl)-1,3-thiazol-2-yl]-2-(1,3-diméthyl-2,4-dioxo-1,2,3,4-tétrahydrofuro-[2,3-d]pyrimidin-5-yl)acétamide ;
le *N*1-[4-(2,3,4-trichlorophényl)-1,3-thiazol-2-yl]-2-(1,3-diméthyl-2,4-dioxo-1,2,3,4-tétrahydrofuro-[2,3-d]pyrimidin-5-yl)acétamide ;
le *N*1-{4-[2,4-difluoro-3-(trifluorométhyl)phényl]-1,3-thiazol-2-yl}-2-(1,3-diméthyl-2,4-dioxo-1,2,3,4-tétrahydrofuro[2,3-*d*]pyrimidin-5-yl)acétamide ;
le *N*1-{4-[3,5-difluoro-4-(trifluorométhyl)phényl]-1,3-thiazol-2-yl}-2-(1,3-diméthyl-2,4-dioxo-1,2,3,4-tétrahydrofuro[2,3-*d*]pyrimidin-5-yl)acétamide ;
le *N*1-{4-[3,5-difluoro-4-(difluorométhoxy)phényl]-1,3-thiazol-2-yl}-2-(1,3-diméthyl-2,4-dioxo-1,2,3,4-tétrahydrofuro[2,3-*d*]pyrimidin-5-yl)acétamide ;
le *N*1-{4-[3,5-dichloro-4-(difluorométhoxy)phényl]-1,3-thiazol-2-yl}-2-(1,3-diméthyl-2,4-dioxo-1,2,3,4-tétrahydrofuro[2,3-*d*]pyrimidin-5-yl)acétamide ;
le *N*1-{4-[4-(cyclopropylméthoxy)-3,5-difluorophényl]-1,3-thiazol-2-yl}-2-(1,3-diméthyl-2,4-dioxo-1,2,3,4-tétrahydrofuro[2,3-*d*]pyrimidin-5-yl)acétamide ;
le *N*1-{4-(3,5-difluoro-4-isobutoxyphényl)-1,3-thiazol-2-yl}-2-(1,3-diméthyl-2,4-dioxo-1,2,3,4-tétrahydrofuro-[2,3-d]pyrimidin-5-yl)acétamide ;
le *N*1-[4-(3,5-dichloro-4-isobutoxyphényl)-1,3-thiazol-2-yl]-2-(1,3-diméthyl-2,4-dioxo-1,2,3,4-tétrahydrofuro-[2,3-d]pyrimidin-5-yl)acétamide ;
le N1-{4-[3,5-difluoro-4-(2,2-diméthylpropoxy)phényl]-1,3-thiazol-2-yl}-2-(1,3-diméthyl-2,4-dioxo-1,2,3,4-tétrahydrofuro[2,3-*d*]pyrimidin-5-yl)acétamide ;
le *N*1-{4-[3,5-dichloro-4-(2,2-diméthylpropoxy)phényl]-1,3-thiazol-2-yl}-2-(1,3-diméthyl-2,4-dioxo-1,2,3,4-tétrahydrofuro[2,3-*d*]pyrimidin-5-yl)acétamide ;
le *N*1-{4-[3-chloro-5-fluoro-4-(2,2-diméthylpropoxy)-phényl]-1,3-thiazol-2-yl}-2-(1,3-diméthyl-2,4-dioxo-1,2,3,4-tétrahydrofuro[2,3-*d*]pyrimidin-5-yl)acétamide ;
le *N*1-{4-[3,5-difluoro-4-(3-méthylbutoxy)phényl]-1,3-thiazol-2-yl}-2-(1,3-diméthyl-2,4-dioxo-1,2,3,4-tétrahydrofuro[2,3-*d*]pyrimidin-5-yl)acétamide ;
le *N*1-{4-[3,5-difluoro-4-(2,2,2-trifluoroéthoxy)-phényl]-1,3-thiazol-2-yl}-2-(1,3-diméthyl-2,4-dioxo-1,2,3,4-tétrahydrofuro[2,3-*d*]pyrimidin-5-yl)acétamide ;
le *N*1-{4-[3,5-dichloro-4-(2,2,2-trifluoroéthoxy)-phényl]-1,3-thiazol-2-yl}-2-(1,3-diméthyl-2,4-dioxo-1,2,3,4-tétrahydrofuro[2,3-*d*]pyrimidin-5-yl)acétamide ;
le *N*1-{4-[3-chloro-5-fluoro-4-(2,2,2-trifluoroéthoxy)-phényl]-1,3-thiazol-2-yl}-2-(1,3-diméthyl-2,4-dioxo-1,2,3,4-tétrahydrofuro[2,3-d]pyrimidin-5-yl)acétamide ;
et
le *N*1-{4-[3,5-difluoro-4-(3,3,3-trifluoropropoxy)-phényl]-1,3-thiazol-2-yl}-2-(1,3-diméthyl-2,4-dioxo-1,2,3,4-tétrahydrofuro[2,3-*d*]pyrimidin-5-yl)acétamide ;
ou un sel pharmaceutiquement acceptable de celui-ci.

12. Composé selon la revendication 1 choisi parmi :
le *N*1-{4-[4-(trifluorométhyl)phényl]-1,3-thiazol-2-yl}-2-(1,3,6-triméthyl-2,4-dioxo-1,2,3,4-tétrahydrofuro-[2,3-d]pyrimidin-5-yl)acétamide ;
le *N*1-{4-[3-(trifluorométhyl)phényl]-1,3-thiazol-2-yl}-2-(1,3,6-triméthyl-2,4-dioxo-1,2,3,4-tétrahydrofuro-[2,3-d]pyrimidin-5-yl)acétamide ;
le *N*1-{4-[3-(trifluorométhoxy)phényl]-1,3-thiazol-2-yl}-2-(1,3,6-triméthyl-2,4-dioxo-1,2,3,4-tétrahydrofuro[2,3-*d*]pyrimidin-5-yl)acétamide ;
le *N*1-{4-[4-(trifluorométhoxy)phényl]-1,3-thiazol-2-yl}-2-(1,3,6-triméthyl-2,4-dioxo-1,2,3,4-tétrahydrofuro[2,3-*d*]pyrimidin-5-yl)acétamide ;
le *N*1-[4-(3,4-dichlorophényl)-1,3-thiazol-2-yl]-2-(1,3,6-triméthyl-2,4-dioxo-1,2,3,4-tétrahydrofuro-[2,3-d]pyrimidin-5-yl)acétamide ;
le *N*1-[4-(2,3-dichlorophényl)-1,3-thiazol-2-yl]-2-(1,3,6-triméthyl-2,4-dioxo-1,2,3,4-tétrahydrofuro-[2,3-d]pyrimidin-5-yl)acétamide ;
le *N*1-[4-(2,4-dichlorophényl)-1,3-thiazol-2-yl]-2-(1,3,6-triméthyl-2,4-dioxo-1,2,3,4-tétrahydrofuro-[2,3-d]pyrimidin-5-yl)acétamide ;
le *N*1-[4-(2,4-difluorophényl)-1,3-thiazol-2-yl]-2-(1,3,6-triméthyl-2,4-dioxo-1,2,3,4-tétrahydrofuro-[2,3-*d*]pyrimidin-5-yl)acétamide ;
le *N*1-{4-[4-fluoro-3-(trifluorométhyl)phényl]-1,3-thiazol-2-yl}-2-(1,3,6-triméthyl-2,4-dioxo-1,2,3,4-tétrahydrofuro-[2,3-*d*]pyrimidin-5-yl)acétamide ;
le *N*1-{4-[3-fluoro-4-(trifluorométhyl)phényl]-1,3-thiazol-2-yl}-2-(1,3,6-triméthyl-2,4-dioxo-1,2,3,4-tétrahydrofuro-[2,3-*d*]pyrimidin-5-yl)acétamide ;
le *N*1-{4-[2-fluoro-4-(trifluorométhyl)phényl]-1,3-thiazol-2-yl}-2-(1,3,6-triméthyl-2,4-dioxo-1,2,3,4-tétrahydrofuro-[2,3-*d*]pyrimidin-5-yl)acétamide ;
le *N*1-{4-[2-fluoro-3-(trifluorométhyl)phényl]-1,3-thiazol-2-yl}-2-(1,3,6-triméthyl-2,4-dioxo-1,2,3,4-tétrahydrofuro-[2,3-*d*]pyrimidin-5-yl)acétamide ;
le *N*1-{4-[3-fluoro-5-(trifluorométhyl)phényl]-1,3-thiazol-2-yl}-2-(1,3,6-triméthyl-2,4-dioxo-1,2,3,4-tétrahydrofuro-[2,3-*d*]pyrimidin-5-yl)acétamide ;
le *N*1-{4-[2-fluoro-5-(trifluorométhyl)phényl]-1,3-thiazol-2-yl}-2-(1,3,6-triméthyl-2,4-dioxo-1,2,3,4-tétrahydrofuro-[2,3-*d*]pyrimidin-5-yl)acétamide ;
le *N*1-{4-[4-chloro-3-(trifluorométhyl)phényl]-1,3-thiazol-2-yl}-2-(1,3,6-triméthyl-2,4-dioxo-1,2,3,4-tétrahydrofuro-[2,3-*d*]pyrimidin-5-yl)acétamide ;
le *N*1-{4-[3-chloro-5-(trifluorométhyl)phényl]-1,3-thiazol-2-yl}-2-(1,3,6-triméthyl-2,4-dioxo-1,2,3,4-tétrahydrofuro-[2,3-*d*]pyrimidin-5-yl)acétamide ;
le *N*1-{4-[3-chloro-4-(trifluorométhyl)phényl]-1,3-thiazol-2-yl}-2-(1,3,6-triméthyl-2,4-dioxo-1,2,3,4-tétrahydrofuro-[2,3-*d*]pyrimidin-5-yl)acétamide ;
le *N*1-{4-[3-fluoro-4-(trifluorométhoxy)phényl]-1,3-thiazol-2-yl}-2-(1,3,6-triméthyl-2,4-dioxo-1,2,3,4-tétrahydrofuro-[2,3-*d*]pyrimidin-5-yl)acétamide ;
le *N*1-{4-[4-fluoro-3-(trifluorométhoxy)phényl]-1,3-thiazol-2-yl}-2-(1,3,6-triméthyl-2,4-dioxo-1,2,3,4-tétrahydrofuro-[2,3-*d*]pyrimidin-5-yl)acétamide ;
le *N*1-{4-[4-chloro-3-(trifluorométhoxy)phényl]-1,3-thiazol-2-yl}-2-(1,3,6-triméthyl-2,4-dioxo-1,2,3,4-tétrahydrofuro-[2,3-*d*]pyrimidin-5-yl)acétamide ;
le *N*1-{4-[3-chloro-4-(trifluorométhoxy)phényl]-1,3-thiazol-2-yl}-2-(1,3,6-triméthyl-2,4-dioxo-1,2,3,4-tétrahydrofuro-[2,3-*d*]pyrimidin-5-yl)acétamide ; et le *N*1-{4-[3-chloro-5-(trifluorométhoxy)phényl]-1,3-thiazol-2-yl}-2-(1,3,6-triméthyl-2,4-dioxo-1,2,3,4-tétrahydrofuro-[2,3-*d*]pyrimidin-5-yl)acétamide ;
ou un sel pharmaceutiquement acceptable de celui-ci.

13. Composé selon la revendication 1 choisi parmi :
le *N*1-{4-[3-chloro-4-(difluorométhoxy)phényl]-1,3-thiazol-2-yl}-2-(1,3,6-triméthyl-2,4-dioxo-1,2,3,4-tétrahydrofuro-[2,3-*d*]pyrimidin-5-yl)acétamide ;
le *N*1-{4-[3-fluoro-4-(2,2,2-trifluoroéthoxy)phényl]-1,3-thiazol-2-yl}-2-(1,3,6-triméthyl-2,4-dioxo-1,2,3,4-tétrahydrofuro-[2,3-*d*]pyrimidin-5-yl)acétamide ;
le *N*1-{4-[3-chloro-4-(2,2,2-trifluoroéthoxy)phényl]-1,3-thiazol-2-yl}-2-(1,3,6-triméthyl-2,4-dioxo-1,2,3,4-tétrahydrofuro-[2,3-*d*]pyrimidin-5-yl)acétamide ;
le *N*1-[4-(2,4,5-trifluorophényl)-1,3-thiazol-2-yl]-2-(1,3,6-triméthyl-2,4-dioxo-1,2,3,4-tétrahydrofuro-[2,3-d]pyrimidin-5-yl)acétamide ;
le *N*1-[4-(3,4,5-trifluorophényl)-1,3-thiazol-2-yl]-2-(1,3,6-triméthyl-2,4-dioxo-1,2,3,4-tétrahydrofuro-[2,3-d]pyrimidin-5-yl)acétamide ;
le *N*1-[4-(2,3,4-trifluorophényl)-1,3-thiazol-2-yl]-2-(1,3,6-triméthyl-2,4-dioxo-1,2,3,4-tétrahydrofuro-[2,3-d]pyrimidin-5-yl)acétamide ;
le *N*1-[4-(3-chloro-2,4-difluorophényl)-1,3-thiazol-2-yl]-2-(1,3,6-triméthyl-2,4-dioxo-1,2,3,4-tétrahydrofuro[2,3-*d*]pyrimidin-5-yl)acétamide ;
le *N*1-[4-(2,4-dichloro-5-fluorophényl)-1,3-thiazol-2-yl]-2-(1,3,6-triméthyl-2,4-dioxo-1,2,3,4-tétrahydrofuro[2,3-*d*]pyrimidin-5-yl)acétamide ;
le *N*1-{4-(2,2-diméthylpropoxy)-3,5-difluorophényl]-1,3-thiazol-2-yl}-2-(1,3,6-triméthyl-2,4-dioxo-1,2,3,4-tétrahydrofuro[2,3-*d*]pyrimidin-5-yl)acétamide ;
le *N*1-[4-(3,5-difluoro-4-isobutoxyphényl)-1,3-thiazol-2-yl]-2-(1,3,6-triméthyl-2,4-dioxo-1,2,3,4-tétrahydrofuro[2,3-*d*]pyrimidin-5-yl)acétamide ;
le *N*1-[4-(3,5-dichloro-4-isobutoxyphényl)-1,3-thiazol-2-yl]-2-(1,3,6-triméthyl-2,4-dioxo-1,2,3,4-tétrahydrofuro[2,3-*d*]pyrimidin-5-yl)acétamide ;
le *N*1-{4-[2,4-difluoro-3-(trifluorométhyl)phényl]-1,3-thiazol-2-yl}-2-(1,3,6-triméthyl-2,4-dioxo-1,2,3,4-tétrahydrofuro[2,3-*d*]pyrimidin-5-yl)acétamide ;
le *N*1-{4-[3,5-difluoro-4-(trifluorométhyl)phényl]-1,3-thiazol-2-yl}-2-(1,3,6-triméthyl-2,4-dioxo-1,2,3,4-tétrahydrofuro[2,3-*d*]pyrimidin-5-yl)acétamide ;
le *N*1-{4-[3,5-difluoro-4-(difluorométhoxy)phényl]-1,3-thiazol-2-yl}-2-(1,3,6-triméthyl-2,4-dioxo-1,2,3,4-tétrahydrofuro[2,3-*d*]pyrimidin-5-yl)acétamide ;
le *N*1-{4-[3,5-dichloro-4-(difluorométhoxy)phényl]-1,3-thiazol-2-yl}-2-(1,3,6-triméthyl-2,4-dioxo-1,2,3,4-tétrahydrofuro[2,3-*d*]pyrimidin-5-yl)acétamide ;
le *N*1-{4-[3,5-difluoro-4-(2,2,2-trifluoroéthoxy)-phényl]-1,3-thiazol-2-yl}-2-(1,3,6-triméthyl-2,4-dioxo-1,2,3,4-tétrahydrofuro[2,3-*d*]pyrimidin-5-yl)acétamide ;
le *N*1-{4-[3,5-dichloro-4-(2,2,2-trifluoroéthoxy)-phényl]-1,3-thiazol-2-yl}-2-(1,3,6-triméthyl-2,4-dioxo-1,2,3,4-tétrahydrofuro[2,3-*d*]pyrimidin-5-yl)acétamidé ;
le *N*1-{4-[4-(cyclopropylméthoxy)-3,5-difluorophényl]-1,3-thiazol-2-yl}-2-(1,3,6-triméthyl-2,4-dioxo-1,2,3,4-tétrahydrofuro[2,3-*d*]pyrimidin-5-yl)acétamide ;
le *N*1-{4-[4-fluoro-3-(trifluorométhyl)phényl]-5-méthyl-1,3-thiazol-2-yl}-2-(1,3,6-triméthyl-2,4-dioxo-1,2,3,4-tétrahydrofuro[2,3-*d*]pyrimidin-5-yl)acétamide ;
le *N*1-[5-(4-bromophényl)-1,3,5-thiadiazol-2-yl]-2-(1,3,6-triméthyl-2,4-dioxo-1,2,3,4-tétrahydrofuro-[2,3-d]-pyrimidin-5-yl)acétamide ;
ou un sel pharmaceutiquement acceptable de celui-ci.

14. Composé selon la revendication 1 ayant la structure 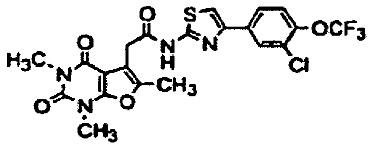 ou un sel pharmaceutiquement acceptable de celui-ci.

15. Composé selon la revendication 1 ayant la structure 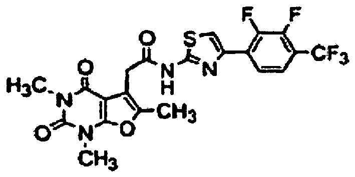 ou un sel pharmaceutiquement acceptable de celui-ci.

16. Composition pharmaceutique comprenant un ou plusieurs composés selon l'une quelconque des revendications 1 à 15, et un ou plusieurs excipients, supports, diluants pharmaceutiquement acceptables ou des mélanges de ceux-ci.

17. Composé selon l'une quelconque des revendications 1 à 15, pour une utilisation dans la prévention ou le traitement d'une maladie ou d'une affection associée à la fonction de TRPA1 chez un sujet.

18. Composé selon l'une quelconque des revendications 1 à 15, pour une utilisation dans la prévention ou le traitement des symptômes d'une maladie ou d'une affection associée à la fonction de TRPA1 qui est choisie parmi la douleur, la douleur chronique, le syndrome de douleur régionale complexe, la douleur neuropathique, la douleur postopératoire, la douleur due à la polyarthrite rhumatoïde, la douleur due à l'arthrose, la douleur du dos, la douleur viscérale, la douleur due à un cancer, une algésie, une névralgie, la migraine, des neuropathies, la neuropathie diabétique, la sciatique, une neuropathie associée au VIH, une névralgie postherpétique, une fibromyalgie, une lésion nerveuse, une ischémie, une neurodégénérescence, un accident vasculaire cérébral, la douleur après un accident vasculaire cérébral, la sclérose en plaques, des maladies respiratoires, l'asthme, la toux, une BPCO, des troubles inflammatoires, une oesophagite, un trouble de reflux gastro-oesophagien (GERD), le syndrome de l'intestin irritable, une maladie intestinale inflammatoire, une hypersensibilité pelvienne, l'incontinence urinaire, une cystite, des brûlures, un psoriasis, un eczéma, des vomissements, un ulcère gastroduodénal et un prurit.

19. Composé selon l'une quelconque des revendications 1 à 15 pour une utilisation dans le traitement de la douleur, où le composé est dans une quantité thérapeutiquement efficace.

20. Composé selon l'une quelconque des revendications 1 à 15, pour une utilisation dans le traitement de la douleur, où la douleur est une douleur chronique, une douleur neuropathique, une douleur rhumatoïde ou une douleur arthrosique.

21. Composé ayant la structure :
